# EUROPÄISCHE PATENTSCHRIFT

(11) **EP 1 220 841 B1**
(45) Veröffentlichungstag und Bekanntmachung des Hinweises auf die Patenterteilung: **20.07.2011**
(21) Anmeldenummer: 00967741.0
(22) Anmeldetag: 19.09.2000
(51) Int. Cl.: C07D 209/96, A01N 43/38, C07D 307/94

(54) **TRIFLUORMETHYLSUBSTITUIERTE SPIROCYCLISCHE KETOENOLE UND IHRE VERWENDUNG ALS SCHÄDLINGSBEKÄMPFUNGSMITTEL UND HERBIZIDE**
TRIFLUOROMETHYL-SUBSTITUTED SPIROCYCLIC KETOENOLS
CETOENOLS SPIROCYCLIQUES SUBSTITUES PAR TRIFLUOROMETHYLE

(30) Priorität: 29.09.1999 DE 19946625
(43) Veröffentlichungstag der Anmeldung: 10.07.2002
(73) Patentinhaber: Bayer CropScience AG, 40789 Monheim (DE)
(72) Erfinder: FISCHER, Reiner, 40789 Monheim (DE); GRAFF, Alan, 51375 Leverkusen (DE); BRETSCHNEIDER, Thomas, 53797 Lohmar (DE); ERDELEN, Christoph, 42799 Leichlingen (DE); DREWES, Mark, Wilhelm, 40764 Langenfeld (DE); FEUCHT, Dieter, 40789 Monheim (DE)
(86) Internationale Anmeldenummer: PCT/EP2000/009270
(87) Internationale Veröffentlichungsnummer: WO 2001/023354

(56) Entgegenhaltungen:
- EP-A- 0 528 156
- EP-A- 0 647 637
- EP-A- 0 668 267
- WO-A-95/26954
- WO-A-97/36868
- WO-A-98/05638

## Beschreibung

Die vorliegende Erfindung betrifft neue trifluormethylsubstituierte spirocyclische Ketoenole, mehrere Verfahren zu ihrer Herstellung und ihre Verwendung als Schädlingsbekämpftungsmittel und Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-A-415 211) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 94/01 997, WO 95/26 954, WO 95/20 572, EP-A-0 668 267, WO 96/25 395, WO 96/35 664, WO 97/01 535, WO 97/02 243, WO 97/36 868, WO 98/05 638, WO 98/06 721, WO 98/25 928, WO 99/16 748 und WO 99/24 437).

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-di-hydrofuranon-Derivate mit herbiziden, akariziden und insektiziden Eigenschaften aus EP-A-528 156, EP-A-647 637, WO 95/26 345, WO 96/20 196, WO 96/25 395, WO 96/35 664, WO 97/01 535, WO 97/02 243, WO 97/36 868, WO 98/05 638 WO 99/16 748 und WO 98/25 928 bekannt. Auch 3-Aryl-Δ³-dihydrothiphen-on-Derivate sind bekannt (WO 95/26 345, WO 96/25 395, WO 97/01 535, WO 97/02 243, WO 97/36 868, WO 98/05 638, WO 98/25 928, WO 99/16 748).

Die herbizide, akarizide und insektizide Wirksamkeit und/oder die Wirkungsbreite und die Pflanzenverträglichkeit dieser Verbindungen insbesondere gegenüber Kulturpflanzen ist jedoch nicht immer ausreichend.

Es wurden nun neue Verbindungen der Formel (I) 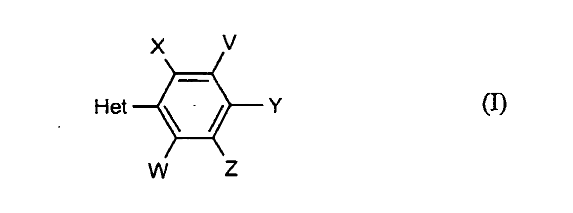 gefunden,
in welcher
- V: für Wasserstoff, Halogen, Alkyl oder Alkoxy steht,
- W: für Wasserstoff, Cyano, Nitro, Halogen, Alkyl, Alkenyl, Alkinyl, Alkoxy, Halogenalkyl, Halogenalkoxy, jeweils gegebenenfalls substituiertes Phenyl, Phenoxy, Phenylthio, Phenylalkoxy oder Phenylalkylthio steht,
- X: für Halogen, Alkyl, Alkenyl, Alkinyl, Alkoxy, Halogenalkyl, Halogenalkoxy, Cyano, Nitro, jeweils gegebenenfalls substituiertes Phenyl, Phenoxy, Phenyl- thio, Phenylalkyloxy oder Phenylalkylthio steht,
- Y: für Wasserstoff, Halogen, Alkyl, Alkoxy, Halogenalkyl, Halogenalkoxy, Cyano oder Nitro steht,
- Z: für Wasserstoff, Halogen, Alkyl, Alkoxy, Halogenalkyl, Halogenalkoxy, Hydroxy, Cyano, Nitro oder jeweils gegebenenfalls substituiertes Phenoxy, Phenylthio, 5- oder 6-gliedriges Hetaryloxy, 5- oder 6-gliedriges Hetarylthio, Phenylalkyloxy oder Phenylalkylthio steht,
- Het: für eine der Gruppen 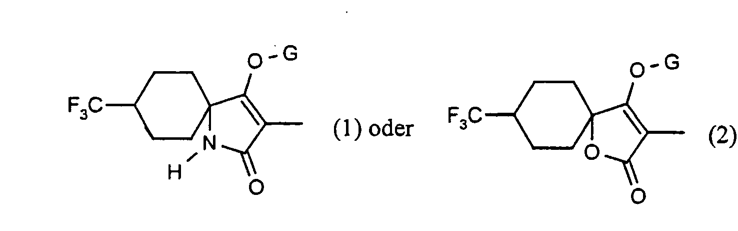 steht,
worin
- G: für Wasserstoff (a) oder für eine der Gruppen 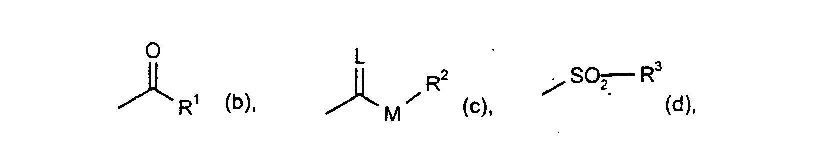 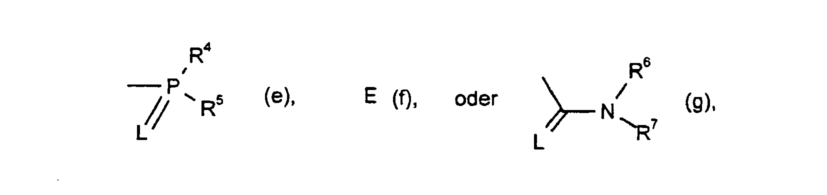 steht,
worin
E für ein Metallion oder ein Ammoniumion steht,
L für Sauerstoff oder Schwefel steht,
M für Sauerstoff oder Schwefel steht,
- R¹: für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen oder Cyano substituiertes Alkyl, Alkenyl, Alkoxyalkyl oder Polyalkoxyallcyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alkenyl- thio oder Cycloalkylthio oder für jeweils gegebenenfalls substituiertes Phe- nyl, Benzyl, Phenoxy oder Phenylthio stehen,
- R⁶ und R⁷: unabhängig voneinander für Wasserstoff, für jeweils gegebenenfalls durch Halogen oder Cyano substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden,
ausgenommen die Verbindung I-a-79 aus EP-A-528 156

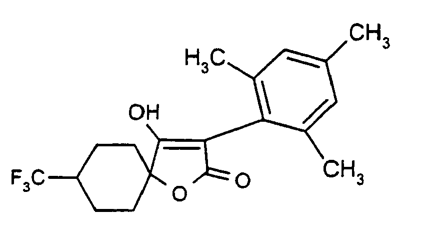

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (2) der Gruppe Het ergeben sich folgende hauptsächliche Strukturen (I-1) bis (I-2): 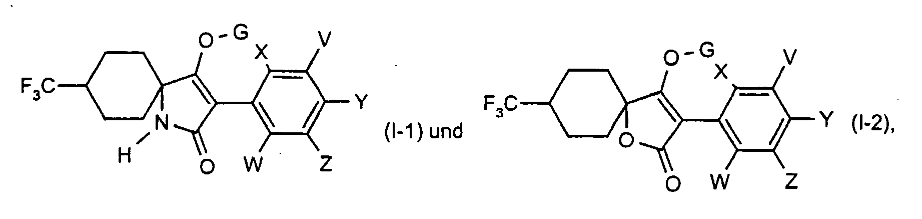 worin
G, V, W, X, Y und Z die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn Het für die Gruppe (1) steht, 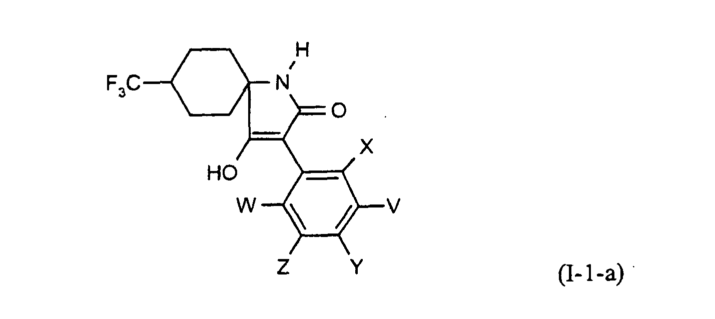 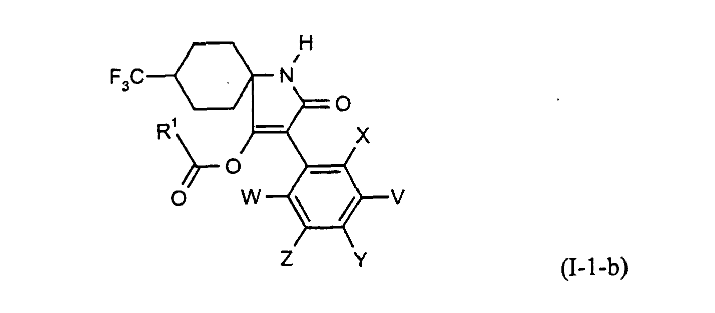 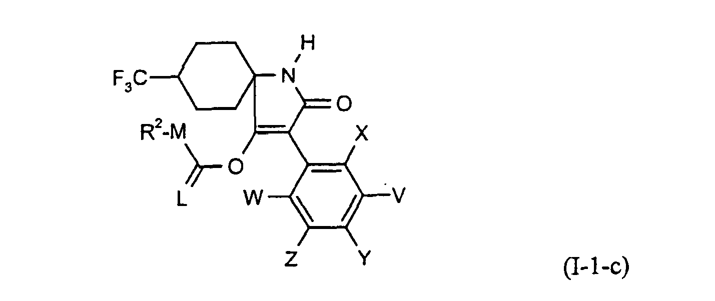 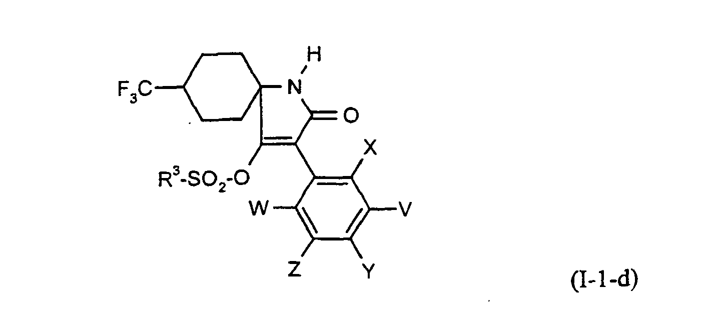 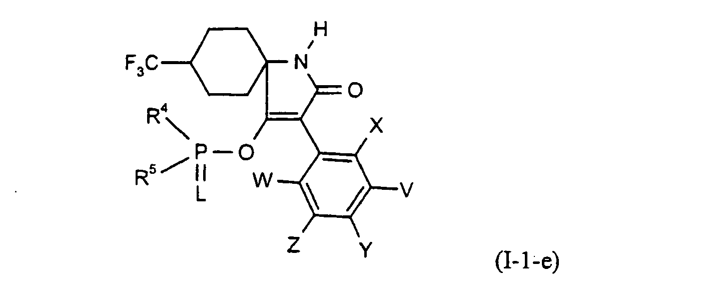 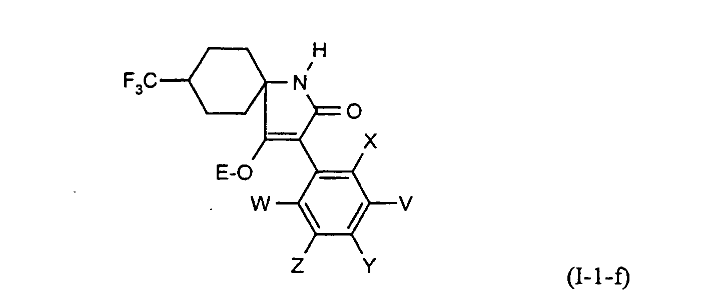 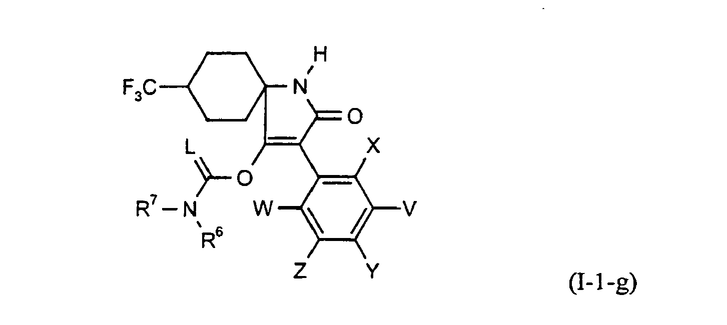 worin
E, L, M, V, W, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn Het für die Gruppe (2) steht, 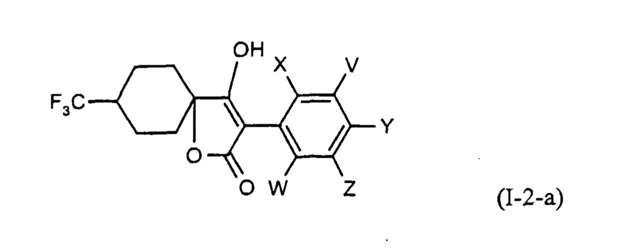 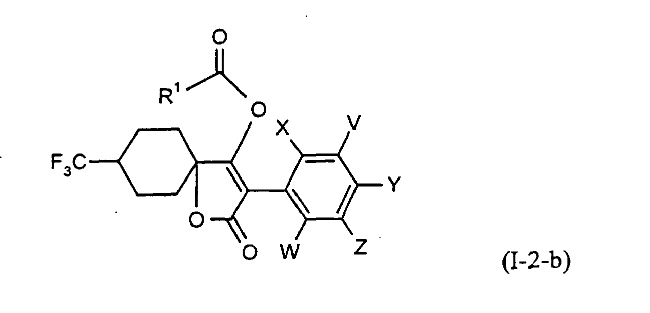 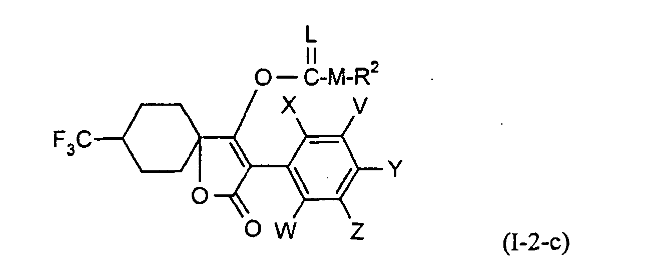 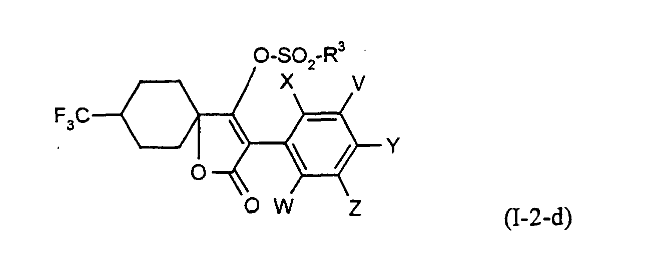 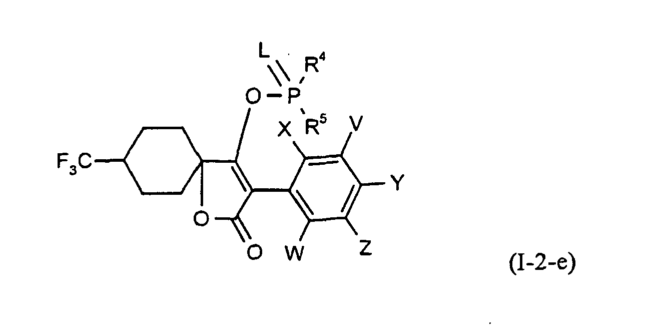 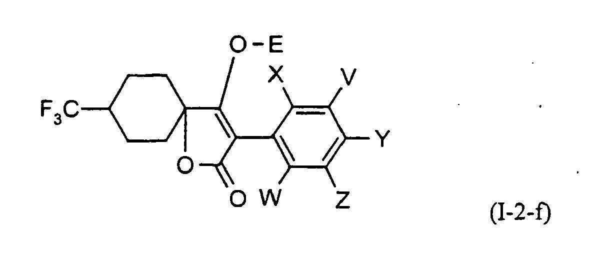 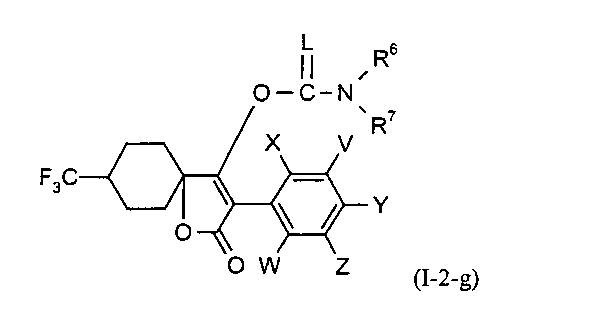 worin
E, L, M, V, W, X, Y, Z, R¹, R², R³, R⁴, R⁵, R⁶ und R⁷ die oben angegebene Bedeutung haben.

Weiterhin wurde gefunden, daß man die neuen Verbindungen der Formel (I) nach den im folgenden beschriebenen Verfahren erhält:
(A) Man erhält Verbindungen der Formel (I-1-a) 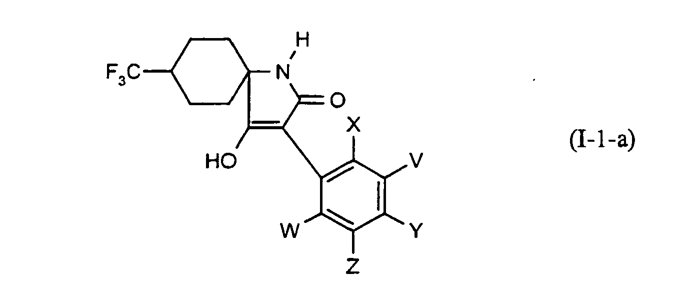 in welcher
   V, W, X, Y und Z die oben angegebenen Bedeutungen haben,
   wenn man
   Verbindungen der Formel (II) 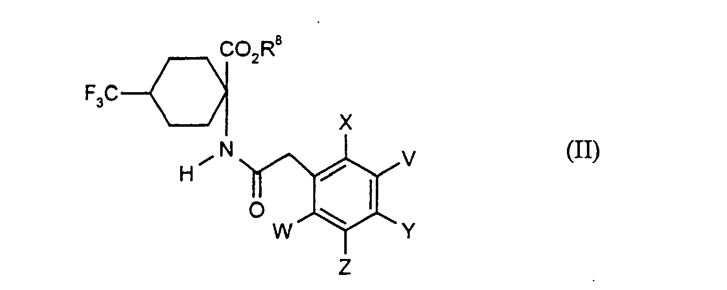 in welcher
   V, W, X, Y und Z die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, daß man Verbindungen der Formel (I-2-a) 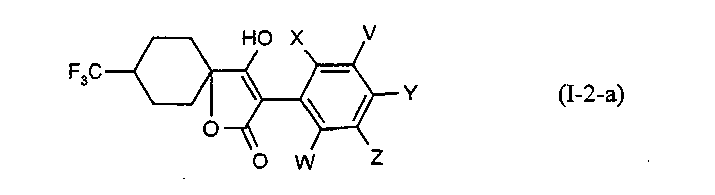 in welcher
   V, W, X, Y und Z die oben angegebenen Bedeutungen haben,
   erhält, wenn man
   Verbindungen der Formel (III) 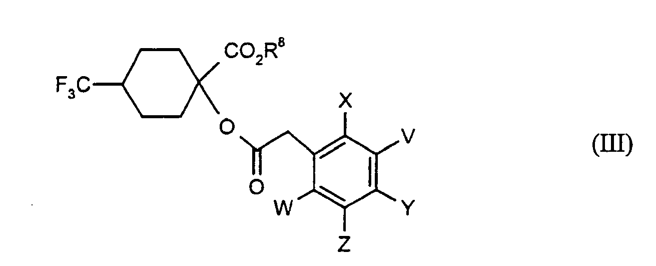 in welcher
   V, W, X, Y, Z und R⁸ die oben angegebenen Bedeutungen haben,
   in Gegenwart eines Verdünnungsmittels und in Gegenwart einer Base intramolekular kondensiert.
   Außerdem wurden gefunden
(C) daß man die Verbindungen der oben gezeigten Formeln (I-1-b) bis (I-2-b), in welchen R¹, V, W, X, Y und Z die oben angebenen Bedeutungen haben, erhält, wenn man Verbindungen der oben gezeigten Formeln (I-1-a) bis (I-2-a), in welchen V, W, X, Y und Z die oben angegebenen Bedeutungen haben, jeweils
   α) mit Verbindungen der Formel (IV) 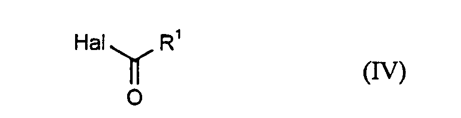 in welcher
   - R¹: die oben angegebene Bedeutung hat und
   - Hal: für Halogen (insbesondere Chlor oder Brom) steht
   oder
   β) mit Carbonsäureanhydriden der Formel (V)

   R¹-CO-O-CO-R¹ (V)

   in welcher
   R¹ die oben angegebene Bedeutung hat,
   gegebenenfalls in Gegenwart eines Verdünnungsmittels und gegebenenfalls in Gegenwart eines Säurebindemittels umsetzt;
(D) daß man die Verbindungen der oben gezeigten Formeln (I-1-c) bis (I-2-c), in welchen R², V, W, M, X, Y und Z die oben angegebenen Bedeutungen haben und L für Sauerstoff steht, erhält, wenn man Verbindungen der oben gezeigten Formeln (I-1-a) bis (1-2-a), in welchen V, W, X, Y und Z die oben angegebenen Bedeutungen haben, jeweils
   mit Chlorameisensäureestern oder Chlorameisensäurethioestem der Formel (VI)

   R²-M-CO-Cl (VI)

   in welcher
   R² und M die oben angegebenen Bedeutungen haben, gegebenenfalls in Gegenwart eines Verdünnungsmittels und gegebenenfalls in Gegenwart eines Säurebindemittels umsetzt;
(E) daß man Verbindungen der oben gezeigten Formeln (I-1-c) bis (1-2-c), in welchen R², V, W, M, X, Y und Z die oben angegebenen Bedeutungen haben und L für Schwefel steht, erhält, wenn man Verbindungen der oben gezeigten Formeln (I-1-a) bis (I-2-a), in welchen V, W, X, Y und Z die oben angegebenen Bedeutungen haben, jeweils
   mit Chlormonothioameisensäureestern oder Chlordithioameisensäureestern der Formel (VII) 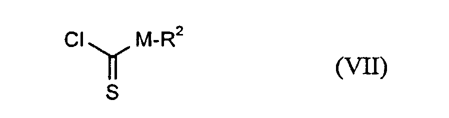 in welcher
   M und R² die oben angegebenen Bedeutungen haben,
   gegebenenfalls in Gegenwart eines Verdünnungsmittels und gegebenenfalls in Gegenwart eines Säurebindemittels umsetzt,
(F) daß man Verbindungen der oben gezeigten Formeln (I-1-d) bis (1-2-d), in welchen R³, V, W, X, Y und Z die oben angegebenen Bedeutungen haben, erhält, wenn man Verbindungen der oben gezeigten Formeln (I-1-a) bis (I-2-a), in welchen V, W, X, Y und Z die oben angegebenen Bedeutungen haben, jeweils
   mit Sulfonsäurechloriden der Formel (VIII)

   R³-SO₂-Cl (VIII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(G) daß man Verbindungen der oben gezeigten Formeln (I-1-e) bis (1-2-e), in welchen L, R⁴, R⁵, V, W, X, Y und Z die oben angegebenen Bedeutungen haben, erhält, wenn man Verbindungen der oben gezeigten Formeln (I-1-a) bis (I-2-a), in welchen V, W, X, Y und Z die oben angegebenen Bedeutungen haben, jeweils
   mit Phosphorverbindungen der Formel (IX) 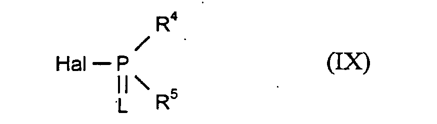 in welcher
   L, R⁴ und R⁵ die oben angegebenen Bedeutungen haben und
   Hal für Halogen (insbesondere Chlor oder Brom) steht,
   gegebenenfalls in Gegenwart eines Verdünnungsmittels und gegebenenfalls in Gegenwart eines Säurebindemittels umsetzt,
(H) daß man Verbindungen der oben gezeigten Formeln (I-1-f) bis (I-2-f), in welchen E, V, W, X, Y und Z die oben angegebenen Bedeutungen haben, erhält, wenn man Verbindungen der Formeln (I-1-a) bis (1-2-a), in welchen V, W, X, Y und Z die oben angegebenen Bedeutungen haben, jeweils
   mit Metallverbindungen oder Aminen der Formeln (X) oder (XI)

   Me(OR¹⁰)ₜ (X)

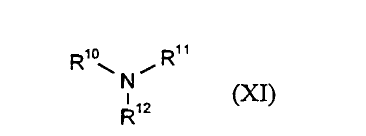

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   R¹⁰, R¹¹, R¹² unabhängig voneinander für Wasserstoff oder Alkyl (bevorzugt C₁-C₈-Alkyl) stehen,
   gegebenenfalls in Gegenwart eines Verdünnungsmittels umsetzt,
(I) daß man Verbindungen der oben gezeigten Formeln (I-1-g) bis (I-2-g), in welchen L, R⁶, R⁷, V, W, X; Y und Z die oben angegebenen Bedeutungen haben, erhält, wenn man Verbindungen der oben gezeigten Formeln (I-1-a) bis (I-2-a), in welchen V, W, X, Y und Z die oben angegebenen Bedeutungen haben, jeweils
   α) mit Isocyanaten oder Isothiocyanaten der Formel (XII)

   R⁶-N=C=L (XII)

   in welcher
   R⁶ und L die oben angegebenen Bedeutungen haben, gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XIII) 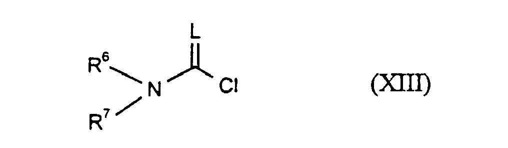 in welcher
   L, R⁶ und R⁷ die oben angegebenen Bedeutungen haben,
   gegebenenfalls in Gegenwart eines Verdünnungsmittels und gegebenenfalls in Gegenwart eines Säurebindemittels, umsetzt.

Weiterhin wurde gefunden, daß die neuen Verbindungen der Formel (I) eine sehr gute Wirksamkeit als Schädlingsbekämpfungsmittel, vorzugsweise als Insektizide, Akarizide und Herbizide aufweisen und darüber hinaus häufig sehr gut pflanzenverträglich, insbesondere gegenüber Kulturpflanzen, sind.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- V: steht bevorzugt für Wasserstoff, Halogen, C₁-C₆-Alkyl oder C₁-C₆-Alkoxy.
- W: steht bevorzugt für Wasserstoff, Nitro, Cyano, Halogen, C₁-C₆-Alkyl, C₂- C₆-Alkenyl oder C₂-C₆-Alkinyl, C₁-C₆-Alkoxy, C₁-C₄-Halogenalkyl, C₁- C₄-Halogenalkoxy oder jeweils gegebenenfalls durch Halogen, C₁-C₆-Alkyl, C₁-C₆-Alkoxy, C₁-C4-Halogenakyl, C₁-C₄-Halogenallcoxy, Nitro oder Cyano substituiertes Phenyl, Phenoxy, Phenylthio, Phenyl-C₁-C₄-alkoxy oder Phenyl-C₁-C₄-alkylthio.
- X: steht bevorzugt für Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy, Cyano, Nitro oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄- alkylthio.
- Y: steht bevorzugt für Wasserstoff, Halogen, C₁-C₆-Alkyl, C₁-C₆-Alkoxy, C₁- C₄-Halogenalkyl, C₁-C₄-Halogenalkoxy, Cyano oder Nitro.
- Z: steht bevorzugt für Wasserstoff, Halogen, C₁-C₆-Alkyl, C₁-C₆-Alkoxy, C₁- C₄-Halogenalkyl, C₁-C₄-Halogenalkoxy, Hydroxy, Cyano, Nitro oder jeweils gegebenenfalls durch Halogen, C₁-C₄-Alkyl, C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyl- oxy, Phenyl-C₁-C₄-alkyloxy oder Phenyl-C₁-C₄-alkylthio oder
- Het: steht bevorzugt für eine der Gruppen 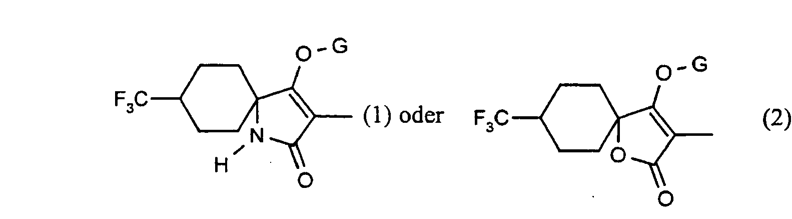 steht,
- G: steht bevorzugt für Wasserstoff (a) oder für eine der Gruppen 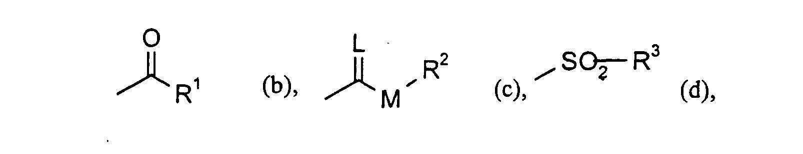 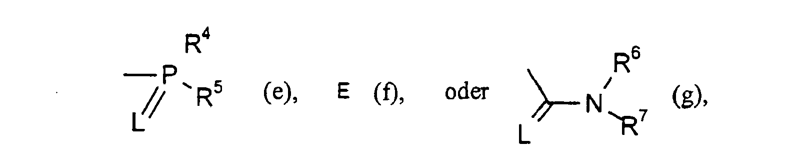 in welchen
E für ein Metallion oder ein Ammoniumion steht,
L für Sauerstoff oder Schwefel steht und
M für Sauerstoff oder Schwefel steht.
- R¹: steht bevorzugt für jeweils gegebenenfalls durch Halogen oder Cyano sub- stituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substi- tuiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆- Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆- Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6- gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy- C₁-C₆₋alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff.
- R²: steht bevorzugt für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substi- tuiertes C₃-C₈-Cycloalkyl oder
für jeweils gegebenenfalls durch Halogen, Cyano, Nitro, C₁-C₆-Alkyl, C₁- C₆-Alkoxy, C₁-C₆-Halogenalkyl oder C₁-C₆-Halogenalkoxy substituiertes Phenyl oder Benzyl.
- R³: steht bevorzugt für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl.
- R⁴ und R⁵: stehen unabhängig voneinander bevorzugt für jeweils gegebenenfalls durch Halogen substituiertes C₁-C₈-Alkyl, C₁-C₈-Alkoxy, C₁-C₈- Alkyl- amino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄- Alkyl oder C₁-C₄-Halogenalkyl substituiertes Phenyl, Phenoxy oder Phenyl- thio.
- R⁶: und R⁷ stehen unabhängig voneinander bevorzugt für Wasserstoff, für jeweils gegebenenfalls durch Halogen oder Cyano substituiertes C₁-C₈-Alkyl, C₃- C₈-Cycloalkyl, C₁-C₈-Alkoxy, C₃-C₈-Alkenyl oder C₁-C₈-Alkoxy-C₂-C₈- alkyl, für jeweils gegebenenfalls durch Halogen, C₁-C₈-Alkyl, C₁-C₈- Halogenalkyl oder C₁-C₈-Alkoxy substituiertes Phenyl oder Benzyl oder zu- sammen für einen gegebenenfalls durch C₁-C₆-Alkyl substituierten C₃-C₆- Alkylenrest, in welchem gegebenenfalls eine Methylengruppe durch Sauerstoff oder Schwefel ersetzt ist.
- V: steht besonders bevorzugt für Wasserstoff, Fluor, Chlor, Brom, C₁-C₄-Alkyl oder C₁-C₄-Alkoxy.
- W: steht besonders bevorzugt für Wasserstoff, Fluor, Chlor, Brom, C₁-C₄-Alkyl, C₁-C₄-Alkoxy, C₁-C₂-Halogenalkyl oder C₁-C₂-Halogenalkoxy.
- X: steht besonders bevorzugt für Fluor, Chlor, Brom, C₁-C₄-Alkyl, C₁-C₄- Alkoxy, C₁-C₂-Halogenalkyl, C₁-C₂-Halogenalkoxy, Cyano oder Nitro.
- Y: steht besonders bevorzugt für Wasserstoff, Fluor, Chlor, Brom, C₁-C₄-Alkyl, C₁-C₄-Alkoxy, C₁-C₂-Halogenalkyl, C₁-C₂-Halogenalkoxy, Cyano oder Nitro.
- Z: steht besonders bevorzugt für Wasserstoff, Fluor, Chlor, Brom, C₁-C₄-Alkyl, C₁-C₄-Alkoxy, C₁-C₂-Halogenalkyl, C₁-C₂-Halogenalkoxy, Hydroxy, Cyano, Nitro oder jeweils gegebenenfalls durch Fluor, Chlor, Brom, C₁-C₄-Alkyl, C₁-C₄-Alkoxy, C₁-C2-Halogenalkyl, C₁-C₂-Halogenalkoxy, Nitro oder Cyano substituiertes Phenoxy oder Benzyloxy.
- Het: steht besonders bevorzugt für eine der Gruppen 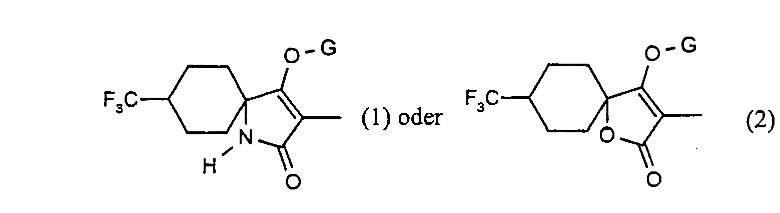 steht,
- G: steht besonders bevorzugt für Wasserstoff (a) oder für eine der Gruppen 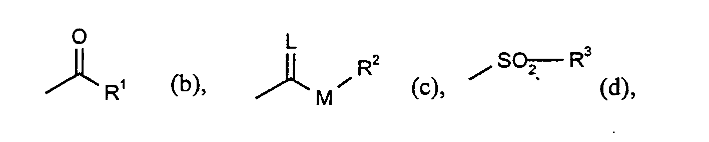 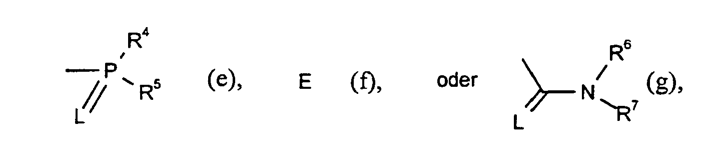 in welchen
E für ein Metallion oder ein Ammoniumion steht,
L für Sauerstoff oder Schwefel steht und
M für Sauerstoff oder Schwefel steht.
- R¹: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy sub- stituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkyl- thio oder C₁₋C4-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁- C₄-alkyl,
für jeweils gegebenenfalls durch Fluor, Chlor, Brom oder C₁-C₄-Alkyl sub- stituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄- Alkyl substituiertes Pyridyloxy-C₁-C₅-alkyl, Pyrimidyloxy-C₁-C₅-alkyl oder Thiazolyloxy-C₁-C₅-alkyl.
- R²: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy sub- stituiertes C₃-C₇-Cycloalkyl oder
für jeweils gegebenenfalls durch Fluor, Chlor, Brom, Cyano, Nitro, C₁-C₄- Alkyl, C₁-C₃-Alkoxy, C₁-C₃-Halogenalkyl oder C₁-C₃-Halogenalkoxy sub- stituiertes Phenyl oder Benzyl.
- R³: steht besonders bevorzugt für gegebenenfalls durch Fluor oder Chlor substituiertes C₁-C₆-Alkyl oder jeweils gegebenenfalls durch Fluor, Chlor, Brom, C₁-C₄-Alkyl, C₁-C₄-Alkoxy, C₁-C₂-Halogenalkoxy, C₁-C₂-Halo- genalkyl, Cyano oder Nitro substituiertes Phenyl oder Benzyl.
- R⁴ und R⁵: stehen unabhängig voneinander besonders bevorzugt für jeweils gegebenenfalls durch Fluor oder Chlor substituiertes C₁-C₆-Alkyl, C₁-C₆- Alkoxy, C₁-C₆-Alkylamino, Di-(C₁-C₆-alkyl)amino, C₁-C₆-Alkylthio oder C₃-C₄-Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁- C₃-Halogenalkylthio, C₁-C₃-Alkyl oder C₁-C₃-Halogenalkyl substituiertes Phenyl, Phenoxy oder Phenylthio.
- R⁶ und R⁷: stehen unabhängig voneinander besonders bevorzugt für Wasserstoff, für jeweils gegebenenfalls durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂- C₆-alkyl, für jeweils gegebenenfalls durch Fluor, Chlor, Brom, C₁-C₅-Halo- genalkyl, C₁-C₅-Alkyl oder C₁-C₅-Alkoxy substituiertes Phenyl oder Ben- zyl, oder zusammen für einen gegebenenfalls durch C₁-C₄-Alkyl substituierten C₃-C₆-Alkylenrest, in welchem gegebenenfalls eine Methylen- gruppe durch Sauerstoff oder Schwefel ersetzt ist.
- V: steht ganz besonders bevorzugt für Wasserstoff, Fluor, Chlor, Brom, Methyl, Ethyl, Methoxy oder Ethoxy.
- W: steht ganz besonders bevorzugt für Wasserstoff, Fluor, Chlor, Brom, Methyl, Ethyl, Propyl, Methoxy oder Ethoxy.
- X: steht ganz besonders bevorzugt für Fluor, Chlor, Brom, Methyl, Ethyl, Propyl, iso-Propyl, Methoxy, Ethoxy, Propoxy, iso-Propoxy, Trifluormethyl, Trifluormethoxy, Difluormethoxy oder Cyano.
- Y: steht ganz besonders bevorzugt für Wasserstoff, Fluor, Chlor, Brom, Methyl, Ethyl, Propyl, iso-Propyl, tert.-Butyl, Methoxy, Ethoxy, Propoxy, iso- Propoxy, Trifluormethyl, Trifluormethoxy, Difluormethoxy, Cyano oder Nitro.
- Z: steht ganz besonders bevorzugt für Wasserstoff, Fluor, Chlor, Brom, Methyl, Ethyl, Propyl, iso-Propyl, tert.-Butyl, Methoxy, Ethoxy, Propoxy, iso- Propoxy, Trifluormethyl, Trifluormethoxy, Difluormethoxy, Cyano oder Nitro.
- Het: steht ganz besonders bevorzugt für eine der Gruppen 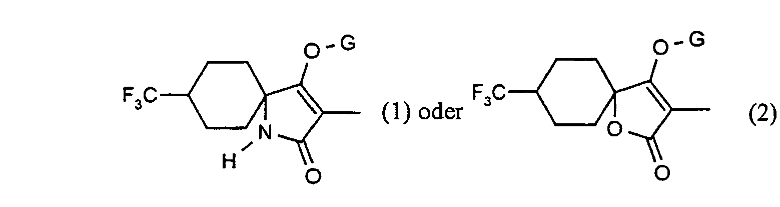 steht,
- G: steht ganz besonders bevorzugt für Wasserstoff (a) oder für eine der Gruppen 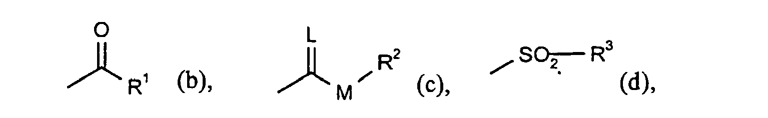 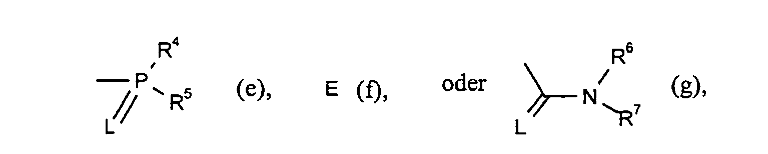 in welchen
E für ein Metallion oder ein Ammoniumion steht,
L für Sauerstoff oder Schwefel steht und
M für Sauerstoff oder Schwefel steht.
- R¹: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₁-C₆- alkyl, C₁-C₄-Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n- Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, Methyl, Ethyl, n- Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i- Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl sub- stituiertes Pyridyloxy-C₁-C₄-alkyl, Pyrimidyloxy-C₁-C₄-alkyl oder Thiazo- lyloxy-C₁-C₄-alkyl.
- R²: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₂-C₆- alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluor- methoxy substituiertes Phenyl oder Benzyl.
- R³: steht ganz besonders bevorzugt für jeweils gegebenenfalls durch Fluor oder Chlor substituiertes Methyl, Ethyl, Propyl, iso-Propyl, Butyl, tert.-Butyl, oder jeweils gegebenenfalls durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl oder Benzyl.
- R⁴ und R⁵: stehen unabhängig voneinander ganz besonders bevorzugt für jeweils gegebenenfalls durch Fluor oder Chlor substituiertes C₁-C₄-Alkyl, C₁-C₄- Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio.
- R⁶ und R⁷: stehen unabhängig voneinander ganz besonders bevorzugt für Wasser- stoff, für jeweils gegebenenfalls durch Fluor oder Chlor substituiertes C₁-C₄- Alkyl, C₃-C₆-Cycloalkyl, C₁-C₄-Alkoxy, C₃-C₄-Alkenyl oder C₁-C₄-Alk- oxy-C₂-C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest, in welchem gegebenenfalls eine Methylengruppe durch Sauerstoff oder Schwefel ersetzt ist.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

**Tabelle 1**

| | | | | |
|---|---|---|---|---|
| 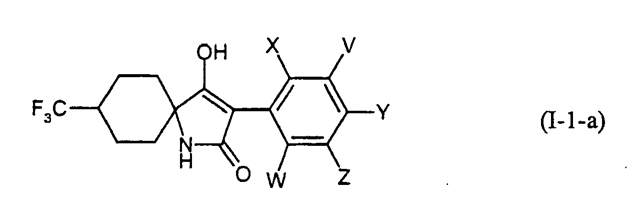 | | | | |

| V | X | W | Y | Z |
|---|---|---|---|---|
| H | Br | H | Cl | H |
| H | Cl | H | Br | H |
| H | Cl | H | Cl | H |
| H | Cl | H | F | H |
| H | F | H | Cl | H |
| H | Cl | H | OCH₃ | H |
| H | Cl | H | CH₃ | H |
| H | OCH₃ | H | Cl | H |
| H | OCH₃ | H | OCH₃ | H |
| H | CH₃ | H | Cl | H |
| H | CH₃ | H | F | H |
| H | CH₃ | H | OCH₃ | H |
| H | CH₃ | H | t-C₄H₉ | H |
| H | CH₃ | H | CH₃ | H |
| H | Cl | Cl | H | H |
| H | Cl | F | H | H |
| H | Cl | OCH₃ | H | H |
| H | Cl | CH₃ | H | H |
| H | Cl | OC₂H₅ | H | H |
| H | OCH₃ | OCH₃ | H | H |
| H | CH₃ | CH₃ | H | H |
| H | Br | CH₃ | Br | H |
| H | Cl | Cl | CH₃ | H |
| H | CH₃ | Br | CH₃ | H |
| H | CH₃ | Cl | CH₃ | H |
| H | CH₃ | OCHF₂ | CH₃ | H |
| H | CH₃ | OCH₂CF₃ | CH₃ | H |
| H | CH₃ | OC₂H₅ | CH₃ | H |
| H | CH₃ | OCH₃ | CH₃ | H |
| H | CH₃ | CH₃ | CH₃ | H |
| H | Br | Br | CH₃ | H |
| H | Cl | Cl | CH₃ | H |
| H | C₂H₅ | C₂H₅ | Br | H |
| H | CH₃ | CH₃ | Br | H |
| H | CH₃ | CH₃ | OCH₃ | H |
| H | Br | Cl | CH₃ | H |
| H | Br | CH₃ | Cl | H |
| H | Cl | CH₃ | Br | H |
| H | C₂H₅ | Br | CH₃ | H |
| H | CH₃ | O-C₃H₇ | CH₃ | H |
| H | CH₃ | CH₃ | Cl | H |
| H | Cl | H | Cl | Cl |
| H | CH₃ | H | CH₃ | CH₃ |
| H | CH₃ | H | Cl | CH₃ |
| H | Br | H | Cl | CH₃ |
| H | Br | H | CH₃ | CH₃ |
| H | Cl | H | Br | CH₃ |
| H | Cl | H | Cl | CH₃ |
| H | CH₃ | H | Br | CH₃ |
| H | Cl | H | Cl | F |
| H | Cl | H | CH₃ | Cl |
| H | CH₃ | H | H | H |
| H | Cl | H | H | H |
| V | X | W | Y | Z |
| H | Br | H | H | H |
| H | CF₃ | H | H | H |
| H | OCH₃ | H | H | H |
| H | CH₃ | CH₃ | CH₃ | CH₃ |
| CH₃ | CH₃ | H | CH₃ | CH₃ |
| CH₃ | CH₃ | CH₃ | H | CH₃ |
| H | CH₃ | CH₃ | CH₃ | F |
| H | CH₃ | CH₃ | CH₃ | Cl |
| H | CH₃ | CH₃ | CH₃ | Br |
| H | CH₃ | CH₃ | H | Cl |
| H | CH₃ | CH₃ | H | Br |
| H | Cl | Cl | H | Br |
| CH₃ | CH₃ | CH₃ | CH₃ | CH₃ |

Im einzelnen seien außer den bei den Herstellungsbeispielen genannten Verbindungen die folgenden Verbindungen der Formel (I-2-a) genannt.

### Tabelle 2

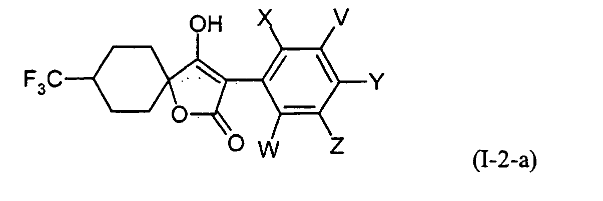

in welcher
V, W, X, Y und Z die in Tabelle 1 angegebenen Bedeutungen haben.

Verwendet man gemäß Verfahren (A) N-[(4-Chlor-2,6-dimethyl)-phenylacetyl]-1-amino-4-trifluormethyl-cyclohexancarbonsäureethylester als Ausgangsstoff, so kann der Verlauf des erfindungsgemäßen Verfahrens durch folgendes Reaktionsschema wiedergegeben werden:

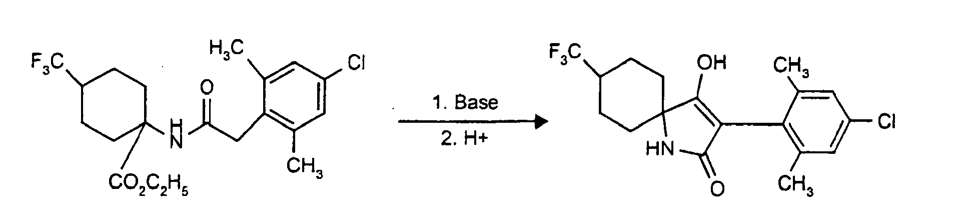

Verwendet man gemäß Verfahren (B) O-[(2-Chlor-6-methyl)-phenylacetyl]-1-hydroxy-4-trifluormethyl-cyclohexancarbonsäureethylester, so kann der Verlauf des erfindungsgemäßen Verfahrens durch folgendes Reaktionsschema wiedergegeben werden:

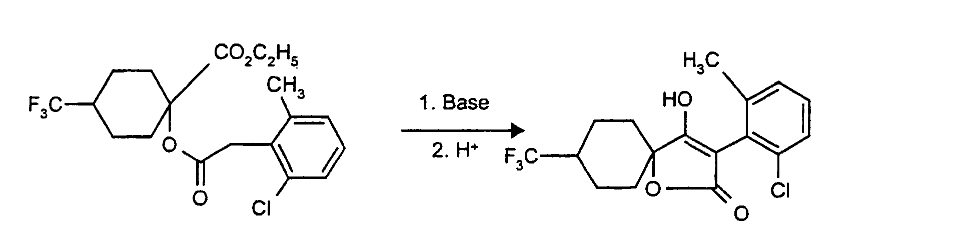

Verwendet man gemäß Verfahren (Cα) 3-[(2-Chlor-4-methyl)-phenyl]-5,5-(3-trifluormethyl-pentamethylendiyl)-pyrrolidin-2,4-dion und Pivaloylchlorid als Ausgangsstoffe, so kann der Verlauf des erfindungsgemäßen Verfahrens durch folgendes Reaktionsschema wiedergegeben werden:

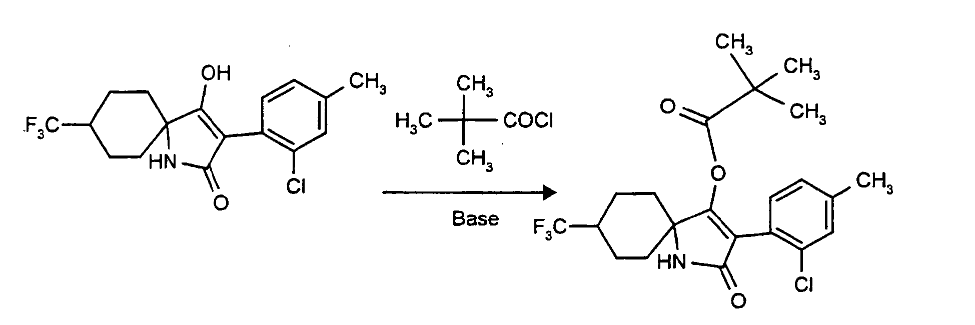

Verwendet man gemäß Verfahren (C) (Variante β) 3-[(2,4-Dichlor)-phenyl]-4-hydroxy-5,5-(3-trifluormethyl-pentanlethylendiyl)-Δ³-dihydrofüran-2-on und Acetanhydrid als Ausgangsverbindungen, so kann der Verlauf des erfindungsgemäßen Verfahrens durch folgendes Reaktionsschema wiedergegeben werden:

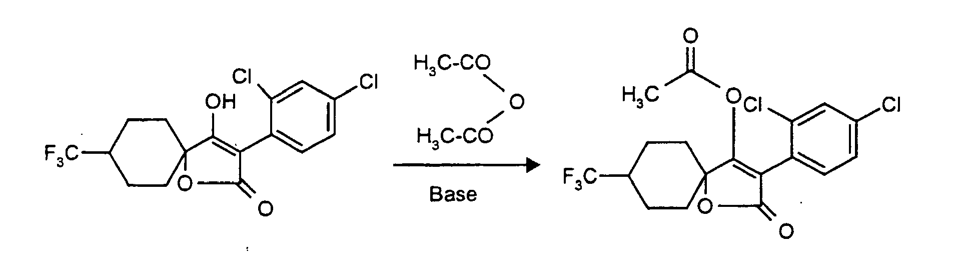

Verwendet man gemäß Verfahren (D) 8-[(2,4-Dichlor)-phenyl]-5,5-(3-trifluormethylpentamethylendiyl)-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

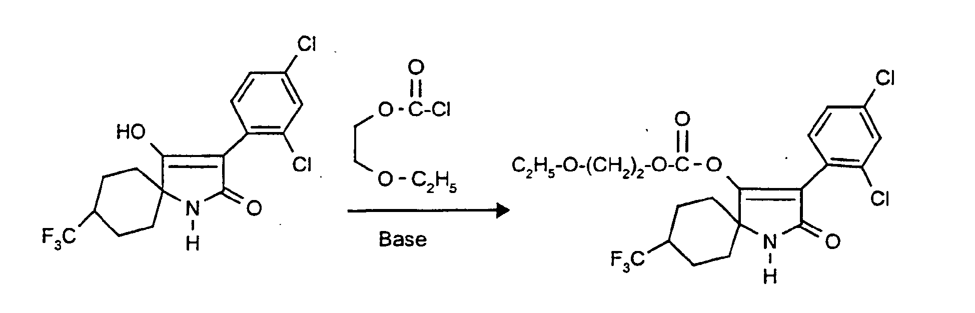

Verwendet man gemäß Verfahren (E) 3-[(2,6-Dibrom-4-methyl)-phenyl]-4-hydroxy-5,5-(3-trifluormethyl-pentamethylendiyl)-Δ³-dihydrofuran-2-on und Chlormonothioameisensäuremethylester als Ausgangsprodukte, so kann der Reaktionsverlauf folgendermaßen wiedergegeben werden:

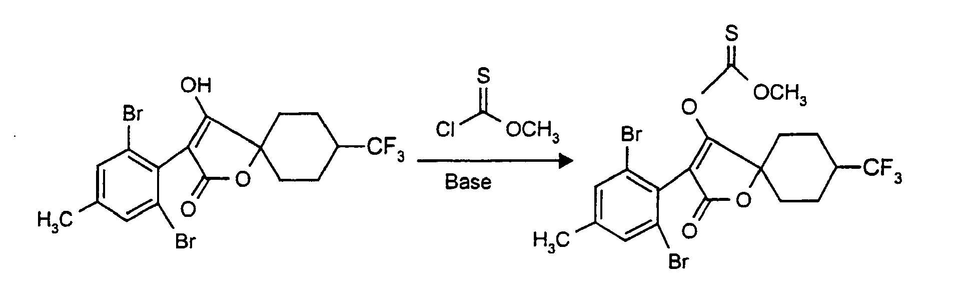

Verwendet man gemäß Verfahren (F) 2-[(2,4,6-Trimethyl)-phenyl]-5,5-(3-trifluormethyl-pentamethylendiyl)-pyrrolidin-2,4-dion und Methansulfonsäurechlorid als Ausgangsprodukt, so kann der Reaktionsverlauf durch folgendes Reaktionsschema wiedergegeben werden:

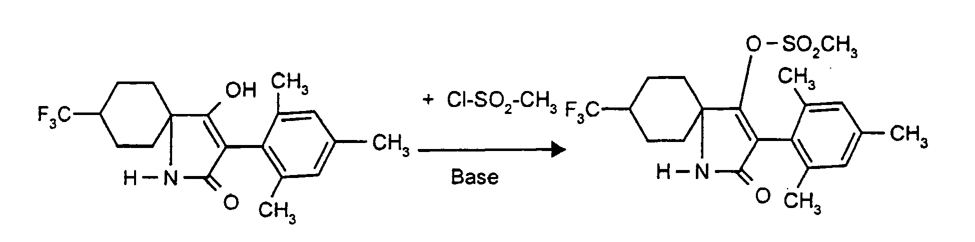

Verwendet man gemäß Verfahren (G) 2-[(4-Brom-2-chlor-6-methyl)-phenyl]-4-hydroxy-5,5-(3-trifluormethyl-pentamethylendiyl)-Δ³-dihydrofuran-2-on und Methanthio-phosphonsäurechlorid-(2,2,2-trifluorethylester) als Ausgangsprodukte, so kann der Reaktionsverlauf durch folgendes Reaktionsschema wiedergegeben werden:

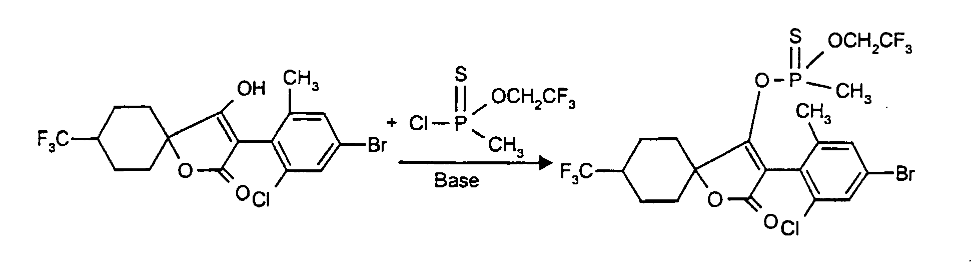

Verwendet man gemäß Verfahren (H) 3-[(2,4-Dichlor)-6-methylphenyl]-5,5-(3-tiifluormethyl-pentamethylendiyl)-pyrrolidin-2,4-dion und NaOH als Komponenten, so kann der Verlauf des erfindungsgemäßen Verfahrens durch folgendes Reaktionsschema wiedergegeben werden:

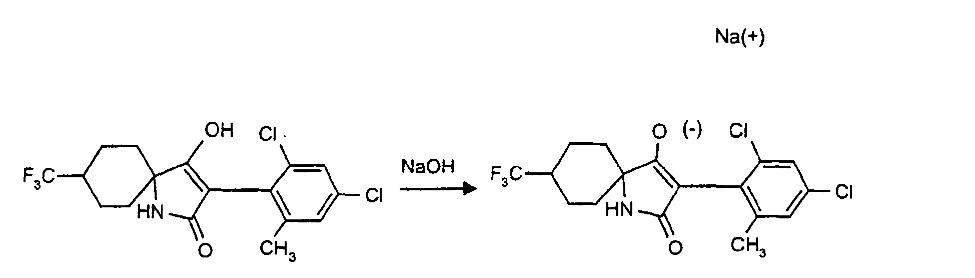

Verwendet man gemäß Verfahren (I) (Variante α) 3-[(2-Chlor-4-brom-5-methyl)-phenyl]-4-hydroxy-5,5-(3-trifluormethyl-pentamethylendiyl)-Δ³-dihydro-furan-2-on und Ethylisocyanat als Ausgangsprodukte, so kann der Reaktionsverlauf durch folgendes Reaktionsschema wiedergegeben werden:

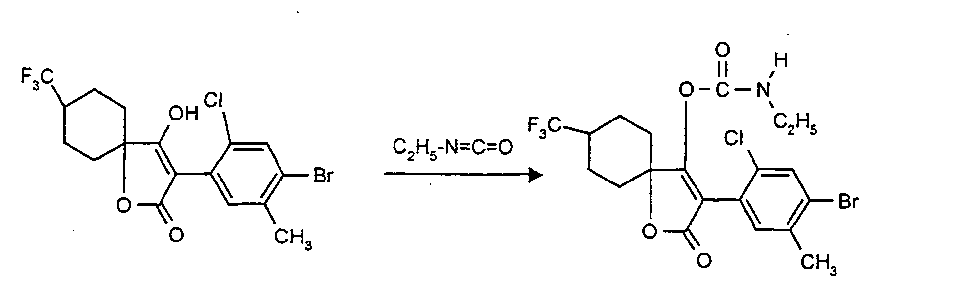

Verwendet man gemäß Verfahren (I) (Variante β) 3-[(2-Chlor-4,6-dimethyl)-phenyl]-5,5-(3-trifluormethyl-pentamethylendiyl)-pyrrolidin-2,4-dion und Dimethylcarbamidsäurechlorid als Ausgangsprodukte, so kann der Reaktionsverlauf durch folgendes Schema wiedergegeben werden:

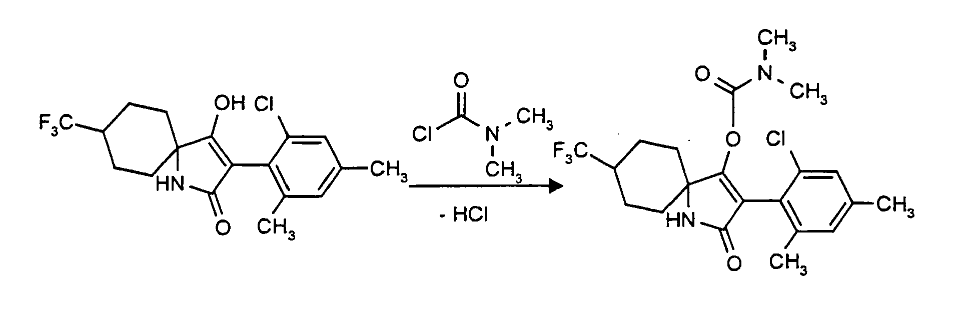

Die beim erfindungsgemäßen Verfahren (A) als Ausgangsstoffe benötigten Verbindungen der Formel (II) 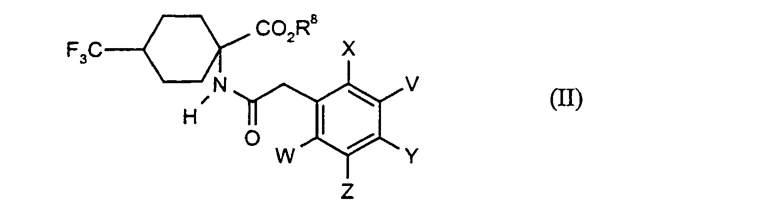 in welcher
V, W, X, Y, Z und R⁸ die oben angegebenen Bedeutungen haben,
sind neu.

Man erhält die Acylaminosäureester der Formel (II) beispielsweise, wenn man Aminosäurederivate der Formel (XIV) 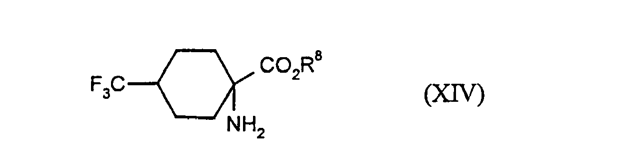 in welcher
R⁸ die oben angegebene Bedeutung hat,
mit substituierten Phenylessigsäurehalogeniden der Formel (XV) 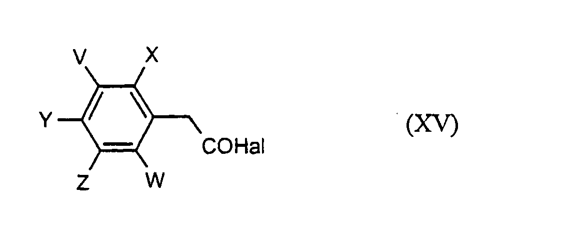 in welcher
V, W, X, Y und Z die oben angegebenen Bedeutungen haben und
Hal für Chlor oder Brom steht,
acyliert (Chem. Reviews 52, 237-416 (1953); Bhattacharya, Indian J. Chem. 6, 341-5, 1968)
oder wenn man Acylaminosäure der Formel (XVI) 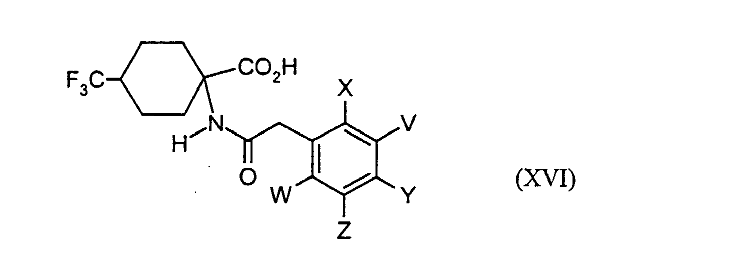 in welcher
V, W, X, Y und Z die oben angegebenen Bedeutungen haben,
verestert (Chem. Ind. (London) 1568 (1968)).

Die Verbindung der Formel (XVI) 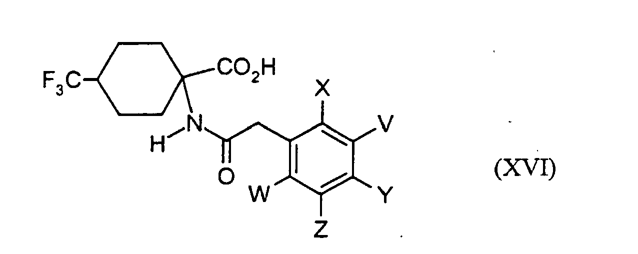 in welcher
V, W, X, Y und Z die oben angegebenen Bedeutungen haben,
sind neu.

Man erhält die Verbindungen der Formel (XVI) beispielsweise, wenn manl-Amino-4-trifluormethyl-cyclohexan-carbonsäure der Formel (XVII) 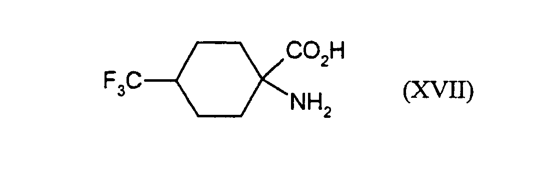 mit substituierten Phenylessigsäurehalogeniden der Formel (XV) 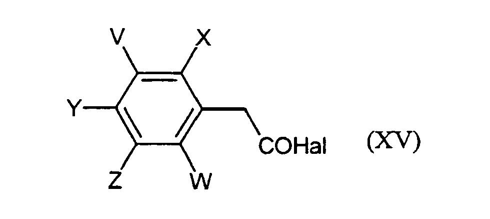 in welcher
- V, W, X, Y und Z: die oben angegebenen Bedeutungen haben und
- Hal: für Chlor oder Brom steht,

nach Schotten-Baumann acyliert (Organikum, VEB Deutscher Verlag der Wissenschaften, Berlin 1977, S. 505).

Die Verbindungen der Formel (XV) sind bekannt und lassen sich nach den in den eingangs zitierten Offenlegungsschriften bekannten Verfahren herstellen.

Die Verbindungen der Formel (XIV) und (XVII) sind neu und lassen sich nach bekannten Verfahren darstellen (siehe z.B. Compagnon, Ann. Chim. (Paris) [14] 5, S. 11-22, 23-27 (1970), L. Munday, J. Chem. Soc. 4372 (1961); J.T. Eward, C. Jitrangeri, Can. J. Chem. 53, 3339 (1975).

Die 1-Amino-4-trifluormethyl-cyclohexan-carbonsäure (XVII) ist im allgemeinen nach der Bucherer-Bergs-Synthese oder nach der Strecker-Synthese erhältlich und fällt dabei jeweils in unterschiedlichen Isomerenformen an. So erhält man unter den Bedingungen der Buclierer-Bergs-Synthese vorwiegend das Isomer (im folgenden der Einfachheit halber als β bezeichnet), in welchem die Trifluormethylgruppe und die Carboxylgruppe äquatorial stehen, während nach den Bedingungen der Strecker-Synthese vorwiegend das Isomer (im folgenden der Einfachheit halber als α bezeichnet) anfällt, bei der die Aminogruppe und die Trifluormethylgruppe äquatorial stehen. 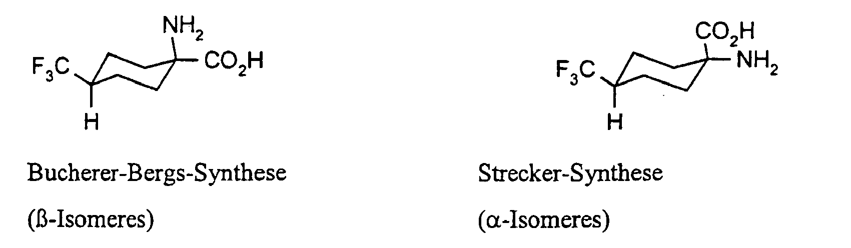 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 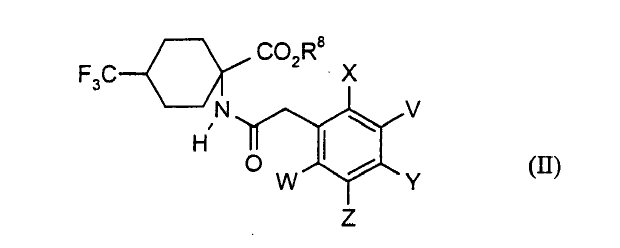 in welcher
- V, W, X, Y, Z und R⁸: die oben angegebenen Bedeutungen haben,
herstellen, wenn man 1-Amino-4-trifluormethyl-cyclohexan-carbonsäurenitril der Formel (XVIII) 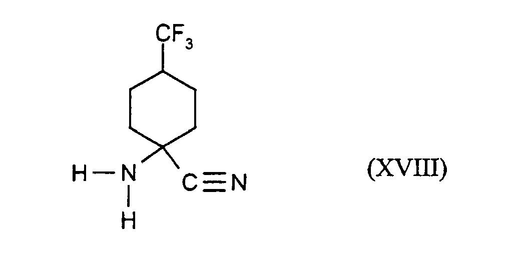 mit substituierten Phenylessigsäurehalogeniden der Formel (XV) 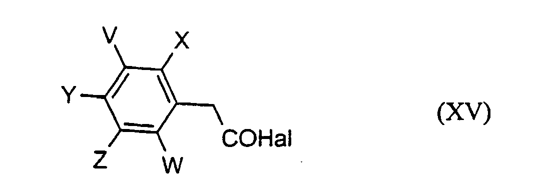 in welcher
V, W, X, Y, Z und Hal die oben angegebenen Bedeutungen haben,
zu Verbindungen der Formel (XIX) 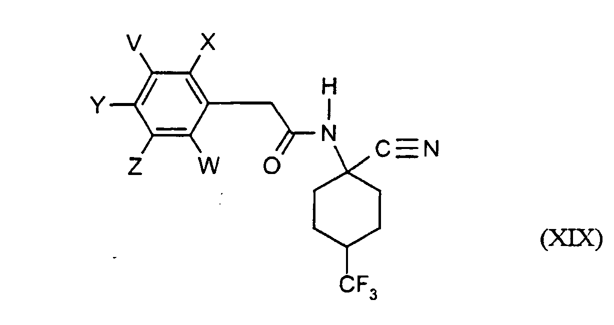 in welcher
V, W, X, Y und Z die oben angegebenen Bedeutungen haben,
umsetzt,
und diese anschließend einer sauren Alkoholyse unterwirft.

Die Verbindungen der Formel (XIX) sind ebenfalls neu. Die Verbindungen der Formel (XVIII) sind ebenfalls neu.

Die bei dem erfindungsgemäßen Verfahren (B) als Ausgangstoffe benötigten Verbindungen der Formel (III) 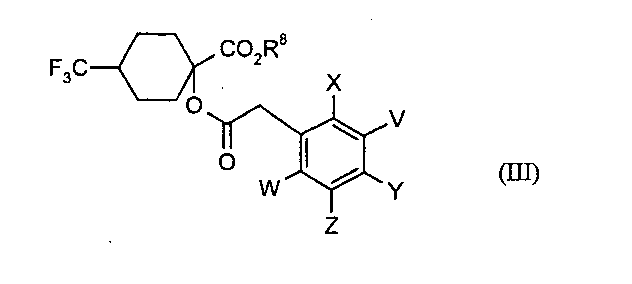 in welcher
V, W, X, Y, Z und R⁸ die oben angegebenen Bedeutungen haben,
sind neu.

Ausgenommen ist folgende Verbindung

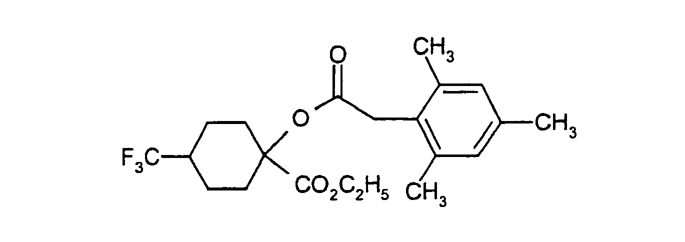

Sie lassen sich nach im Prinzip bekannten Methoden in einfacher Weise herstellen.

Man erhält die Verbindungen der Formel (III) beispielsweise, wenn man
1-Hydroxy-4-trifluormethyl-cyclohexan-carbonsäureester der Formel (XX) 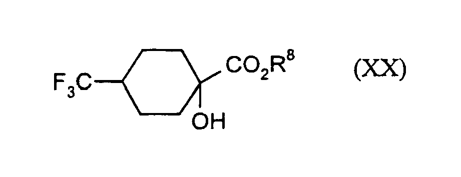 in welcher
- R⁸: die oben angegebene Bedeutung hat,
mit substituierten Phenylessigsäurehalogeniden der Formel (XV) 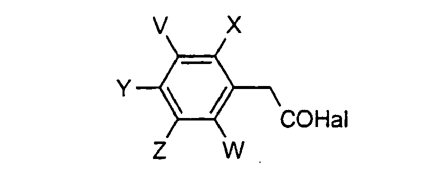 in welcher
V, W, X, Y, Z und Hal die oben angegebenen Bedeutungen haben,
acyliert (Chem. Reviews 52, 237-416 (1953)).

Die 1-Hydroxy-4-trifluormethyl-cyclohexyl-carbonsäureester der Formel (XX) sind teilweise neu. Man erhält sie beispielsweise, indem man 1-Hydroxy-4-trifluormethylcyclohexan-carbonsäurenitril in Gegenwart von Säuren, z.B. nach Pinner alkoholisiert (siehe Herstellungsbeispiel). Das Cyanhydrin erhält man beispielsweise durch Umsetzung von 4-Trifluormethyl-cyclohexan-1-on mit Blausäure.

Die zur Durchführung der erfindungsgemäßen Verfahren (C), (D), (E), (F), (G), (H) und (I) außerdem als Ausgangsstoffe benötigten Säurehalogenide der Formel (IV), Carbonsäureanhydride der Formel (V), Chlorameisensäureester oder Chlorameisensäurethioester der Formel (VI), Chlormonothioameisensäureester oder Chlordithioameisensäureester der Formel (VII), Sulfonsäurechloride der Formel (VIII), Phosphorverbindungen der Formel (IX) und Metallhydroxide, Metallalkoxide oder Amine der Formel (X) und (XI) und Isocyanate der Formel (XII) und Carbamidsäurechloride der Formel (XIII) sind allgemein bekannte Verbindungen der organischen bzw. anorganischen Chemie.

Die Verbindungen der Formeln (XV) sind darüber hinaus aus den eingangs zitierten Patentanmeldungen bekannt und/oder lassen sich nach den dort angegebenen Methoden herstellen.

Das Verfahren (A) ist dadurch gekennzeichnet, daß man Verbindungen der Formel (II), in welcher V, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -75°C und 200°C, vorzugsweise zwischen -50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich; die eine oder andere Komponente in einem größeren Überschuß (bis zu 3 Mol) zu verwenden.

Das Verfahren (B) ist dadurch gekennzeichnet, daß man Verbindungen der Formel (III), in welcher V, W, X, Y, Z und R⁸ die oben angegebenen Bedeutungen haben, in Gegenwart eines Verdünnungsmittels und in Gegenwart einer Base intramolekular kondensiert.

Als Verdünnungsmittel können bei dem erfindungsgemäßen Verfahren (B) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (B) innerhalb eines größeren Bereiches variiert werden. Im allgemeinen arbeitet man bei Temperaturen zwischen -75°C und 200°C, vorzugsweise zwischen -50°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (C_{α}) ist dadurch gekennzeichnet, daß man Verbindungen der Formeln (I-1-a) bis (I-2-a) jeweils mit Carbonsäurehalogeniden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C_{α}) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (C_{α}) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (C_{α}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C_{α}) werden die Ausgangsstoffe der Formeln (I-1-a) bis (I-2-a) und das Carbonsäurehalogenid der Formel (IV) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (C_{β}) ist dadurch gekennzeichnet, daß man Verbindungen der Formeln (I-1-a) bis (I-2-a) jeweils mit Carbonsäureanhydriden der Formel (V) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C_{β}) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (C_{β}) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (C_{β}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C_{β}) werden die Ausgangsstoffe der Formeln (I-1-a) bis (1-2-a) und das Carbonsäureanhydrid der Formel (V) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (D) ist dadurch gekennzeichnet, daß man Verbindungen der Formeln (I-1-a) bis (1-2-a) jeweils mit Chlorameisensäureestern oder Chlorameisensäurethiolestern der Formel (VI) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (D) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethylanilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (D) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (D) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (D) wird im allgemeinen unter Normaldruck durchgerührt.

Bei der Durchführung des erfindungsgemäßen Verfahrens (D) werden die Ausgangsstoffe der Formeln (I-1-a) bis (I-2-a) und der entsprechende Chlorameisensäureester bzw. Chlorameisensäurethiolester der Formel (VI) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (E) ist dadurch gekennzeichnet, daß man Verbindungen der Formeln (I-1-a) bis (I-2-a) jeweils mit Verbindungen der Formel (VII) in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formeln (I-1-a) bis (I-2-a) ca. 1 Mol Chlormonothioameisensäureester bzw. Chlordithioameisensäureester der Formel (VII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid, Essigsäureethylester oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-2-a) dar, kann auf den weiteren Zusatz von Säurebindemitteln verzichtet werden.

Als Basen können beim Verfahren (E) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, daß man Verbindungen der Formeln (I-1-a) bis (I-2-a) jeweils mit Sulfonsäurechloriden der Formel (VIII) gegebenenfalls in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formel (I-1-a) bis (I-2-a) ca. 1 Mol Sulfonsäurechlorid der Formel (VIII) bei -20 bis 150°C, vorzugsweise bei 0 bis 70°C um.

Das Verfahren (F) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Essigsäureethylester, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist dadurch gekennzeichnet, daß man Verbindungen der Formeln (I-1-a) bis (1-2-a) jeweils mit Phosphorverbindungen der Formel (IX) gegebenenfalls in Gegenwart eines Verdünnungsmittels und gegebenenfalls in Gegenwart eines Säurebindemittels umsetzt.

Beim Herstellungsverfahren (G) setzt man zum Erhalt von Verbindungen der Formeln (I-1-e) bis (I-2-e) auf 1 Mol der Verbindungen (I-1-a) bis (I-2-a), 1 bis 2, vorzugsweise 1 bis 1,3 Mol der Phosphorverbindung der Formel (IX) bei Temperaturen zwischen -40°C und 150°C, vorzugsweise zwischen -10 und 110°C um.

Das Verfahren (G) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise lurch Kristallisation, chromatographische Reinigung oder durch sogenanntes "Andestillieren", d.h. Entfernung der flüchtigen Bestandteile im Vakuum gereinigt.

Das Verfahren (H) ist dadurch gekennzeichnet, daß man Verbindungen der Formeln (I-1-a) bis (I-2-a) jeweils mit Metallhydroxiden bzw. Metallalkoxiden der Formel (X) oder Aminen der Formel (XI), gegebenenfalls in Gegenwart eines Verdünnungsmittels, umsetzt.

Als Verdünnungsmittel können bei dem erfindungsgemäßen Verfahren (H) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (H) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (I) ist dadurch gekennzeichnet, daß man Verbindungen der Formeln (I-1-a) bis (I-2-a) jeweils mit (Iα) Verbindungen der Formel (XII) gegebenenfalls in Gegenwart eines Verdünnungsmittels und gegebenenfalls in Gegenwart eines Katalysators oder (IB) mit Verbindungen der Formel (XIII) gegebenenfalls in Gegenwart eines Verdünnungsmittels und gegebenenfalls in Gegenwart eines Säurebindemittels umsetzt.

Bei Herstellungsverfahren (Iα) setzt man pro Mol Ausgangsverbindung der Formeln (I-1-a) bis (I-2-a) ca. 1 Mol Isocyanat der Formel (XII) bei 0 bis 100°C, vorzugsweise bei 20 bis 50°C um.

Das Verfahren (Iα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Iβ) setzt man pro Mol Ausgangsverbindung der Formeln (I-1-a) bis (I-2-a) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus cörporis, Haematopinus spp., Linognathus spp., Trichodectes spp., Damalinia spp..

Aus der Ordnung der Thysanoptera z.B. Hercinothrips femoralis, Thrips tabaci, Thrips palmi, Frankliniella occidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..

Aus der Ordnung der Siphonaptera z.B. Xenopsylla cheopis, Ceratophyllus spp..

Aus der Klasse der Arachnida z.B. Scorpio maurus, Latrodectus mactans, Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chloiphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-B-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, BromophosA, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Isazofos, Isofenphos, Isoxathion, Ivermectin,
Kempolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.. Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Omithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus und Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

Hautflügler wie
Sirex juvencus, Urocerus gigas, Urocerus gigas taignus, Urocerus augur.

Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-FungizidGemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, daß die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfemaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert.*-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[b]thiophencarbonsäurecyclohexylamid-S,S-dioxid, Dichlofluanid, Fluorfolpet, 3-Iod-2-propinyJ-butylcarbwnat, Tolylfluanid und Azole wie Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb;
oder herkömmliche Antifouling-Wirkstoffe wie
4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Erfindungsgemäße Verbindungen können in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera, Aegilops, Phalaris.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Steuen, Aufstreichen und bei Vermehrungsmaterial, insbesondere bei Samen, weiterhin durch ein- oder mehrschichtiges Umhüllen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise
Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinidon(-ethyl), Cinmethylin, Cinosulfuron, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epoprodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Flamprop(-isopropyl), Flamprop(-isopropyl-L), Flamprop(-methyl), Flazasulfuron, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Flüoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron, Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxazielomefone, Oxyfluorfen, Paraquat, Pelargonsäure, Pendimethalin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide; Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.
Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele

### Beispiel (I-1-a-1)

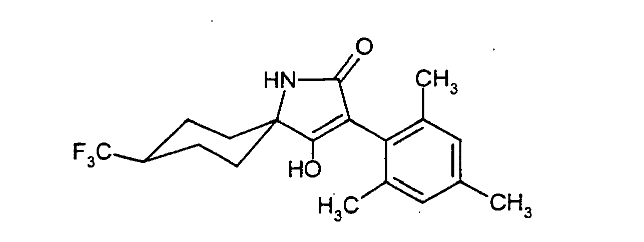

Zu 7,4 g Kalium-tert.-butylat in 30 ml wasserfreiem Dimethylformamid (DMF) tropft man bei 60°C 9 g der Verbindung gemäß Beispiel (II-1) in 20 ml wasserfreiem DMF und rührt 2 h bei 60°C. Dann gießt man die Reaktionslösung in 250 ml Eiswasser, säuert bei 0 bis 10°C mit konzentrierter Salzsäure auf pH 2 an, saugt ab und trocknet.

Das Rohprodukt wird in Methyl-tert.-butylether (MTBE)/n-Hexan aufgekocht, abgesaugt und getrocknet.

Ausbeute: 6,15 g (74 % der Theorie); Fp.: >250°C.

In Analogie zu Beispiel (I-1-a-1) und gemäß den allgemeinen Angaben zur Herstellung wurden folgende Verbindungen der Fomel (I-1-a) hergestellt.

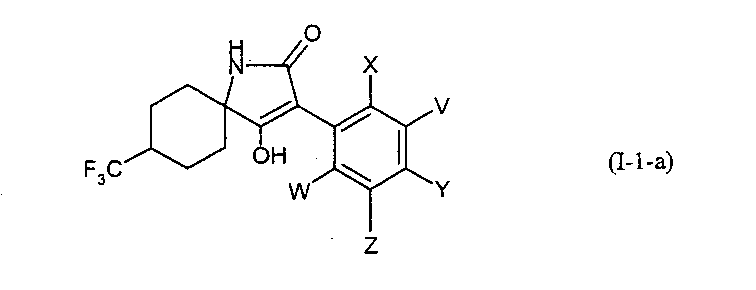

| Bsp.-Nr. | V | W | X | Y | Z | Fp°C | Isomer |
|---|---|---|---|---|---|---|---|
| I-1-a-2 | H | H | CH₃ | H | CH₃ | 245 | β |
| I-1-a-3 | H | H | CH₃ | CH₃ | CH₃ | 175 | β |
| I-1-a-4 | H | Cl | Cl | CH₃ | H | >250 | β |
| I-1-a-5 | CH₃ | CH₃ | CH₃ | CH₃ | H | >250 | β |
| I-1-a-6 | H | CH₃ | CH₃ | Cl | H | >248 | β |
| I-1-a-7 | H | H | Br | H | CH₃ | 212 | β |

### Beispiel (I-1-b-1)

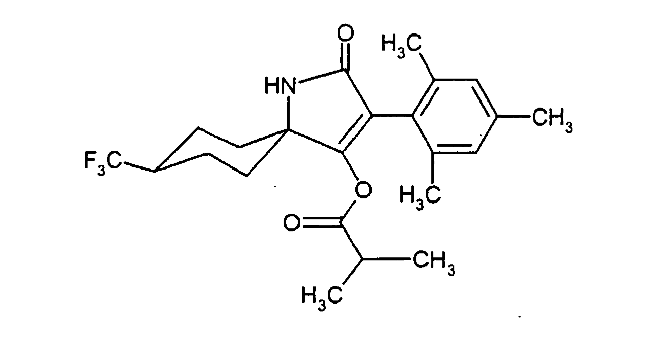

1,42 g der Verbindung gemäß Beispiel (I-1-a-1) werden in 40 ml wasserfreiem Essigsäureethylester vorgelegt, mit 0,62 ml (4,4 mmol) Triethylamin versetzt und unter Rückfluß 0,46 ml (0,0046 mol) Isobuttersäurechlorid in 5 ml wasserfreiem Essigsäureethylester zugetropft. Nach 16 h Rückfluß wird der Ansatz eingeengt, der Rückstand in Methylenchlorid aufgenommen, 2 x mit 30 ml 0,5 N NaOH gewaschen, getrocknet und eingedampft. Der Rückstand wird an Kieselgel mit Methylenchlorid/Essigsäureethylester 3:1 chromatographiert.

Ausbeute: 1,12 g (66 % der Theorie); Fp.: >240°C

In Analogie zu Beispiel (I-1-b-1) und gemäß den allgemeinen Angaben zur Herstellung wurden folgende Verbindungen der Fomel (I-1-b) hergestellt.

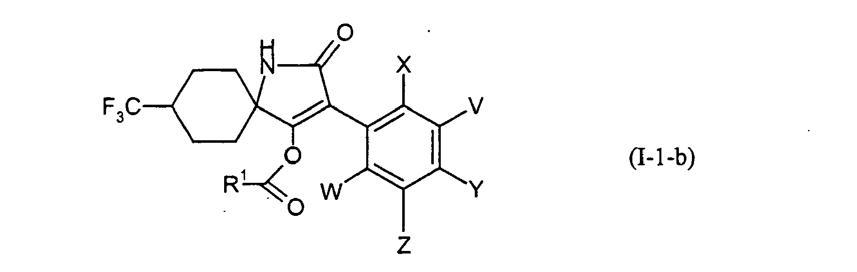

| Bsp.-Nr. | V | W | X | Y | Z | R¹ | Fp°C | Isomer |
|---|---|---|---|---|---|---|---|---|
| I-1-b-2 | H | H | CH₃ | H | CH₃ | i-C₃H₇ | >240 | β |
| I-1-b-3 | H | H | CH₃ | CH₃ | CH₃ | i-C₃H₇ | 209 | β |
| I-1-b-4 | CH₃ | CH₃ | CH₃ | CH₃ | H | i-C₃H₇ | 236 | β |

### Beispiel (I-1-c-1)

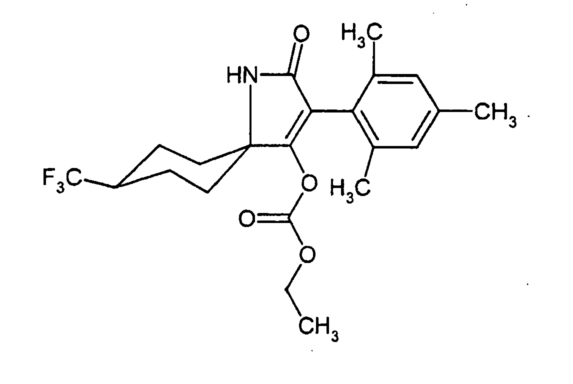

1,42 g der Verbindung gemäß Beispiel (I-1-a-1) werden in 40 ml wasserfreiem Methylenchlorid vorgelegt, mit 0,56 ml (4 mmol) Triethylamin versetzt und bei 10 bis 20°C 0,4 ml (4 mmol) Chlorameisensäureethylester in 5 ml wasserfreiem Methylenchlorid zugetropft. Die Reaktion wird mittels Dünnschichtchromatographie verfolgt.

Der Ansatz wird dann eingeengt, der Rückstand in Methylenchlorid aufgenommen, 2 x mit 30 ml 0,5N NaOH gewaschen, getrocknet und eingedampft. Der Rückstand wird an Kieselgel mit Methylenchlorid/Essigsäureethylester 3:1 chromatographiert.

Ausbeute: 1,03 g (60 % der Theorie); Fp.: = 208°C

In Analogie zu Beispiel (I-1-c-1) und gemäß den allgemeinen Angaben zur Herstellung wurden folgende Verbindungen der Fomel (I-1-c) hergestellt.

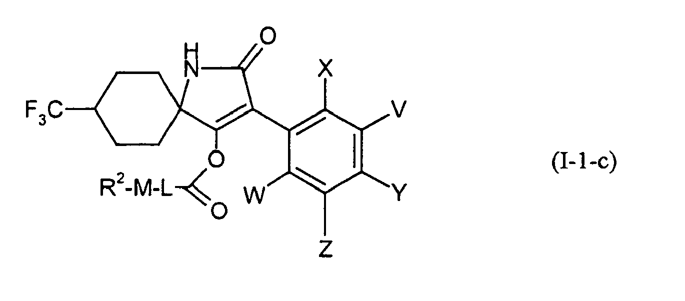

| Bsp.-Nr. | V | W | X | Y | Z | L | M | R² | Fp°C | Isomer |
|---|---|---|---|---|---|---|---|---|---|---|
| I-1-c-2 | H | H | CH₃ | H | CH₃ | O | O | C₂H₅ | 177 | β |
| I-1-c-3 | H | H | CH₃ | CH₃ | CH₃ | O | O | C₂H₅ | 196 | β |
| I-1-c-4 | H | CH₃ | CH₃ | CH₃ | H | O | O | C₆H₅-CH₂- | 167 | β |
| I-1-c-5 | H | CH₃ | Cl | Cl | H | O | O | C₂H₅ | 214 | β |
| I-1-c-6 | CH₃ | CH₃ | CH₃ | CH₃ | H | O | O | C₂H₅ | 183 | β |

### Beispiel II-1

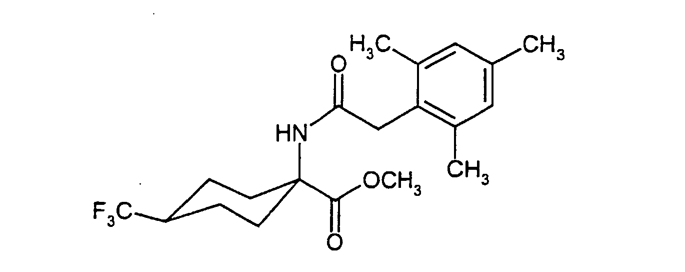

7,86 g 1-Amino-4-trifluormethyl-cyclohexan-1-carbonsäureethylester-hydrochlorid werden in 30 ml wasserfreiem Acetonitril vorgelegt, mit 13,8 g (0,1 mol) gemahlenem Kaliumcarbonat versetzt und bei 5 bis 10°C werden 5,9 g Mesitylenessigsäurechlorid in 10 mm wasserfreiem Acetonitril innerhalb von 10 min. zugetropft. Es wird 3 h bei Raumtemperatur gerührt.

Die Reaktionslösung wird in 200 ml Eiswasser gegossen und der pH-Wert geprüft. Nach Animpfung wird der Niederschlag abgesaugt und in Methylenchlorid aufgenommen, getrocknet und einrotiert. Der Rückstand wird an Kieselgel mit Methylenchlorid/Essigsäureethylester 10:1 chromatographisch gereinigt.

Ausbeute: 9,7 g (83,6 % der Theorie); Fp.: = 148°C

In Analogie zu Beispiel II-1 und gemäß den allgemeinen Angaben zur Herstellung wurden folgende Verbindungen der Fomel (II) hergestellt.

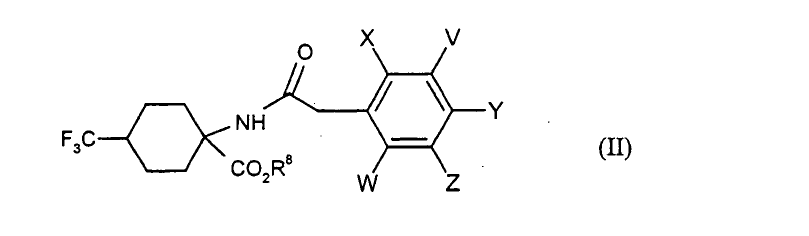

| Bsp.-Nr. | V | W | X | Y | Z | R⁸ | Fp°C | Isomer |
|---|---|---|---|---|---|---|---|---|
| In-2 | H | H | CH₃ | H | CH₃ | CH₃ | 138 | β |
| II-3 | H | H | CH₃ | CH₃ | CH₃ | CH₃ | 158 | β |
| II-4 | H | CH₃ | Cl | Cl | H | CH₃ | 145 | β |
| II-5 | CH₃ | CH₃ | CH₃ | CH₃ | H | CH₃ | 173 | β |
| II-6 | H | CH₃ | CH₃ | Cl | H | CH₃ | 181 | β |
| II-7 | H | H | Br | H | CH₃ | CH₃ | 139 | β |

### Beispiel XIV-1

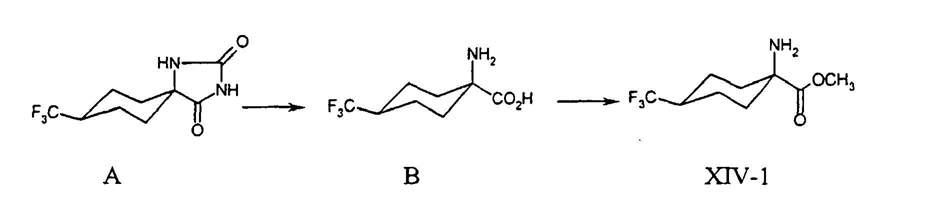

113,33 g (1,18 mol) Ammoniumcarbonat werden in 337 ml Wasser vorgelegt, 25,04 g (0,51 mol) Natriumcyanid werden zugegeben und 42,1 g (0,25 mol) Trifluormethyl-cyclohexanon-4-on in 379 ml Ethanol zugetropft. Die Reaktionslösung wird 10 h bei 55 bis 60°C gerührt. Anschließend wird der pH-Wert des Ansatzes mit konzentrierter Salzsäure auf pH 1-2 eingestellt und der Niederschlag abgesaugt und mit Wasser gewaschen (49,3 g =̂ 83 % der Theorie, Fp.: >250°C). 48,7 g des erhaltenen Hydantoins A werden in 270 ml 30 %iger KOH-Lösung suspendiert und 1 Tag bei Rückfluß unter Schutzgas gerührt. Der Reaktionsansatz wird bei 0 bis 10°C mit konzentrierter Salzsäure auf pH 5,2 bis 5,3 angesäuert, der Niederschlag wird abgesaugt. Das Rohprodukt B wird dann in 260 ml wasserfreiem Methanol vorgelegt und bei 0 bis 5°C wird 21,3 ml (0,253 mol) Thionylchlorid zugetropft. Die Suspension wird 30 min. bei 0°C gerührt, dann 8 h bei 40°C. Der Ansatz wird auf 0 bis 5°C abgekühlt, der Niederschlag abgesaugt, mit 15 ml MeOH gewaschen und eingeengt und in 40 ml Methyl-tert.-butylether aufgekocht, abgekühlt und der Niederschlag abgesaugt und getrocknet. Man erhält die Verbindung der Formel XIV-1.

Ausbeute: 42,46 g (95 % der Theorie), Fp.: 183°C.

### Beispiel I-2-a-1

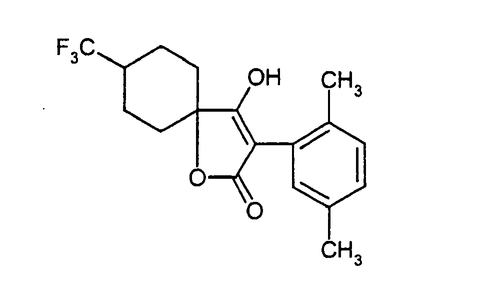

10 mmol der Verbindung gemäß Beispiel III-1 werden in 5 ml wasserfreiem DMF gelöst und langsam werden 12 ml 1M.KOtBu-Lösung in DMF zugetropft und 48 h bei Raumtemperatur gerührt. Der Ansatz wird einrotiert, der Rückstand in Wasser gelöst und mit verdünnter Salzsäure angesäuert und 2 h gerührt. Der Niederschlag wird abgesaugt und getrocknet.

Ausbeute: 2,59 g (76 % der Theorie), Fp.: 200 bis 202°C.

In Analogie zu Beispiel (I-2-a-1) und gemäß den allgemeinen Angaben zur Herstellung wurden folgende Verbindungen der Fomel (I-2-a) hergestellt.

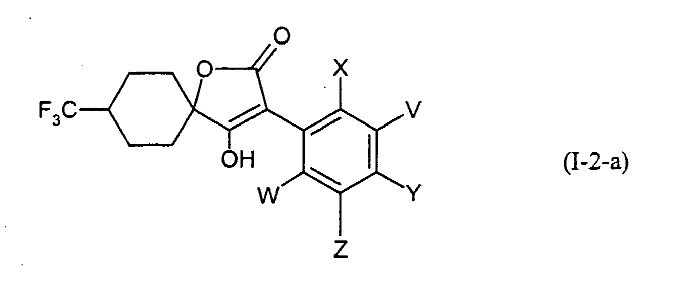

| Bsp.-Nr. | V | W | X | Y | Z | Fp. °C |
|---|---|---|---|---|---|---|
| I-2-a-2 | H | H | CH₃ | CH₃ | CH₃ | 212-214 |
| I-2-a-3 | CH₃ | CH₃ | CH₃ | CH₃ | H | 159-161 |

### Beispiel I-2-b-1

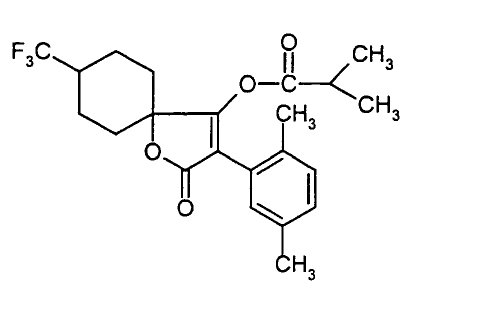

1,02 g (3 mmol) der Verbindung gemäß Beispiel I-2-a-1 werden in 20 ml wasserfreiem Dichlormethan vorgelegt, mit 0,32 g (3,2 mmol) Triethylamin versetzt und unter Rückfluß 0,33 g (3,1 mmol) Isobuttersäurechlorid in 5ml wasserfreiem Dichlormethan zugetropft. Nach 16 h Rückfluß wird der Ansatz eingeengt, der Rückstand in Dichlormethan aufgenommen, mit 0,5 N NaOH gewaschen, getrocknet und eingedampft. Der Rückstand wird chromatographisch gereinigt.

Ausbeute: 1,11 g (90 % der Theorie), Öl.

¹H-NMR (300 MHz, CDCl₃): δ = 1,05 (d, 6H, CH(CH₃)₂), 2,22, 2,29 (2s, 6H, Ph-CH₃), 2,61 (m, 1H, CH(CH₃)₂), 6,89 (s(b), 1H, Ph-6H), 7,05-7,15 (m, 2H, Ph-3H, Ph-4-H) ppm.

In Analogie zu Beispiel (I-2-b-1) und gemäß den allgemeinen Angaben zur Herstellung wurden folgende Verbindungen der Fomel (1-2-b) hergestellt.

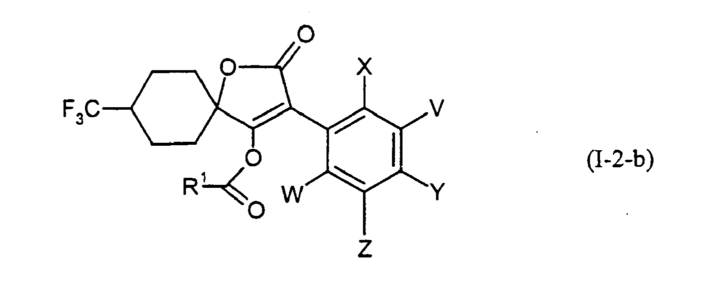

| Bsp.-Nr. | V | W | X | Y | Z | R¹ | Fp°C |
|---|---|---|---|---|---|---|---|
| I-2-b-2 | H | H | CH₃ | CH₃ | CH₃ | i-C₃H₇ | Öl |
| I-2-b-3 | CH₃ | CH₃ | CH₃ | CH₃ | H | i-C₃H₇ | Öl |

### Beispiel III-1

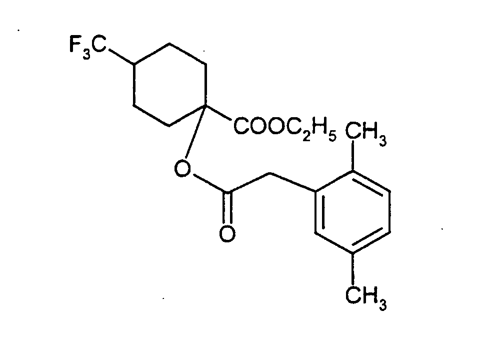

1,83 g (10 mmol) 2,5-Dimethylphenylessigsäurechlorid und 2,4 g (10 mmol) 1-Hydroxy-4-trifluormethylcyclohexan-1-carbonsäureethylester werden bei 140°C 8 h gerührt.

Das Produkt wurde nach GC/MS-Analyse ohne weitere Reinigung zur Herstellung des Beispiels 1-2-a-1 verwendet.

In Analogie zu Beispiel III-1 und gemäß den allgemeinen Angaben zur Herstellung wurden folgende Verbindungen der Fomel (III) hergestellt.

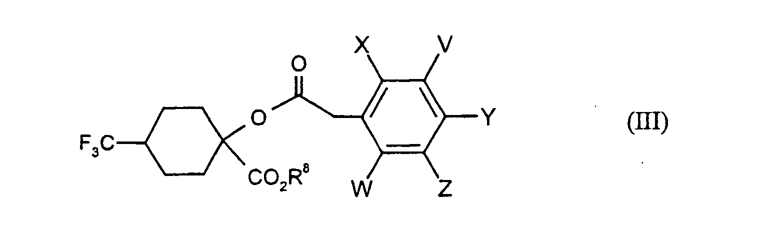

| Bsp.-Nr. | V | W | X | Y | Z | R⁸ | Fp°C |
|---|---|---|---|---|---|---|---|
| III-2* | H | H | CH₃ | CH₃ | CH₃ | C₂H₅ | Öl |
| III-3* | CH₃ | CH₃ | CH₃ | CH₃ | H | C₂H₅ | Öl |

| | | | | | | | |
|---|---|---|---|---|---|---|---|
| * Die Verbindungen wurden nach GC/MS-Analyse ohne weitere Aufreinigung zur Herstellung von Verbindungen der Formel I-2-a verwendet. | | | | | | | |

### Beispiel XX-1

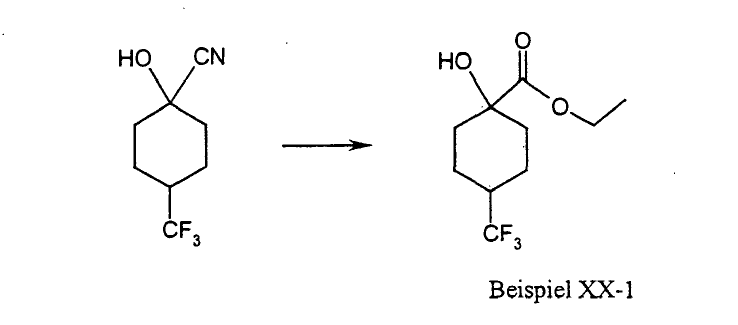

55 g (0,284 mol) der oben gezeigten Verbindung werden in 500 ml Ethanol gelöst und bei -20°C mit Salzsäure gesättigt. Es wird 2 h bei 0°C gerührt und der Ansatz innerhalb von 8 h auf Raumtemperatur gebracht. Es wird entgast, eingeengt und der Rückstand wird in 500 ml Eiswasser aufgenommen, 1 h gerührt und anschließend mit 500 ml Dichlormethan extrahiert. Die organische Phase wird getrocknet und eingeengt.

Ausbeute: 60 g (88 % der Theorie), Kp (0,05 mbar) = 56°C

### Anwendungsbeispiele

### Beispiel A

### Myzus-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt und man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele überlegene Wirksamkeit gegenüber dem Stand der Technik:

**Tabelle A**

| Pflanzenschädigende Insekten | | |
|---|---|---|
| Myzus-Test | | |
| Wirkstoffe Beispiel-Nr. | Wirkstoffkonzentration in ppm | Abtötungsgrad in % nach 6^{d} |
| I-b-25 bekannt aus EP-A-596 298 | 1000 | 0 |
| I-1-b-1 erfindungsgemäß | 1000 | 95 |
| I-c-24 bekannt aus EP-A-596 298 | 1000 | 0 |
| I-1-c-1 erfindungsgemäß | 1000 | 100 |
| I-1-a-3 bekannt aus WO 97/36868 | 100 | 80 |
| I-1-a-5 erfindungsgemäß | 100 | 100 |
| I-1-a-1 bekannt aus WO 97/01535 | 1000 | 0 |
| I-1-a-3 erfindungsgemäß | 1000 | 80 |
| I-1-b-2 bekannt aus WO 97/01535 | 100 | 70 |
| I-1-b-3 erfindungsgemäß | 100 | 95 |
| I-2-a-2 bekannt aus WO 97/01535 | 1000 | 60 |
| I-2-a-2 erfindungsgemäß | 1000 | 100 |

### Beispiel B

### Phaedon-Larven-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigen z.B. die folgenden Verbindungen der Herstellungsbeispiele überlegene Wirksamkeit gegenüber dem Stand der Technik:

**Tabelle B**

| Pflanzenschädigende Insekten | | |
|---|---|---|
| Phaedon-Larven-Test | | |
| Wirkstoffe Bsp.-Nr. | Wirkstoffkonzentration in ppm | Abtötungsgrad in % nach 7^{d} |
| I-b-25 Bekannt aus EP-A 596 298 | 100 | 60 |
| I-1-b-1 Erfindungsgemäß | 100 | 100 |
| I-1-b-4 Bekannt aus WO 98/05638 | 200 | 10 |
| I-1-b-2 Erfindungsgemäß | 200 | 100 |
| I-1-b-4 Bekannt aus WO 97/36868 | 1000 | 0 |
| I-1-b-4 Erfindungsgemäß | 1000 | 80 |
| I-1-b-2 Bekannt aus WO 97/01535 | 1000 | 80 |
| I-1-b-3 Erfindungsgemäß | 1000 | 100 |

### Beispiel D

### Aphis gossypii-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschten Konzentration.

Baumwollblätter (Gossypium hirsutum), die stark an der Baumwollblattlaus (Aphis gossypii) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z.B. die folgende Verbindung der Herstellungsbeispiele überlegene Wirksamkeit gegenüber dem Stand der Technik:

**Tabelle D**

| Pflanzenschädigende Insekten | | |
|---|---|---|
| Aphis gossypii-Test | | |
| Wirkstoffe Bsp.-Nr. | Wirkstoffkonzentration in ppm | Abtötungsgrad in % nach 6^{d} |
| I-1-b-4 bekannt aus WO 98/05638 | 200 | 0 |
| I-1-b-2 erfindungsgemäß | 200 | 75 |

### Beispiel E

### Tetranychus-Test (OP-resistent/Tauchbehandlung)

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigt z.B. die folgende Verbindung der Herstellungsbeispiele überlegene Wirksamkeit gegenüber dem Stand der Technik:

**Tabelle E**

| Pflanzenschädigende Milben | | |
|---|---|---|
| Tetranychus-Test (OP-resistent/Tauchbehandlung) | | |
| Wirkstoffe Bsp.-Nr. | Wirkstoffkonzentration in ppm | Abtötungsgrad in % nach 7^{d} |
| I-1-b-4 bekannt aus WO 97/36868 | 10 | 0 |
| I-1-b-4 erfindungsgemäß | 10 | 60 |

### Beispiel F

### Meloidogyne-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larvensuspension und Salatsamen gefiillt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigt z.B. die folgende Verbindung der Herstellungsbeispiele überlegene Wirksamkeit gegenüber dem Stand der Technik:

**Tabelle F**

| Pflanzenschädigende Nematoden | | |
|---|---|---|
| Meloidogyne-Test | | |
| Wirkstoffe Bsp.-Nr. | Wirkstoffkonzentration in ppm | Abtötungsgrad in % nach 7^{d} |
| I-2-a-7 bekannt aus WO 98/05638 | 20 | 0 |
| I-2-a-1 Erfindungsgemäß | 20 | 90 |

### Beispiel G

### Grenzkonzentrations-Test / Wurzelsystemische Wirkung

### Testinsekt: Myzus persicae

| | |
|---|---|
| Lösungsmittel: | 4 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche im ppm (mg/ml) angegeben wird. Man füllt den behandelten Boden in 250 ml Töpfe und bepflanzt diese vorgekeimten Ackerbohnen. Der Wirkstoff kann so von den Pflanzenwurzeln aus dem Boden aufgenommen und in die Blätter transportiert werden.

Für den Nachweis des wurzelsystemischen Effektes werden nach 7 Tagen die Pflanzen mit den oben genannten Testtieren besetzt und nach weiteren 8 Tagen erfolgt die Auswertung durch Zählen oder Schätzen der toten Tiere. Aus den Abtötungszahlen wird die wurzelsystemische Wirkung des Wirkstoffes abgeleitet. Sie ist 100 %, wenn alle Testtiere abgetötet sind und 0 %, wenn noch genau so viele Testinsekten leben wie bei der unbehandelten Kontrolle.

Wirkstoffe, Aufwandmengen und Resultate gehen aus der nachfolgenden Tabelle hervor:

**Tabelle G**

| Wurzelsystemisch | |
|---|---|
| Myzus persicae | |
| Wirkstoffe Bsp.-Nr. | Abtötungsgrad in % bei Wirkstoffkonzentrationen in ppm |
| Ia-11 bekannt aus EP-A-596 298 | 20 ppm = 100 % |
| I-1-a-1 Erfindungsgemäß | 1,25 ppm = 100 % |

## Patentansprüche

1. Verbindungen der Formel (I) 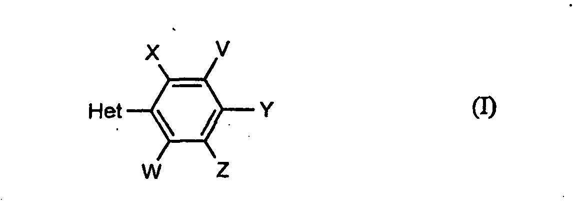 in welcher
V für Wasserstoff, Halogen, Alkyl oder Alkoxy steht,
W für Wasserstoff, Cyano, Nitro, Halogen, Alkyl, Alkenyl, Alkinyl, Alkoxy, Halogenalkyl, Halogenalkoxy, jeweils gegebenenfalls substituiertes Phenyl, Phenoxy, Phenylthio, Phenylalkoxy oder Phenylalkylthio steht,
X für Halogen, Alkyl, Alkenyl, Alkinyl, Alkoxy, Halogenalkyl, Halogenalkoxy, Cyano, Nitro, jeweils gegebenenfalls substituiertes Phenyl, Phenoxy, Phenylthio, Phenylalkyloxy oder Phenylalkylthio steht,
Y für Wasserstoff, Halogen, Alkyl, Alkoxy, Halogenalkyl, Halogenalkoxy, Cyano oder Nitro steht,
Z für Wasserstoff, Halogen,Alkyl, Alkoxy, Halogenalkyl, Halogenalkoxy, Hydroxy, Cyano, Nitro oder jeweils gegebenenfalls substituiertes Phenoxy, Phenylthio, 5- oder 6-gliedriges Hetaryloxy, 5- oder 6-gliedriges Hetarylthio, Phenylalkyloxy oder Phenylalkylthio steht,
Het für eine der Gruppen 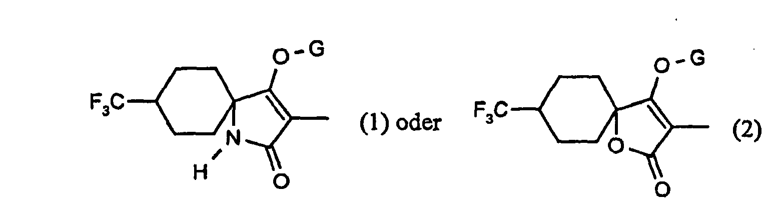 steht,
worin
G für Wasserstoff (a) oder für eine der Gruppen 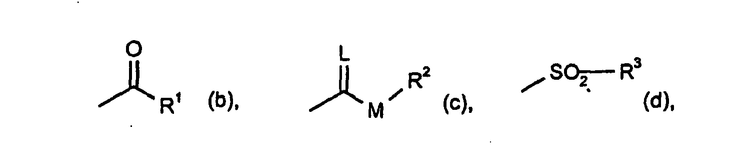 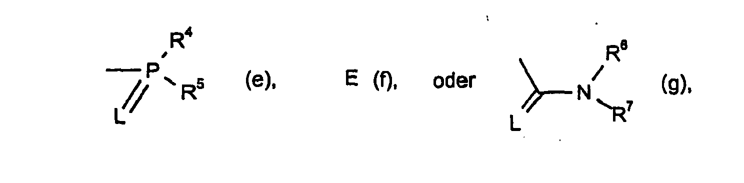 steht,
worin
E für ein Metallion oder ein Ammoniumion steht,
L für Sauerstoff oder Schwefel steht,
M für Sauerstoff oder Schwefel steht,
R¹ für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebe- nenfalls substituiertes Phenyl, Phenylalkyl, Hetaryl, Phenoxyalkyl oder Hetaryloxyalkyl steht,
R² für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Diakylamino, Alkylthio, Alkenylthio oder Cycloalkylthio oder für jeweils gegebe- nenfalls substituiertes Phenyl, Benzyl, Phenoxy oder Phenylthio stehen,
R⁶ und R⁷ unabhängig voneinander für Wasserstoff, für jeweils gegebe- nenfalls durch Halogen oder Cyano substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden,
ausgenommen die Verbindung I-a-79 aus EP 528 156

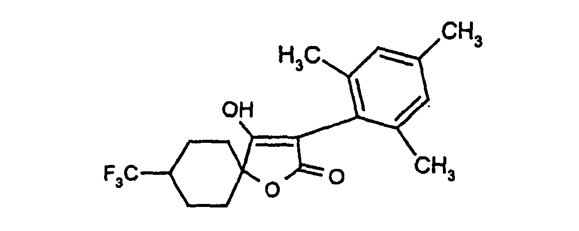

2. Verbindungen der Formel (I) gemäß Anspruch 1, in welcher
V für Wasserstoff, Halogen, C₁-C₆-Alkyl oder C₁-C₆-Alkoxy steht.
W für Wasserstoff Nitro, Cyano, Halogen, C₁-C₆-Alkyl, C₂-C₆-Alkenyl oder C₂-C₆-Alkinyl, C₁-C₆-Alkoxy, C₁-C₄-Halogenalkyl, C₁-C₄- Halogenalkoxy oder jeweils gegebenenfalls durch Halogen, C₁-C₆- Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl- C₁-C₄-alkoxy oder Phenyl-C₁-C₄-alkylthio steht.
X für Halogen, C₁-C₆-Alkyl, C₂-C₆-Alkenyl, C₂-C₆-Alkinyl, C₁-C₆- Alkoxy, C₁-C₄-Halogenalkyl, C₁-C₄-Halogenalkoxy, Cyano, Nitro oder jeweils gegebenenfalls durch Halogen, C₁-C₆-Alkyl, C₁-C₆- Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄- alkoxy oder Phenyl-C₁-C₄-alkylthio steht.
Y für Wasserstoff, Halogen, C₁-C₆-Alkyl, C₁-C₆-Alkoxy, C₁-C₄-Halogenalkyl, C₁-C₄-Halogenalkoxy, Cyano oder Nitro steht.
Z für Wasserstoff, Halogen, C₁-C₆-Alkyl, C₁-C₆-Alkoxy, C₁-C₄- Halogenalkyl, C₁-C₄-Halogenalkoxy, Hydroxy, Cyano, Nitro oder jeweils gegebenenfalls durch Halogen, C₁-C₄-Alkyl, C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyloxy, Phenyl-C₁-C₄-alkyloxy oder Phenyl- C₁-C₄-alkylthio steht oder
Het für eine der Gruppen 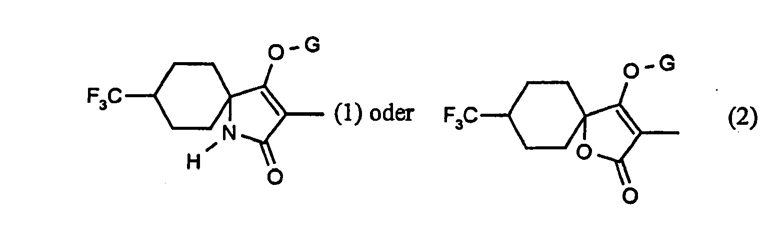 steht,
G für Wasserstoff (a) oder für eine der Gruppen 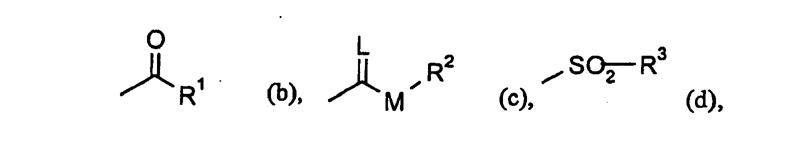 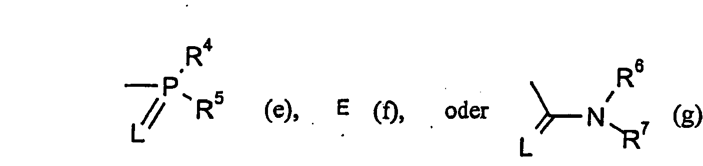 steht,
in welchen
E für ein Metallion oder ein Ammoniumion steht,
L für Sauerstoff oder Schwefel steht und
M für Sauerstoff oder Schwefel steht.
R¹ für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈- Alkylthio-C₁-C₈-alkyl oder Poly-C₁-C₈-akoxy-C₁-C₈-akyl oder für gegebenenfalls durch Halogen, C₁-C₆-Alkyl oder C₁-C₆-Alkoxy substituiertes C₃-C₈-Cycloalkyl steht, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁- C₆-Alkoxy, C₁-C₆-Halogenalkyl, C₁-C₆-Halogenalkoxy, C₁-C₆- Alkylthio oder C₁-C₆-Alkylsulfonyl substituiertes Phenyl,
für gegebenenfalls durch Halogen, Nitro, Cyano, C₁-C₆-Alkyl, C₁- C₆-Alkoxy, C₁-C₆-Halogenalkyl oder C₁-C₆-Halogenalkoxy substi- tuiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substitu- iertes 5- oder 6-gliedriges Hetaryloxy-C₁-C₆-alkyl mit ein oder zwei Heteroatomen aus der Reihe Sauerstoff, Schwefel und Stickstoff steht.
R² für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Akoxy substituiertes C₃-C₈-Cycloalkyl oder
für jeweils gegebenenfalls durch Halogen, Cyano, Nitro, C₁-C₆- Alkyl, C₁-C₆-Alkoxy, C₁-C₆-Halogenalkyl oder C₁-C₆-Halogen- alkoxy substituiertes Phenyl oder Benzyl steht.
R³ für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht.
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di- (C₁-C₈-alkyl)amino, C₁-C₈-Alkylthio oder C₃-C₈-Alkenylthio oder für jeweils gegebenenfalls durch Halogen, Nitro, Cyano, C₁-C₄- Alkoxy, C₁-C₄-Halogenalkoxy, C₁-C₄-Alkylthio, C₁-C₄-Halogen- alkylthio, C₁-C₄-Alkyl oder C₁-C₄-Halogenalkyl substituiertes Phenyl, Phenoxy oder Phenylthio stehen.
R⁶ und R⁷ unabhängig voneinander für Wasserstoff, für jeweils gegebe- nenfalls durch Halogen oder Cyano substituiertes C₁-C₈-Alkyl, C₃- C₈-Cycloalkyl, C₁-C₈-Alkoxy, C₃-C₈-Alkenyl oder C₁-C₈-Alkoxy- C₂-C₈-alkyl, für jeweils gegebenenfalls durch Halogen, C₁-C₈-Alkyl, C₁-C₈-Halogenakyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls ein Methylengruppe durch Sauerstoff oder Schwefel ersetzt ist, ausgenommen die Verbindung I-a-75 aus EP 528 156

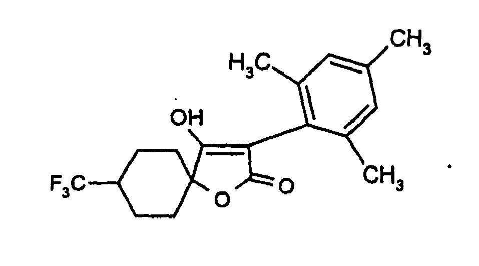

3. Verbindungen der Formel (I) gemäß Anspruch 1, in welcher
V für Wasserstoff, Fluor, Chlor, Brom, C₁-C₄-Alkyl oder C₁-C₄-Alkoxy steht.
W für Wasserstoff, Fluor, Chlor, Brom, C₁-C₄-Alkyl, C₁-C₄-Alkoxy, C₁-C₂-Halogenalkyl oder C₁-C₂-Halogenalkoxy steht.
X für Fluor, Chlor, Brom, C₁-C₄-Alkyl, C₁-C₄-Alkoxy, C₁-C₂-Halo- genalkyl, C₁-C₂-Halogenalkoxy, Cyano oder Nitro steht.
Y für Wasserstoff, Fluor, Chlor, Brom, C₁-C₄-Alkyl, C₁-C₄-Alkoxy, C₁-C₂-Halogenalkyl, C₁-C₂-Halogenalkoxy, Cyano oder Nitro steht.
Z für Wasserstoff, Fluor, Chlor, Brom, C₁-C₄-Alkyl, C₁-C₄-Allcoxy, C₁-C₂-Halogenalkyl, C₁-C₂-Halogenalkoxy, Hydroxy, Cyano, Nitro oder jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenoxy oder Benzyloxy steht.
Het für eine der Gruppen 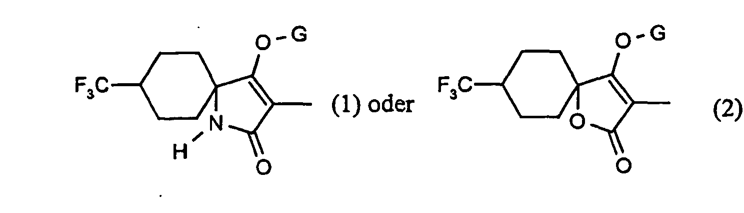 steht,
G für Wasserstoff (a) oder für eine der Gruppen 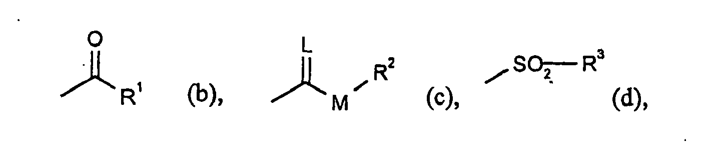 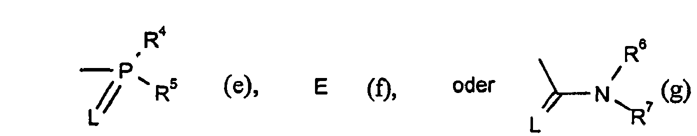 steht,
in welchen
E für ein Metallion oder ein Ammoniumion steht,
L für Sauerstoff oder Schwefel steht und
M für Sauerstoff oder Schwefel steht.
R¹ für jeweils gegebenenfalls durch Fluor oder Chlor substituiertes C₁- C₁₆-Alkyl, C₂-C₁₆-Alkenyl, C₁-C₆-Alkoxy-C₁-C₆-alkyl, C₁-C₆- Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄- Alkyl, C₁-C₄-Alkoxy, Ci-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄- Alkoxy, C₁-C₃-Halogenalkyl oder C₁-C₃-Halogenalkoxy substitu- iertes Phenyl-C₁-C₄-alkyl,
für jeweils gegebenenfalls durch Fluor, Chlor, Brom oder C₁-C₄- Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl sub- stituiertes Phenoxy-C₁-C₅-alkyl oder
für jeweils gegebenenfalls durch Fluor, Chlor, Brom, Amino oder C₁- C₄-Alkyl substituiertes Pyridyloxy-C₁-C₅-alkyl, Pyrimidyloxy-C₁- C₅-alkyl oder Thiazolyloxy-C₁-C₅-alkyl steht.
R² für jeweils gegebenenfalls durch Fluor oder Chlor substituiertes C₁- C₁₆-Alkyl, C₂-C₁₆-Alkenyl, C₁-C₆-Alkoxy-C₂-C₆-alkyl oder Poly- C₁-C₆-alkoxy-C₂-C₆-alkyl,
für gegebenenfalls durch Fluor, Chlor, C₁-C₄-Alkyl oder C₁-C₄- Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃Halogenalkyl oder C₁-C₃-Halo- genalkoxy substituiertes Phenyl oder Benzyl steht.
R³ für gegebenenfalls durch Fluor oder Chlor substituiertes C₁-C₆-Alkyl oder jeweils gegebenenfalls durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl steht.
R⁴ und R⁵ unabhängig voneinander für jeweils gegebenenfalls durch Fluor oder Chlor substituiertes C₁-C₆-Alkyl, C₁-C₆-Alkoxy, C₁-C₆- Alkylamino, Di-(C₁-C₆-alkyl)amino, C₁-C₆-Alkylthio oder C₃-C₄- Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkyl- thio, C₁-C₃-Halogenalkylthio, C₁-C₃-Alkyl oder C₁-C₃-Halogen- alkyl substituiertes Phenyl, Phenoxy oder Phenylthio stehen.
R⁶ und R⁷ unabhängig voneinander für Wasserstoff, für jeweils gege- benenfalls durch Fluor oder Chlor substituiertes C₁-C₆-Alkyl, C₃-C₆- Cycloalkyl, C₁-C₆-Alkoxy, C₃-C₆-Alkenyl oder C₁-C₆-Alkoxy-C₂- C₆-alkyl, für jeweils gegebenenfalls durch Fluor, Chlor, Brom, C₁- C₅-Halogenakyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Akylenrest stehen, in welchem ge- gebenenfalls eine Methylengruppe durch Sauerstoff oder Schwefel ersetzt ist,
ausgenommen die Verbindung I-a-75 aus EP 528 156

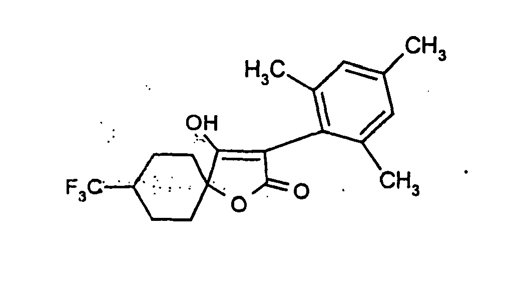

4. Verbindungen der Formel (I) gemäß Anspruch 1, in welcher
V für Wasserstoff, Fluor, Chlor, Brom, Methyl, Ethyl, Methoxy oder Ethoxy steht.
W für Wasserstoff, Fluor, Chlor, Brom, Methyl, Ethyl, Propyl, Methoxy oder Ethoxy steht.
X für Fluor, Chlor, Brom, Methyl, Ethyl, Propyl, iso-Propyl, Methoxy, Ethoxy, Propoxy, iso-Propoxy, Trifluormethyl, Trifluormethoxy, Difluormethoxy oder Cyano steht.
Y für Wasserstoff, Fluor, Chlor, Brom, Methyl, Ethyl, Propyl, iso- Propyl, tert.-Butyl, Methoxy, Ethoxy, Propoxy, iso-Propoxy, Tri- fluormethyl, Trifluormethoxy, Difluormethoxy, Cyano oder Nitro steht.
Z für Wasserstoff, Fluor, Chlor, Brom, Methyl, Ethyl, Propyl, iso- Propyl, tert.-Butyl, Methoxy, Ethoxy, Propoxy, iso-Propoxy, Trifluormethyl, Trifluormethoxy, Difluormethoxy, Cyano oder Nitro steht.
Het für eine der Gruppen 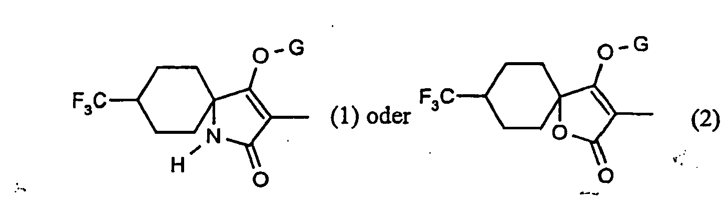 steht,
G für Wasserstoff (a) oder für eine der Gruppen 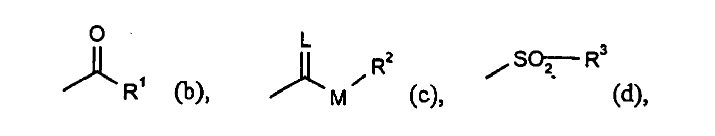 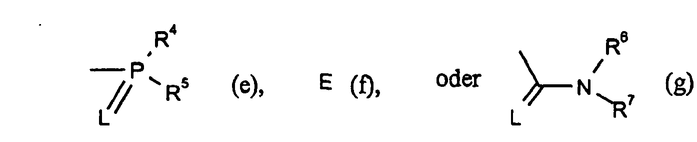 steht,
in welchen
E für ein Metallion oder ein Ammoniumion steht,
L für Sauerstoff oder Schwefel steht und
M für Sauerstoff oder Schwefel steht.
R¹ für jeweils gegebenenfalls durch Fluor oder Chlor substituiertes C₁- C₁₄-Alkyl, C₂-C₁₄-Alkenyl, C₁-C₄-Alkoxy-C₁-C₆-alkyl, C₁-C₄- Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso- Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluor- methoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy sub- stituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substi- tuiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄- alkyl oder Thiazolyloxy-C₁-C₄-alkyl steht.
R² für jeweils gegebenenfalls durch Fluor oder Chlor substituiertes C₁- C₁₄-Alkyl, C₂-C₁₄-Alkenyl, C₁-C₄-Alkoxy-C₂-C₆-alkyl oder Poly- C₁-C₄-alkoxy-C₂-C₆-alkyl,
für gegebenenfalls durch Fluor, Chlor, Methyl, Ethyl, n-Propyl, iso- Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht.
R³ für jeweils gegebenenfalls durch Fluor oder Chlor substituiertes Methyl, Ethyl, Propyl, iso-Propyl, Butyl, tert.-Butyl, oder jeweils gegebenenfalls durch Fluor, Chlor, Brom, Methyl, Ethyl, iso-Propyl, tert.-Butyl, Methoxy, Ethoxy, iso-Propoxy, Trifluormethyl, Trifluor- methoxy, Cyano oder Nitro substituiertes Phenyl oder Benzyl steht.
R⁴ und R⁵ unabhängig voneinander für jeweils gegebenfalls durch Fluor oder Chlor substituiertes C₁-C₄-Alkyl, C₁-C₄Alkoxy, C₁-C₄- Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio stehen.
R⁶ und R⁷ unabhängig voneinander für Wasserstoff, für jeweils gege- benenfalls durch Fluor oder Chlor substituiertes C₁-C₄-Alkyl, C₃-C₆- Cycloalkyl, C₁-C₄-Alkoxy, C₃-C₄-Alkenyl oder C₁-C₄-Alkoxy-C₂- C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest stehen, in welchem gegebe- nenfalls eine Methylengruppe durch Sauerstoff oder Schwefel ersetzt ist,
ausgenommen die Verbindung I-a-75 aus EP 528 156

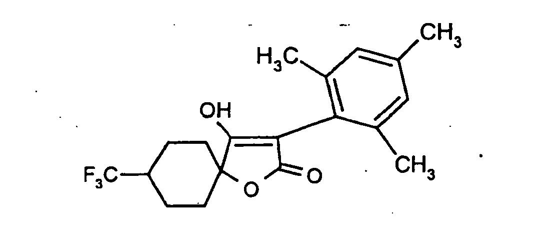

5. Verfahren zur Herstellung von Verbindungen der Formel (I) gemäß Anspruch 1, **dadurch gekennzeichnet, daß** man zum Erhalt von
(A) Verbindungen der Formel (I-1-a) 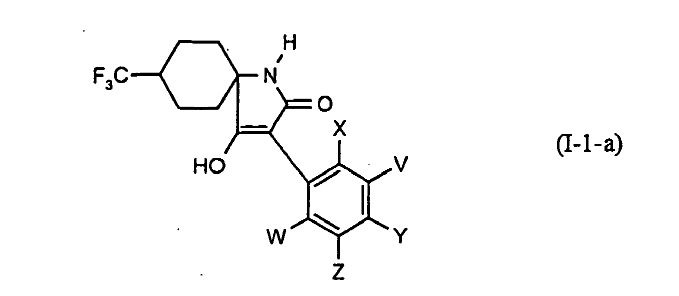 in welcher
V, W, X, Y und Z die oben angegebenen Bedeutungen haben,
Verbindungen der Formel (II) 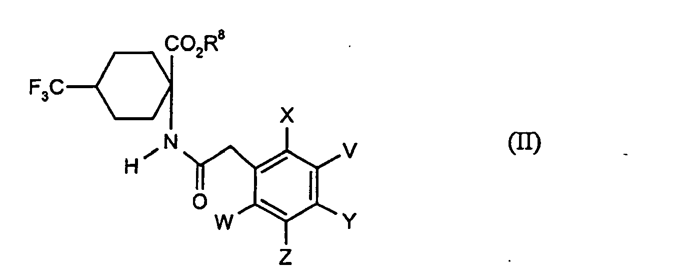 in welcher
V, W, X, Y und Z die oben angegebenen Bedeutungen haben,
und
R8 für Alkyl steht,
in Gegenwart eines Verdünnungsmittels und in Gegenwart einer Base intramolekular kondensiert,
(B) Verbindungen der Formel (I-2-a) 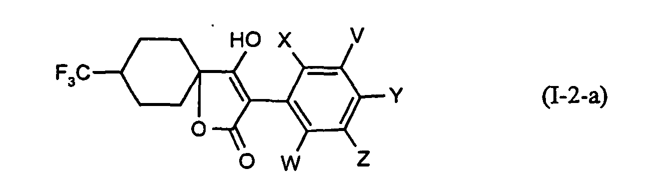 in welcher
V, W, X, Y und Z die oben angegebenen Bedeutungen haben,
Verbindungen der Formel (III) 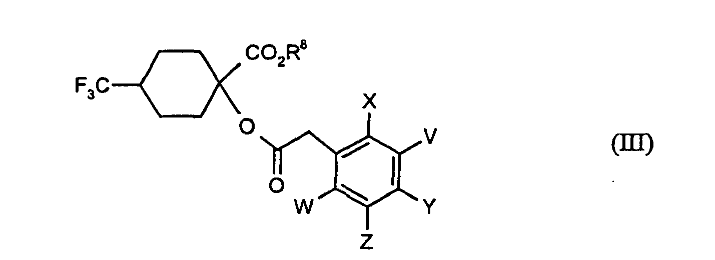 in welcher
V, W, X, Y, Z und R⁸ die oben angegebenen Bedeutungen haben,
in Gegenwart eines Verdünnungsmittels und in Gegenwart einer Base intramolekular kondensiert und die so erhaltenen Verbindungen der Formel (I-1-a) und (I-2-a) gegebenenfalls anschließend
(C) α) mit Verbindungen der Formel (IV) 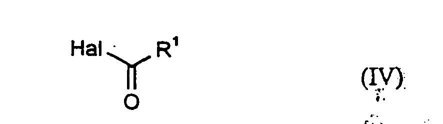 in welcher
R¹ die oben angegebene Bedeutung hat und
Hal für Halogen steht
oder
β) mit Verbindungen der Formel (V)
R¹-CO-O-CO-R¹ (V)
in welcher
R¹ die oben angegebene Bedeutung hat,
gegebenenfalls in Gegenwart eines Verdünnungsmittels und gegebenenfalls in Gegenwart eines Säurebindemittels umsetzt;
(D) mit Verbindungen der Formel (VI)
R²-M-CO-CI (VI)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(E) mit Verbindungen der Formel (VII) 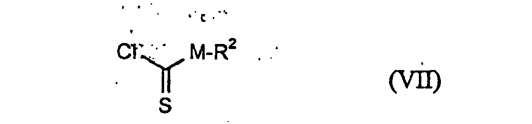 in welcher
M und R² die oben angegebenen Bedeutungen haben,
gegebenenfalls in Gegenwart eines Verdünnungsmittels und gegebenenfalls in Gegenwart eines Säurebindemittels umsetzt,
(F) mit Verbindungen der Formel (VIII)
R³-SO₂-Cl (VIII)
in welcher
R³ die oben angegebene Bedeutung hat, gegebenenfalls in Gegenwart eines Verdünnungsmittels und gegebenenfalls in Gegenwart eines Säurebindemittels umsetzt,
(G) mit Verbindungen der Formel (IX) 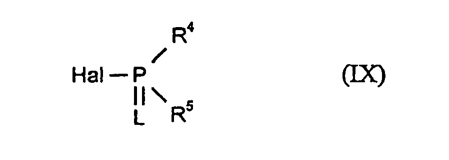 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(H) mit Verbindungen der Formeln (X) oder (XI)
Me(OR¹⁰), (X) 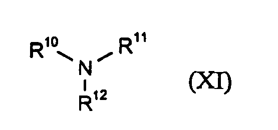
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
(I) α) mit Verbindungen der Formel (XII)
R⁶-N=C=L (XII)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
*β) mit Verbindungen der Formel (XIII) 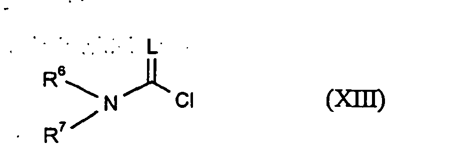 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

6. Verbindungen der Formel (II) 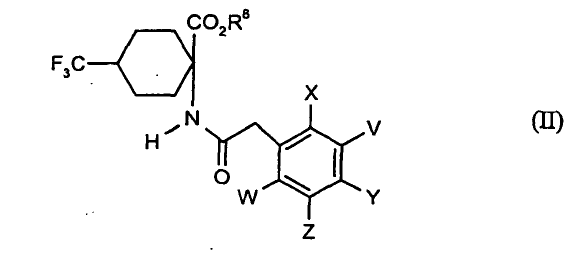 in welcher
V, W, X, Y und Z die oben angegebenen Bedeutungen haben, und
R⁸ für Alkyl steht.

7. Verbindungen der Formel (III) 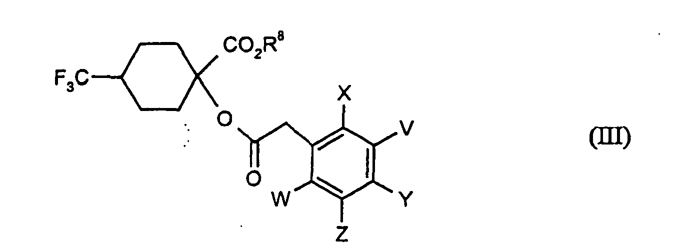 in welcher
V, W, X, Y, Z und R⁸ die oben angegebenen Bedeutungen haben,
ausgenommen ist folgende Verbindung

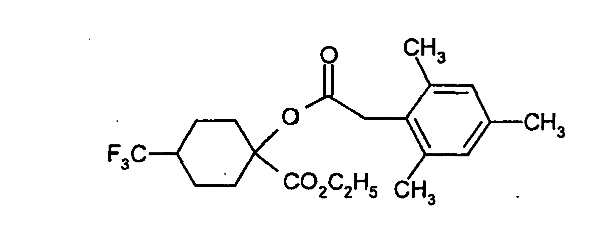

8. Verbindungen der Formel (XVI) 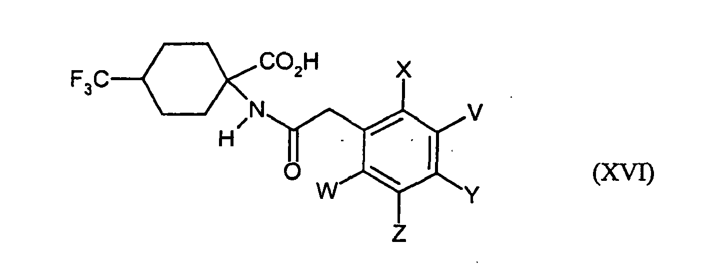 in welcher
V, W, X, Y und Z die oben angegebenen Bedeutungen haben

9. Verbindungen der Formel (XIX) 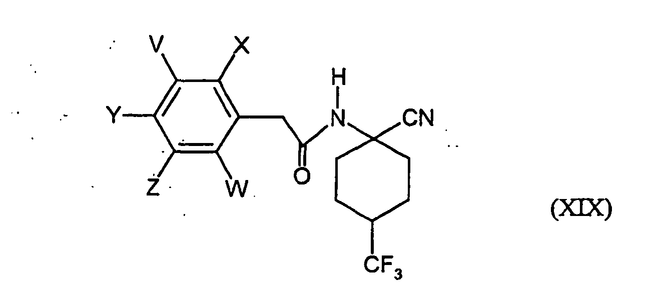 in welcher
V, W, X, Y und Z die oben angegebenen Bedeutungen haben.

10. Verbindung der Formel (XVIII)

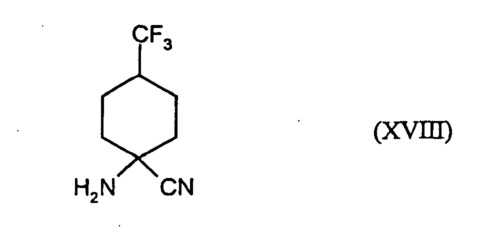

11. Verbindungen der Formel (XIV) 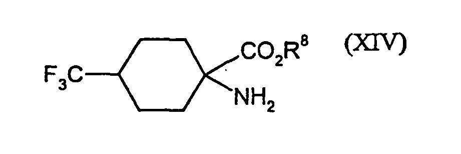 in welcher
R⁸ die oben angegebenen Bedeutungen hat.

12. Schädlingshekämpfungsmittel und/oder Unkrautbekämpfungsmittel, **gekennzeichnet durch** einen Gehalt an mindestens einer Verbindung der Formel (I) gemäß Anspruch 1.

13. Verwendung von Verbindungen der Formel (I) gemäß Anspruch 1 zur Bekämpfung von Schädlingen und Unkräutern, ausgenommen die Verwendung zur Behandlung des menschlichen und tierischen Körpers.

14. Verfahren zur Bekämpfung von Schädlingen und Unkräutern, **dadurch gekennzeichnet, daß** man Verbindungen der Formel (1) gemäß Anspruch 1 auf die Schädlinge, Pflanzen und/oder ihren Lebensraum einwirken läßt, ausgenommen die Verwendung zur Behandlung des menschlichen und tierischen Körpers.

15. Verfahren zur Herstellung von Schädlingsbekämpfungsmitteln und/oder Unkrautbekämpfungsmitteln, **dadurch gekennzeichnet, daß** man Verbindungen der Formel (I) gemäß Anspruch 1 mit Streckmitteln und/oder oberflächenaktiven Mitteln vermischt.

16. Verwendung von Verbindungen der Formel (I) gemäß Anspruch 1 zur Herstellung von Schädlingsbekämpfungsmitteln und Herbiziden.

## Claims

1. Compounds of the formula (I) 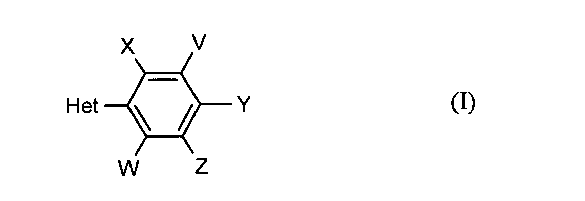 in which
V represents hydrogen, halogen, alkyl or alkoxy,
W represents hydrogen, cyano, nitro, halogen, alkyl, alkenyl, alkinyl, alkoxy, halogenoalkyl, halogenoalkoxy, in each case optionally substituted phenyl, phenoxy, phenylthio, phenylalkoxy or phenylalkylthio,
X represents halogen, alkyl, alkenyl, alkinyl, alkoxy, halogenoalkyl, halogenoalkoxy, cyano, nitro, in each case optionally substituted phenyl, phenoxy, phenylthio, phenylalkyloxy or phenylalkylthio,
Y represents hydrogen, halogen, alkyl, alkoxy, halogenoalkyl, halogenoalkoxy, cyano or nitro,
Z represents hydrogen, halogen, alkyl, alkoxy, halogenoalkyl, halogenoalkoxy, hydroxyl, cyano, nitro or in each case optionally substituted phenoxy, phenylthio, 5- or 6-membered hetaryloxy, 5- or 6-membered hetarylthio, phenylalkyloxy or phenylalkylthio,
Het represents one of the groups 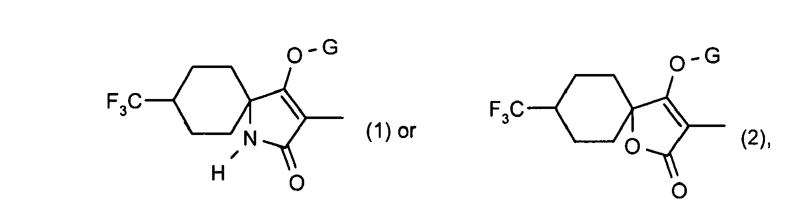 in which
G represents hydrogen (a) or represents one of the groups 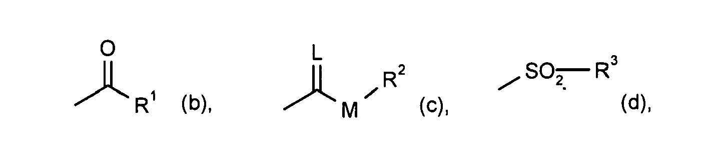 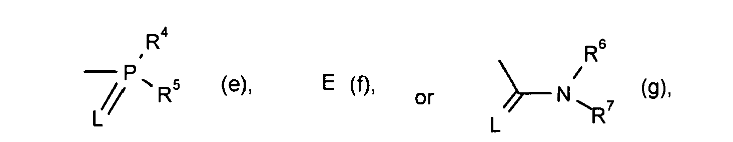 in which
E the represents hydrogen (a) or represents one of groups
L represents oxygen or sulphur,
M represents oxygen or sulphur,
R¹ represents in each case optionally halogen- or cyano-substituted alkyl, alkenyl, alkoxyalkyl, alkylthioalkyl or polyalkoxyalkyl or represents in each case optionally halogen-, alkyl- or alkoxy-substituted cycloalkyl or heterocyclyl or represents in each case optionally substituted phenyl, phenylalkyl, hetaryl, phenoxyalkyl or hetaryloxyalkyl,
R² represents in each case optionally halogen- or cyano-substituted alkyl, alkenyl, alkoxyalkyl or polyalkoxyalkyl or represents in each case optionally substituted cycloalkyl, phenyl or benzyl,
R³, R⁴ and R⁵ independently of one another represent in each case optionally halogen-substituted alkyl, alkoxy, alkylamino, dialkylamino, alkylthio, alkenylthio or cycloalkylthio or represent in each case optionally substituted phenyl, benzyl, phenoxy or phenylthio,
R⁶ and R⁷ independently of one another represent hydrogen, represent in each case optionally halogen- or cyano-substituted alkyl, cycloalkyl, alkenyl, alkoxy, alkoxyalkyl, represent in each case optionally substituted phenyl or benzyl, or together with the N atom to which they are attached form an optionally substituted cycle which optionally contains oxygen or sulphur,
except for the compound I-a-79 from EP 528 156

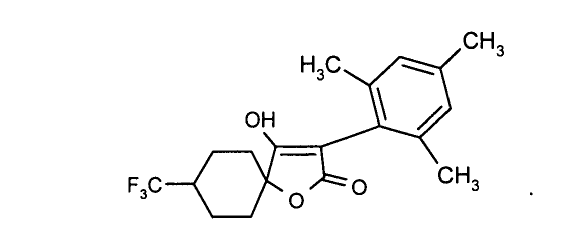

2. Compounds of the formula (I) according to Claim 1, in which
V represents hydrogen, halogen, C₁-C₆-alkyl or C₁-C₆-alkoxy,
W represents hydrogen, nitro, cyano, halogen, C₁- C₆-alkyl, C₂-C₆-alkenyl or C₂-C₆-alkinyl, C₁- C₆-alkoxy, C₁-C₄-halogenoalkyl, C₁-C₄- halogenoalkoxy or in each case optionally halogen-, C₁-C₆-alkyl-, C₁-C₆-alkoxy-, C₁-C₄- halogenoalkyl-, C₁-C₄-halogenoalkoxy-, nitro- or cyano-substituted phenyl, phenoxy, phenylthio, phenyl-C₁-C₄-alkoxy or phenyl-C₁- C₄-alkylthio,
X represents halogen, C₁-C₆-alkyl, C₂-C₆-alkenyl, C₂-C₆-alkinyl, C₁-C₆-alkoxy, C₁-C₄- halogenoalkyl, C₁-C₄-halogenoalkoxy, cyano, nitro or in each case optionally halogen-, C₁- C₆-alkyl-, C₁-C₆-alkoxy-, C₁-C₄-halogenoalkyl-, C₁-C₄-halogenoalkoxy-, nitro- or cyano- substituted phenyl, phenoxy, phenylthio, phenyl-C₁-C₄-alkoxy or phenyl-C₁-C₄-alkylthio,
Y represents hydrogen, halogen, C₁-C₆-alkyl, C₁- C₆-alkoxy, C₁-C₄-halogenoalkyl, C₁-C₄- halogenoalkoxy, cyano or nitro,
Z represents hydrogen, halogen, C₁-C₆-alkyl, C₁- C₆-alkoxy, C₁-C₄-halogenoalkyl, C₁-C₄- halogenoalkoxy, hydroxyl, cyano, nitro or in each case optionally halogen-, C₁-C₄-alkyl-, C₁-C₄-alkoxy-, C₁-C₄-halogenoalkyl-, C₁-C₄-halogenoalkoxy-, nitro- or cyano- substituted phenoxy, phenylthio, thiazolyloxy, pyridinyloxy, pyrimidyloxy, pyrazolyloxy, phenyl-C₁-C₄-alkyloxy or phenyl- C₁-C₄-alkylthio,
Het represents one of the groups 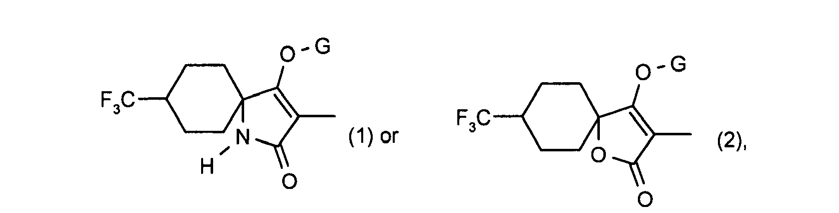
G represents hydrogen (a) or represents one of the groups 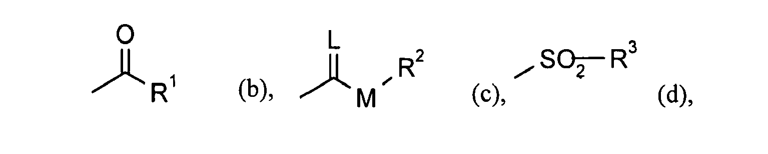 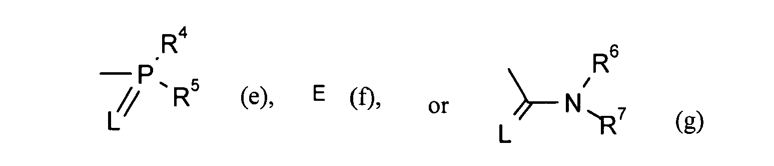 in which
V represents a metal ion or an ammonium ion,
L represents oxygen or sulphur and
M represents oxygen or sulphur,
R¹ represents in each case optionally halogen- or cyano-substituted C₁-C₂₀-alkyl, C₂-C₂₀-alkenyl, C₁-C₈-alkoxy-C₁-C₈-alkyl, C₁-C₈-alkylthio-C₁- C₈-alkyl or poly-C₁-C₈-alkoxy-C₁-C₈-alkyl or represents optionally halogen-, C₁-C₆-alkyl- or C₁-C₆-alkoxy-substituted C₃-C₈-cycloalkyl in which optionally one or two not directly adjacent methylene groups are replaced by oxygen and/or sulphur,
represents optionally halogen-, cyano-, nitro-, C₁-C₆-alkyl-, C₁-C₆-alkoxy-, C₁-C₆- halogenoalkyl-, C₁-C₆-halogenoalkoxy-, C₁-C₆- alkylthio- or C₁-C₆-alkylsulphonyl-substituted phenyl,
represents optionally halogen-, nitro-, cyano-, C₁-C₆-alkyl-, C₁-C₆-alkoxy-, C₁-C₆- halogenoalkyl- or C₁-C₆-halogenoalkoxy- substituted phenyl-C₁-C₆-alkyl,
represents optionally halogen- or C₁-C₆-alkyl- substituted 5- or 6-membered hetaryl having one or two heteroatoms from the group consisting of oxygen, sulphur and nitrogen,
represents optionally halogen- or C₁-C₆-alkyl- substituted phenoxy-C₁-C₆-alkyl or
represents optionally halogen-, amino- or C₁- C₆-alkyl-substituted 5- or 6-membered hetaryloxy-C₁-C₆-alkyl having one or two heteroatoms from the group consisting of oxygen, sulphur and nitrogen,
R² represents in each case optionally halogen- or cyano-substituted C₁-C₂₀-alkyl, C₂-C₂₀-alkenyl, C₁-C₈-alkoxy-C₂-C₈-alkyl or poly-C₁-C₈-alkoxy- C₂-C₈-alkyl,
represents optionally halogen-, C₁-C₆-alkyl- or C₁-C₆-alkoxy-substituted C₃-C₈-cycloalkyl or
represents in each case optionally halogen-, cyano-, nitro-, C₁-C₆-alkyl-, C₁-C₆-alkoxy-, C₁-C₆-halogenoalkyl- or C₁-C₆-halogenoalkoxy- substituted phenyl or benzyl,
R³ represents optionally halogen-substituted C₁- C₈-alkyl or in each case optionally halogen-, C₁-C₆-alkyl-, C₁-C₆-alkoxy-, C₁-C₄- halogenoalkyl-, C₁-C₄-halogenoalkoxy-, cyano- or nitro-substituted phenyl or benzyl,
R⁴ and R⁵ independently of one another represent in each case optionally halogen-substituted C₁-C₈- alkyl, C₁-C₈-alkoxy, C₁-C₈-alkylamino, di(C₁- C₈-alkyl)amino, C₁-C₈-alkylthio or C₃-C₈- alkenylthio or represent in each case optionally halogen-, nitro-, cyano-, C₁-C₄- alkoxy-, C₁-C₄-halogenoalkoxy-, C₁-C₄- alkylthio-, C₁-C₄-halogenoalkylthio-, C₁-C₄- alkyl- or C₁-C₄-halogenoalkyl-substituted phenyl, phenoxy or phenylthio,
R⁶ and R⁷ independently of one another represent hydrogen, represent in each case optionally halogen- or cyano-substituted C₁-C₈-alkyl, C₃- C₈-cycloalkyl, C₁-C₈-alkoxy, C₃-C₈-alkenyl or C₁-C₈-alkoxy-C₂-C₈-alkyl, represent in each case optionally halogen-, C₁-C₈-alkyl-, C₁-C₈- halogenoalkyl- or C₁-C₈-alkoxy-substituted phenyl or benzyl or together represent an optionally C₁-C₆-alkyl-substituted C₃-C₆- alkylene radical in which optionally one methylene group is replaced by oxygen or sulphur,
except for the compound I-a-75 from EP 528 156

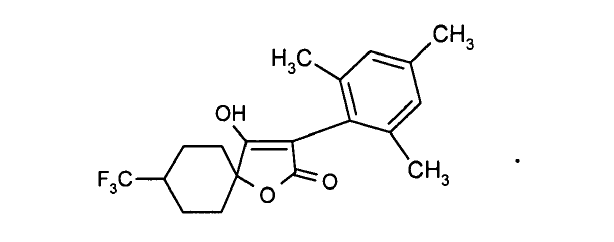

3. Compounds of the formula (I) according to Claim 1, in which
V represents hydrogen, fluorine, chlorine, bromine, C₁-C₄-alkyl or C₁-C₄-alkoxy,
W represents hydrogen, fluorine, chlorine, bromine, C₁-C₄-alkyl, C₁-C₄-alkoxy, C₁-C₂- halogenoalkyl or C₁-C₂-halogenoalkoxy,
X represents fluorine, chlorine, bromine, C₁-C₄- alkyl, C₁-C₄-alkoxy, C₁-C₂-halogenoalkyl, C₁₋ C₂-halogenoalkoxy, cyano or nitro,
Y represents hydrogen, fluorine, chlorine, bromine, C₁-C₄-alkyl, C₁-C₄-alkoxy, C₁-C₂- halogenoalkyl, C₁-C₂-halogenoalkoxy, cyano or nitro,
Z represents hydrogen, fluorine, chlorine, bromine, C₁-C₄-alkyl, C₁-C₄-alkoxy, C₁-C₂- halogenoalkyl, C₁-C₂-halogenoalkoxy, hydroxyl, cyano, nitro or in each case optionally fluorine-, chlorine-, bromine-, C₁-C₄-alkyl-, C₁-C₄-alkoxy-, C₁-C₂-halogenoalkyl-, C₁-C₂- halogenoalkoxy-, nitro- or cyano-substituted phenoxy or benzyloxy,
Het represents one of the groups 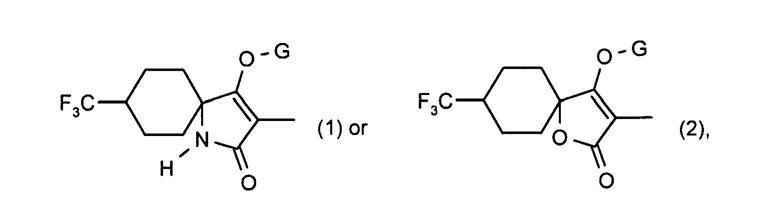
G represents hydrogen (a) or represents one of the groups 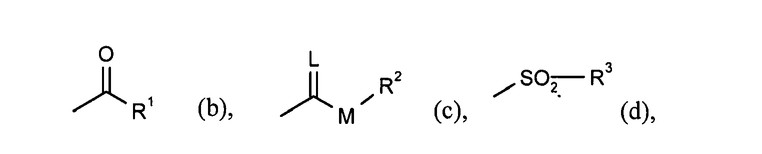 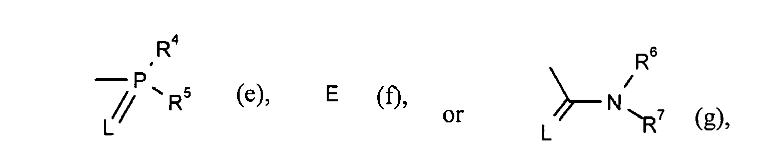 in which
E represents a metal ion or an ammonium ion,
L represents oxygen or sulphur and
M represents oxygen or sulphur,
R¹ represents in each case optionally fluorine- or chlorine-substituted C₁-C₁₆-alkyl, C₂-C₁₆- alkenyl, C₁-C₆-alkoxy-C₁-C₆-alkyl, C₁-C₆- alkylthio-C₁-C₆-alkyl or poly-C₁-C₆-alkoxy-C₁- C₆-alkyl or represents optionally fluorine-, chlorine-, C₁-C₅-alkyl- or C₁-C₅-alkoxy- substituted C₃-C₇-cycloalkyl in which optionally one or two not directly adjacent methylene groups are replaced by oxygen and/or sulphur,
represents optionally fluorine-, chlorine-, bromine-, cyano-, nitro-, C₁-C₄-alkyl-, C₁-C₄- alkoxy-, C₁-C₃-halogenoalkyl-, C₁-C₃- halogenoalkoxy-, C₁-C₄-alkylthio- or C₁-C₄- alkylsulphonyl-substituted phenyl,
represents optionally fluorine-, chlorine-, bromine-, C₁-C₄-alkyl-, C₁-C₄-alkoxy-, C₁-C₃- halogenoalkyl- or C₁-C₃-halogenoalkoxy- substituted phenyl-C₁-C₄-alkyl,
represents in each case optionally fluorine-, chlorine-, bromine- or C₁-C₄-alkyl-substituted pyrazolyl, thiazolyl, pyridyl, pyrimidyl, furanyl or thienyl,
represents optionally fluorine-, chlorine-, bromine- or C₁-C₄-alkyl-substituted phenoxy-C₁- C₅-alkyl or
represents in each case optionally fluorine-, chlorine-, bromine-, amino- or C₁-C₄-alkyl- substituted pyridyloxy-C₁-C₅-alkyl, pyrimidyloxy-C₁-C₅-alkyl or thiazolyloxy-C₁-C₅- alkyl,
R² represents in each case optionally fluorine- or chlorine-substituted C₁-C₁₆-alkyl, C₂-C₁₆- alkenyl, C₁-C₆-alkoxy-C₂-C₆-alkyl or poly-C₁- C₆-alkoxy-C₂-C₆-alkyl,
represents optionally fluorine-, chlorine-, C₁- C₄-alkyl- or C₁-C₄-alkoxy-substituted C₃-C₇- cycloalkyl or
represents in each case optionally fluorine-, chlorine-, bromine-, cyano-, nitro-, C₁-C₄- alkyl-, C₁-C₃-alkoxy-, C₁-C₃-halogenoalkyl- or C₁-C₃-halogenoalkoxy-substituted phenyl or benzyl,
R³ represents optionally fluorine- or chlorine- substituted C₁-C₆-alkyl or in each case optionally fluorine-, chlorine-, bromine-, C₁- C₄-alkyl-, C₁-C₄-alkoxy-, C₁-C₂-halogenoalkoxy- , C₁-C₂-halogenoalkyl-, cyano- or nitro- substituted phenyl or benzyl,
R⁴ and R⁵ independently of one another represent in each case optionally fluorine- or chlorine- substituted C₁-C₆-alkyl, C₁-C₆-alkoxy, C₁-C₆- alkylamino, di(C₁-C₆-alkyl)amino, C₁-C₆- alkylthio or C₃-C₄-alkenylthio or represent in each case optionally fluorine-, chlorine-, bromine-, nitro-, cyano-, C₁-C₃-alkoxy-, C₁-C₃- halogenoalkoxy-, C₁-C₃-alkylthio-, C₁-C₃- halogenoalkylthio-, C₁-C₃-alkyl- or C₁-C₃- halogenoalkyl-substituted phenyl, phenoxy or phenylthio,
R⁶ and R⁷ independently of one another represent hydrogen, represent in each case optionally fluorine- or chlorine-substituted C₁-C₆-alkyl, C₃-C₆-cycloalkyl, C₁-C₆-alkoxy, C₃-C₆-alkenyl or C₁-C₆-alkoxy-C₂-C₆-alkyl, represent in each case optionally fluorine-, chlorine-, bromine-, C₁-C₅-halogenoalkyl-, C₁-C₅-alkyl- or C₁-C₅- alkoxy-substituted phenyl or benzyl, or together represent an optionally C₁-C₄-alkyl- substituted C₃-C₆-alkylene radical in which optionally one methylene group is replaced by oxygen or sulphur,
except for the compound I-a-75 from EP 528 156

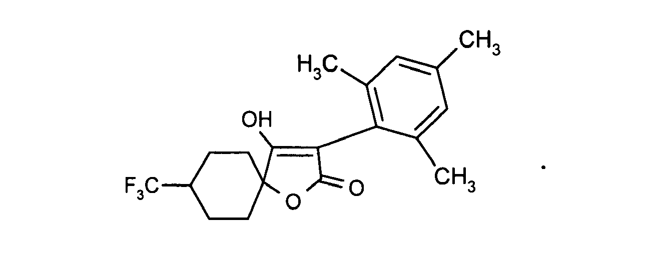

4. Compounds of the formula (I) according to Claim 1, in which
V represents hydrogen, fluorine, chlorine, bromine, methyl, ethyl, methoxy or ethoxy,
W represents hydrogen, fluorine, chlorine, bromine, methyl, ethyl, propyl, methoxy or ethoxy,
X represents fluorine, chlorine, bromine, methyl, ethyl, propyl, isopropyl, methoxy, ethoxy, propoxy, isopropoxy, trifluoromethyl, trifluoromethoxy, difluoromethoxy or cyano,
Y represents hydrogen, fluorine, chlorine, bromine, methyl, ethyl, propyl, isopropyl, tert-butyl, methoxy, ethoxy, propoxy, isopropoxy, trifluoromethyl, trifluoromethoxy, difluoromethoxy, cyano or nitro,
Z represents hydrogen, fluorine, chlorine, bromine, methyl, ethyl, propyl, isopropyl, tert-butyl, methoxy, ethoxy, propoxy, isopropoxy, trifluoromethyl, trifluoromethoxy, difluoromethoxy, cyano or nitro,
Het represents one of the groups 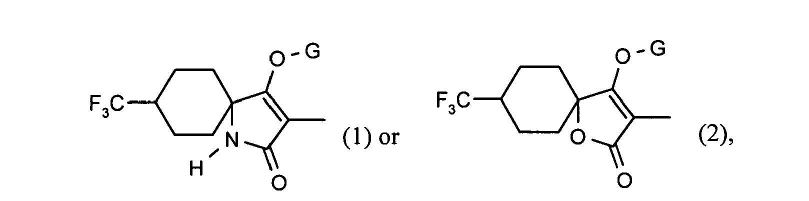
G represents hydrogen (a) or represents one of the groups 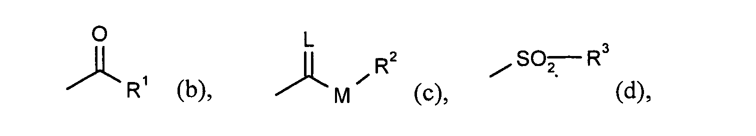 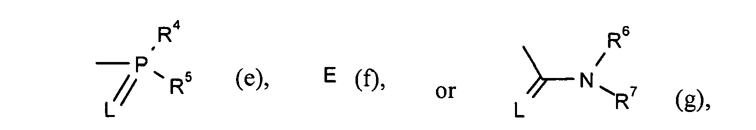 in which
E represents a metal ion or an ammonium ion,
L represents oxygen or sulphur and
M represents oxygen or sulphur,
R¹ represents in each case optionally fluorine- or chlorine-substituted C₁-C₁₄-alkyl, C₂-C₁₄- alkenyl, C₁-C₄-alkoxy-C₁-C₆-alkyl, C₁-C₄- alkylthio-C₁-C₆-alkyl, poly-C₁-C₄-alkoxy-C₁-C₄- alkyl or represents optionally fluorine-, chlorine-, methyl-, ethyl-, n-propyl-, isopropyl-, n-butyl-, isobutyl-, tert-butyl-, methoxy-, ethoxy-, n-propoxy- or isopropoxy- substituted C₃-C₆-cycloalkyl in which optionally one or two not directly adjacent methylene groups are replaced by oxygen and/or sulphur,
represents optionally fluorine-, chlorine-, bromine-, cyano-, nitro-, methyl-, ethyl-, n- propyl-, isopropyl-, methoxy-, ethoxy-, trifluoromethyl-, trifluoromethoxy-, methylthio-, ethylthio-, methylsulphonyl- or ethylsulphonyl-substituted phenyl,
represents optionally fluorine-, chlorine-, bromine-, methyl-, ethyl-, n-propyl-, isopropyl-, methoxy-, ethoxy-, trifluoromethyl- or trifluoromethoxy-substituted benzyl,
represents in each case optionally fluorine-, chlorine-, bromine-, methyl- or ethyl- substituted furanyl, thienyl or pyridyl,
represents optionally fluorine-, chlorine-, methyl- or ethyl-substituted phenoxy-C₁-C₄- alkyl or
represents in each case optionally fluorine-, chlorine-, amino-, methyl- or ethyl-substituted pyridyloxy-C₁-C₄-alkyl, pyrimidyloxy-C₁-C₄- alkyl or thiazolyloxy-C₁-C₄-alkyl,
R² represents in each case optionally fluorine- or chlorine-substituted C₁-C₁₄-alkyl, C₂-C₁₄- alkenyl, C₁-C₄-alkoxy-C₂-C₆-alkyl or poly-C₁- C₄-alkoxy-C₂-C₆-alkyl,
represents optionally fluorine-, chlorine-, methyl-, ethyl-, n-propyl-, isopropyl- or methoxy-substituted C₃-C₆-cycloalkyl,
or represents in each case optionally fluorine- , chlorine-, cyano-, nitro-, methyl-, ethyl-, n-propyl-, isopropyl-, methoxy-, ethoxy-, trifluoromethyl- or trifluoromethoxy- substituted phenyl or benzyl,
R³ represents in each case optionally fluorine- or chlorine-substituted methyl, ethyl, propyl, isopropyl, butyl, tert-butyl, or in each case optionally fluorine-, chlorine-, bromine-, methyl-, ethyl-, isopropyl-, tert-butyl-, methoxy-, ethoxy-, isopropoxy-, trifluoromethyl-, trifluoromethoxy-, cyano- or nitro-substituted phenyl or benzyl,
R⁴ and R⁵ independently of one another represent in each case optionally fluorine- or chlorine- substituted C₁-C₄-alkyl, C₁-C₄-alkoxy, C₁-C₄- alkylamino, di(C₁-C₄-alkyl)amino or C₁-C₄- alkylthio or represent in each case optionally fluorine-, chlorine-, bromine-, nitro-, cyano-, methyl-, methoxy-, trifluoromethyl- or trifluoromethoxy-substituted phenyl, phenoxy or phenylthio,
R⁶ and R⁷ independently of one another represent hydrogen, represent in each case optionally fluorine- or chlorine-substituted C₁-C₄-alkyl, C₃-C₆-cycloalkyl, C₁-C₄-alkoxy, C₃-C₄-alkenyl or C₁-C₄-alkoxy-C₂-C₄-alkyl, represent in each case optionally fluorine-, chlorine-, bromine-, methyl-, methoxy- or trifluoromethyl- substituted phenyl or benzyl, or together represent an optionally methyl- or ethyl- substituted C₅-C₆-alkylene radical in which optionally one methylene group is replaced by oxygen or sulphur,
except for the compound I-a-75 from EP 528 156

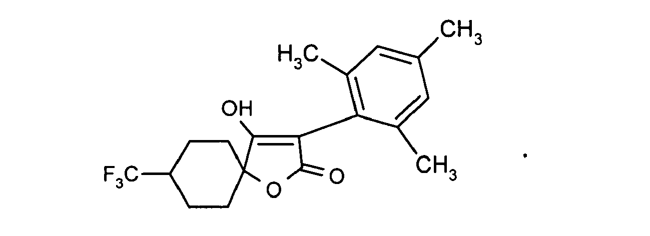

5. Process for preparing compounds of the formula (I) according to Claim 1, **characterized in that**, to obtain
(A) compounds of the formula (I-1-a) 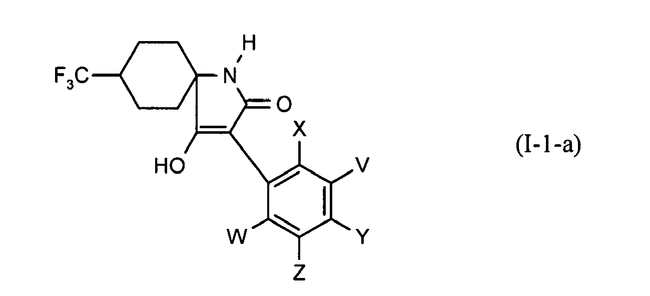 in which
V, W, X, Y and Z have the meanings given above,
compounds of the formula (II) 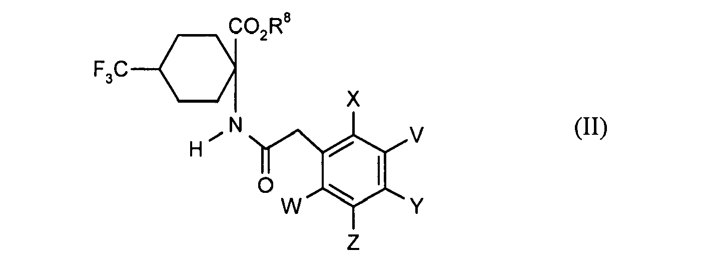 in which
V, W, X, Y and Z have the meanings given above,
and
R⁸ represents alkyl
are condensed intramolecularly in the presence of a diluent and in the presence of a base,
(B) compounds of the formula (I-2-a) 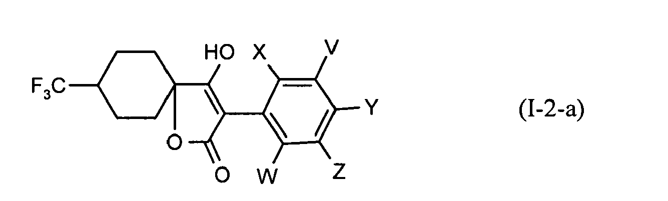 in which
V, W, X, Y and Z have the meanings given above,
compounds of the formula (III) 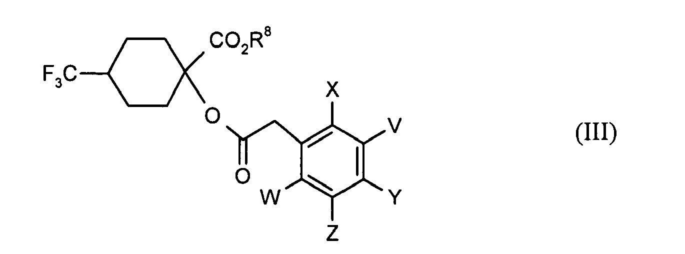 in which
V, W, X, Y, Z and R⁸ have the meanings given above
are condensed intramolecularly in the presence of a diluent and in the presence of a base and the resulting compounds of the formula (I-1-a) and (I-2-a) are, if appropriate, subsequently
(C) α) reacted with compounds of the formula (IV) 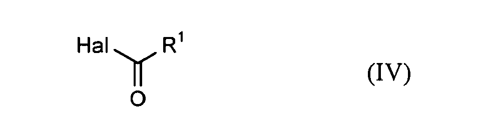 in which
R¹ has the meaning given above and
Hal represents halogen
or
β) reacted with compounds of the formula (V)
R¹-CO-O-CO-R¹ (V)
in which
R¹ has the meaning given above,
if appropriate in the presence of a diluent and if appropriate in the presence of an acid binder;
(D) reacted with compounds of the formula (VI)
R²-M-CO-Cl (VI)
in which
R² and M have the meanings given above,
if appropriate in the presence of a diluent and if appropriate in the presence of an acid binder;
(E) reacted with compounds of the formula (VII) 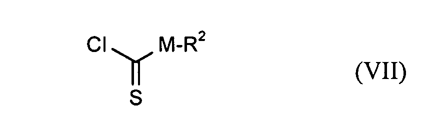 in which
M and R² have the meanings given above,
if appropriate in the presence of a diluent and if appropriate in the presence of an acid binder,
(F) reacted with compounds of the formula (VIII)
R³-SO₂-Cl (VIII)
in which
R³ has the meaning given above,
if appropriate in the presence of a diluent and if appropriate in the presence of an acid binder,
(G) reacted with compounds of the formula (IX) 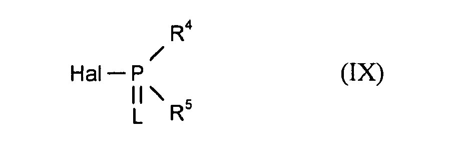 in which
L, R⁴ and R⁵ have the meanings given above and
Hal represents halogen,
if appropriate in the presence of a diluent and if appropriate in the presence of an acid binder,
(H) reacted with compounds of the formula (X) or (XI)
Me(OR¹⁰)ₜ (X) 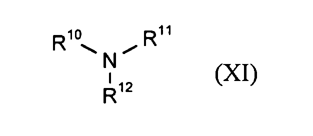
in which
Me represents a mono- or divalent metal,
t represents the number 1 or 2 and
R¹⁰, R¹¹, R¹² independently of one another represent hydrogen or alkyl,
if appropriate in the presence of a diluent,
(I) α) reacted with compounds of the formula (XII)
R⁶-N=C=L (XII)
in which
R⁶ and L have the meanings given above,
if appropriate in the presence of a diluent and if appropriate in the presence of a catalyst or
β) reacted with compounds of the formula (XIII) 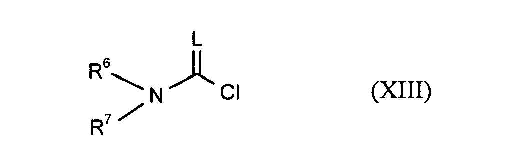 in which
L, R⁶ and R⁷ have the meanings given above,
if appropriate in the presence of a diluent and if appropriate in the presence of an acid binder.

6. Compounds of the formula (II) 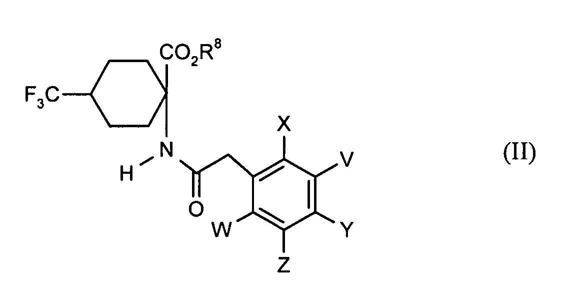 in which
V, W, X, Y and Z have the meanings given above and
R⁸ represents alkyl.

7. Compounds of the formula (III) 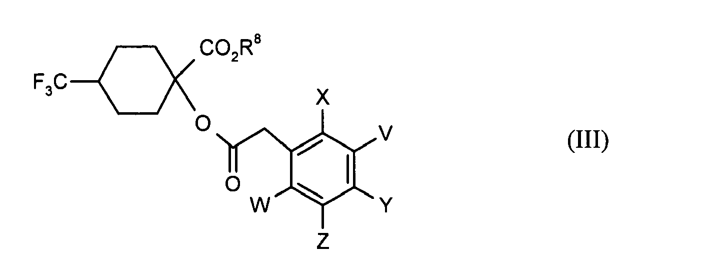 in which
V, W, X, Y, Z and R⁸ have the meanings given above,
except for the compound below

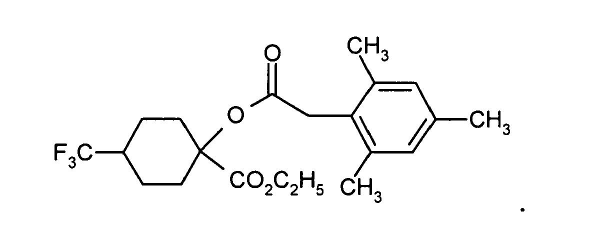

8. Compounds of the formula (XVI) 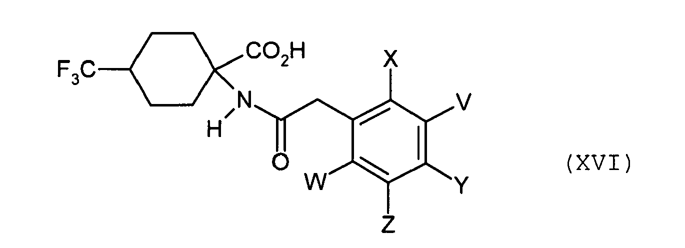 in which
V, W, X, Y and Z have the meanings given above.

9. Compounds of the formula (XIX) 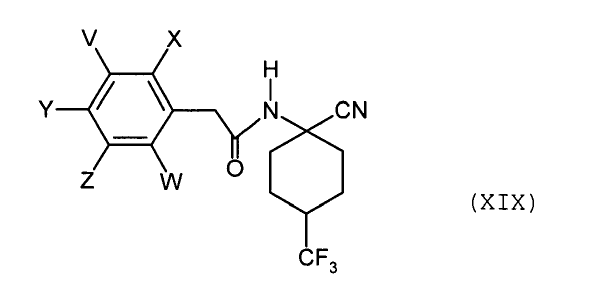 in which
V, W, X, Y and Z have the meanings given above.

10. Compound of the formula (XVIII)

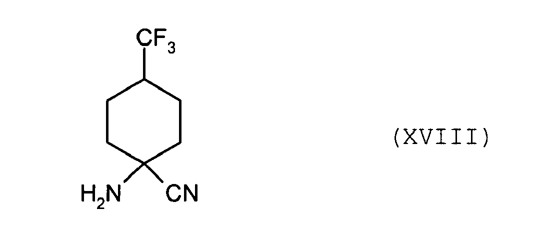

11. Compounds of the formula (XIV) 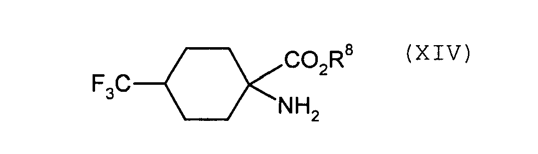 in which
R⁸ has the meanings given above.

12. Pesticide and/or weed killer, **characterized in that** it comprises at least one compound of the formula (I) according to Claim 1.

13. Use of compounds of the formula (I) according to Claim 1 for controlling pests and weeds, except for the use for treating the human and animal body.

14. Method for controlling pests and weeds, **characterized in that** compounds of the formula (I) according to Claim 1 are allowed to act on the pests, plants and/or their habitat, except for the use for treating the human and animal body.

15. Process for preparing pesticides and/or weed killers, **characterized in that** compounds of the formula (I) according to Claim 1 are mixed with extenders and/or surfactants.

16. Use of compounds of the formula (I) according to Claim 1 for preparing pesticides and herbicides.

## Revendications

1. Composés de formule (I) 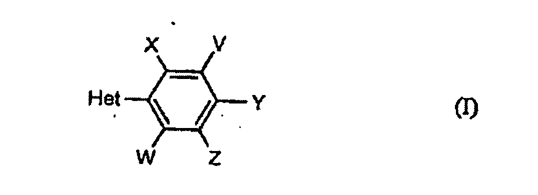 dans laquelle
V représente hydrogène, halogène, alkyle ou alcoxy,
W représente hydrogène, cyano, nitro, halogène, alkyle, alcényle, alcynyle, alcoxy, halogénoalkyle, halogénoalcoxy ; phényle, phénoxy, phénylthio, phénylalcoxy ou phénylalkylthio, chacun éventuellement substitué,
X représente halogène, alkyle, alcényle, alcynyle, alcoxy, halogénoalkyle, halogénoalcoxy, cyano, nitro ; phényle, phénoxy, phénylthio, phénylalkyloxy ou phénylalkylthio, chacun éventuellement substitué,
Y représente hydrogène, halogène, alkyle, alcoxy, halogénoalkyle, halogénoalcoxy, cyano ou nitro,
Z représente hydrogène, halogène, alkyle, alcoxy, halogénoalkyle, halogénoalcoxy, hydroxy, cyano, nitro ; ou phénoxy, phénylthio, hétaryloxy à 5 ou 6 éléments, hétarylthio à 5 ou 6 éléments, phénylalkyloxy ou phénylalkylthio, chacun éventuellement substitué,
Het représente un des groupes 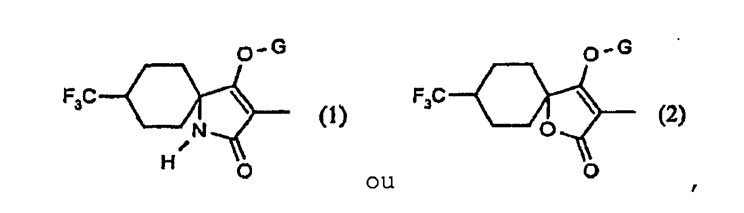 dans lesquels
G représente hydrogène (a) ou un des groupes 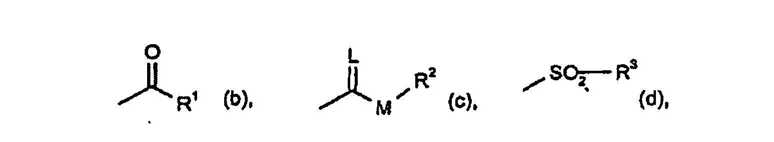 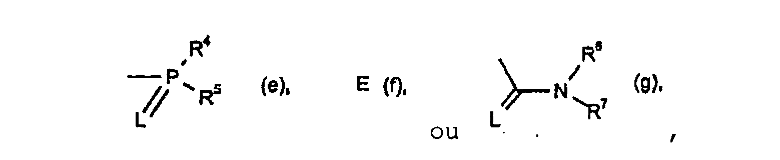 dans lesquels
E représente un ion métallique ou un ion ammonium,
L représente l'oxygène ou le soufre,
M représente l'oxygène ou le soufre,
R¹ représente alkyle, alcényle, alcoxyalkyle, alkylthioalkyle ou polyalcoxyalkyle, chacun éventuellement substitué par halogène ou cyano ; ou cycloalkyle ou hétérocyclyle, chacun éventuellement substitué par halogène, alkyle ou alcoxy ; ou phényle, phénylalkyle, hétaryle, phénoxyalkyle ou hétaryloxyalkyle, chacun éventuellement substitué,
R² représente alkyle, alcényle, alcoxyalkyle ou polyalcoxyalkyle, chacun éventuellement substitué par halogène ou cyano ; ou cycloalkyle, phényle ou benzyle, chacun éventuellement substitué,
R³, R⁴ et R⁵ représentent indépendamment les uns des autres alkyle, alcoxy, alkylamino, dialkylamino, alkylthio, alcénylthio ou cycloalkylthio, chacun éventuellement substitué par halogène ; ou phényle, benzyle, phénoxy ou phénylthio, chacun éventuellement substitué,
R⁶ et R⁷ représentent indépendamment l'un de l'autre hydrogène ; alkyle, cycloalkyle, alcényle, alcoxy, alcoxyalkyle, chacun éventuellement substitué par halogène ou cyano ; phényle ou benzyle, chacun éventuellement substitué ; ou forment ensemble avec l'atome N auquel ils sont reliés un cycle éventuellement substitué et contenant éventuellement de l'oxygène ou du soufre,
à l'exception du composé I-a-79 d'EP 528 156

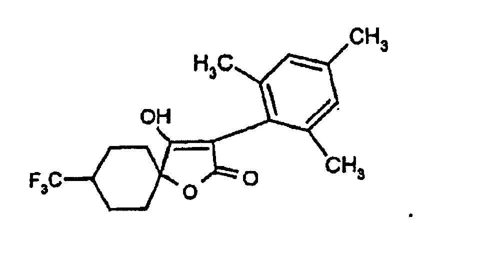

2. Composés de formule (I) selon la revendication 1, dans lesquels
V représente hydrogène, halogène, alkyle en C₁-C₆ ou alcoxy en C₁-C₆,
W représente hydrogène, nitro, cyano, halogène, alkyle en C₁-C₆, alcényle en C₂-C₆ ou alcynyle en C₂-C₆, alcoxy en C₁-C₆, halogénoalkyle en C₁-C₄, halogénoalcoxy en C₁-C₄ ; ou phényle, phénoxy, phénylthio, phénylalcoxy en C₁-C₄ ou phénylalkylthio en C₁-C₉, chacun éventuellement substitué par halogène, alkyle en C₁-C₆, alcoxy en C₁-C₆, halogénoalkyle en C₁-C₉, halogénoalcoxy en C₁-C₄, nitro ou cyano,
X représente halogène, alkyle en C₁-C₆, alcényle en C₂-C₆, alcynyle en C₂-C₆, alcoxy en C₁-C₆, halogénoalkyle en C₁-C₄, halogénoalcoxy en C₁-C₉, cyano, nitro ; ou phényle, phénoxy, phénylthio, phénylalcoxy en C₁-C₄ ou phénylalkylthio en C₁-C₄, chacun éventuellement substitué par halogène, alkyle en C₁-C₆, alcoxy en C₁-C₆, halogénoalkyle en C₁-C₄, halogénoalcoxy en C₁-C₄, nitro ou cyano,
Y représente hydrogène, halogène, alkyle en C₁-C₆, alcoxy en C₁-C₆, halogénoalkyle en C₁-C₄, halogénoalcoxy en C₁-C₄, cyano ou nitro,
Z représente hydrogène, halogène, alkyle en C₁-C₆, alcoxy en C₁-C₆, halogénoalkyle en C₁-C₄, halogénoalcoxy en C₁-C₄, hydroxy, cyano, nitro ; ou phénoxy, phénylthio, thiazolyloxy, pyridinyloxy, pyrimidyloxy, pyrazolyloxy, phénylalkyloxy en C₁-C₄ ou phénylalkylthio en C₁-C₄, chacun éventuellement substitué par halogène, alkyle en C₁-C₄, alcoxy en C₁-C₄, halogénoalkyle en C₁-C₄, halogénoalcoxy en C₁-C₄, nitro ou cyano,
Het représente un des groupes 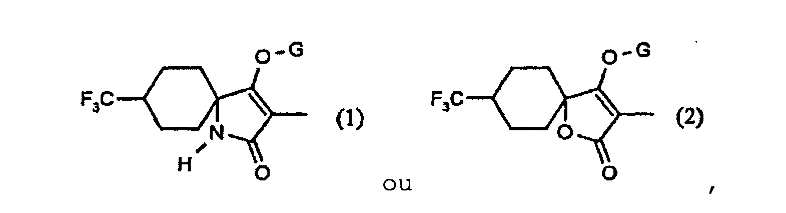
G représente hydrogène (a) ou un des groupes 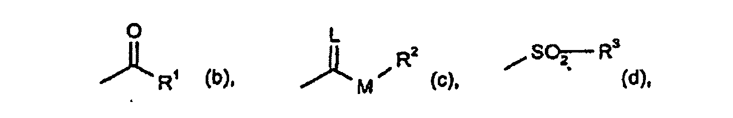 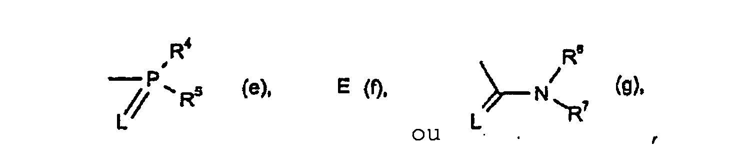 dans lesquels
E représente un ion métallique ou un ion ammonium,
L représente l'oxygène ou le soufre, et
M représente l'oxygène ou le soufre,
R¹ représente alkyle en C₁-C₂₀, alcényle en C₂-C₂₀, alcoxy en C₁-C₈-alkyle en C₁-C₈, alkylthio en C₁-C₈- alkyle en C₁-C₈ ou polyalcoxy en C₁-C₈-alkyle en C₁- C₈, chacun éventuellement substitué par halogène ou cyano ; ou cycloalkyle en C₃-C₈ éventuellement substitué par halogène, alkyle en C₁-C₆ ou alcoxy en C₁-C₆, dans lequel un ou deux groupes méthylène non directement voisins sont éventuellement remplacés par l'oxygène et/ou le soufre,
phényle éventuellement substitué par halogène, cyano, nitro, alkyle en C₁-C₆, alcoxy en C₁-C₆, halogénoalkyle en C₁-C₆, halogénoalcoxy en C₁-C₆, alkylthio en C₁-C₆ ou alkylsulfonyle en C₁-C₆,
phénylalkyle en C₁-C₆ éventuellement substitué par halogène, nitro, cyano, alkyle en C₁-C₆, alcoxy en C₁-C₆, halogénoalkyle en C₁-C₆ ou halogénoalcoxy en C₁-C₆,
hétaryle à 5 ou 6 éléments éventuellement substitué par halogène ou alkyle en C₁-C₆, contenant un ou deux hétéroatomes de la série oxygène, soufre et azote,
phénoxyalkyle en C₁-C₆ éventuellement substitué par halogène ou alkyle en C₁-C₆, ou
hétaryloxyalkyle en C₁-C₆ à 5 ou 6 éléments éventuellement substitué par halogène, amino ou alkyle en C₁-C₆, contenant un ou deux hétéroatomes de la série oxygène, soufre et azote,
R² représente alkyle en C₁-C₂₀, alcényle en C₂-C₂₀, alcoxy en C₁-C₈-alkyle en C₂-C₈ ou polyalcoxy en C₁- C₈-alkyle en C₂-C₈, chacun éventuellement substitué par halogène ou cyano,
cycloalkyle en C₃-C₈ éventuellement substitué par halogène, alkyle en C₁-C₆ ou alcoxy en C₁-C₆, ou phényle ou benzyle, chacun éventuellement substitué par halogène, cyano, nitro, alkyle en C₁- C₆, alcoxy en C₁-C₆, halogénoalkyle en C₁-C₆, ou halogénoalcoxy en C₁-C₆,
R³ représente alkyle en C₁-C₈ éventuellement substitué par halogène ; ou phényle ou benzyle, chacun éventuellement substitué par halogène, alkyle en C₁-C₆, alcoxy en C₁-C₆, halogénoalkyle en C₁-C₄, halogénoalcoxy en C₁-C₄, cyano ou nitro,
R⁴ et R⁵ représentent indépendamment l'un de l'autre alkyle en C₁-C₈, alcoxy en C₁-C₈, alkylamino en C₁- C₈, dialkylamino en C₁-C₈, alkylthio en C₁-C₈ ou alcénylthio en C₃-C₈, chacun éventuellement substitué par halogène ; ou phényle, phénoxy ou phénylthio, chacun éventuellement substitué par halogène, nitro, cyano, alcoxy en C₁-C₄, halogénoalcoxy en C₁-C₄, alkylthio en C₁-C₄, halogénoalkylthio en C₁-C₄, alkyle en C₁-C₄ ou halogénoalkyle en C₁-C₄,
R⁶ et R⁷ représentent indépendamment l'un de l'autre hydrogène ; alkyle en C₁-C₈, cycloalkyle en C₃-C₈, alcoxy en C₁-C₈, alcényle en C₃-C₈ ou alcoxy en C₁- C₈-alkyle en C₂-C₈, chacun éventuellement substitué par halogène ou cyano ; phényle ou benzyle, chacun éventuellement substitué par halogène, alkyle en C₁-C₈, halogénoalkyle en C₁-C₈ ou alcoxy en C₁-C₈ ; ou représentent ensemble un radical alkylène en C₃- C₆ éventuellement substitué par alkyle en C₁-C₆, dans lequel un groupe méthylène est éventuellement remplacé par l'oxygène ou le soufre,
à l'exception du composé I-a-75 d'EP 528 156

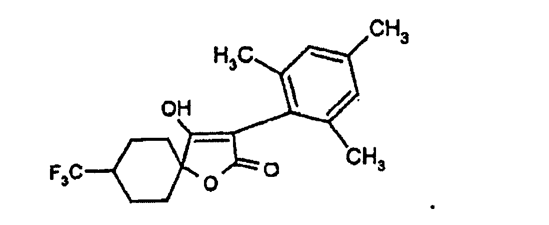

3. Composés de formule (I) selon la revendication 1, dans lesquels
V représente hydrogène, fluor, chlore, brome, alkyle en C₁-C₄ ou alcoxy en C₁-C₄,
W représente hydrogène, fluor, chlore, brome, alkyle en C₁-C₄, alcoxy en C₁-C₄, halogénoalkyle en C₁-C₂ ou halogénoalcoxy en C₁-C₂,
X représente fluor, chlore, brome, alkyle en C₁-C₄, alcoxy en C₁-C₄, halogénoalkyle en C₁-C₂, halogénoalcoxy en C₁-C₂, cyano ou nitro,
Y représente hydrogène, fluor, chlore, brome, alkyle en C₁-C₄, alcoxy en C₁-C₄, halogénoalkyle en C₁-C₂, halogénoalcoxy en C₁-C₂, cyano ou nitro,
Z représente hydrogène, fluor, chlore, brome, alkyle en C₁-C₄, alcoxy en C₁-C₄, halogénoalkyle en C₁-C₂, halogénoalcoxy en C₁-C₂, hydroxy, cyano, nitro ; ou phénoxy ou benzyloxy, chacun éventuellement substitué par fluor, chlore, brome, alkyle en C₁- C₄, alcoxy en C₁-C₄, halogénoalkyle en C₁-C₂, halogénoalcoxy en C₁-C₂, nitro ou cyano,
Het représente un des groupes 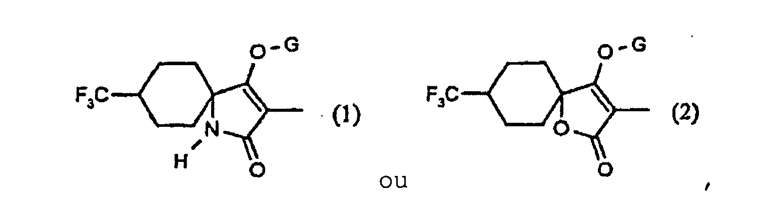
G représente hydrogène (a) ou un des groupes 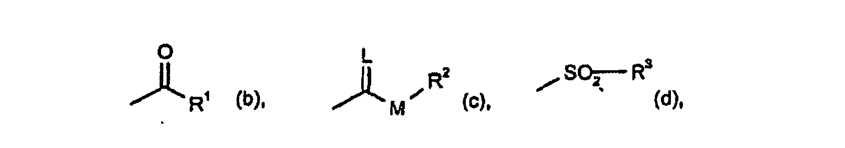 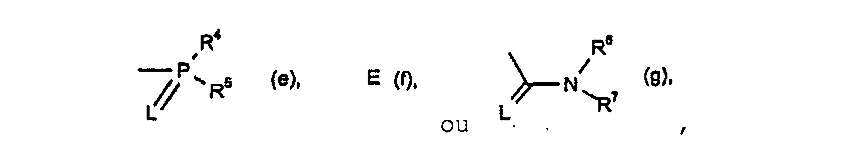 dans lesquels
E représente un ion métallique ou un ion ammonium,
L représente l'oxygène ou le soufre, et
M représente l'oxygène ou le soufre,
R¹ représente alkyle en C₁-C₁₆, alcényle en C₂-C₁₆, alcoxy en C₁-C₆-alkyle en C₁-C₆, alkylthio en C₁-C₆- alkyle en C₁-C₆ ou polyalcoxy en C₁-C₆-alkyle en C₁- C₆, chacun éventuellement substitué par fluor ou chlore ; ou cycloalkyle en C₃-C₇ éventuellement substitué par fluor, chlore, alkyle en C₁-C₅ ou alcoxy en C₁-C₅, dans lequel un ou deux groupes méthylène non directement voisins sont éventuellement remplacés par l'oxygène et/ou le soufre,
phényle éventuellement substitué par fluor, chlore, brome, cyano, nitro, alkyle en C₁-C₄, alcoxy en C₁-C₄, halogénoalkyle en C₁-C₃, halogénoalcoxy en C₁-C₃, alkylthio en C₁-C₄ ou alkylsulfonyle en C₁-C₄,
phénylalkyle en C₁-C₄ éventuellement substitué par fluor, chlore, brome, alkyle en C₁-C₄, alcoxy en C₁-C₄, halogénoalkyle en C₁-C₃ ou halogénoalcoxy en C₁-C₃,
pyrazolyle, thiazolyle, pyridyle, pyrimidyle, furanyle ou thiényle, chacun éventuellement substitué par fluor, chlore, brome ou alkyle en C₁- C₄,
phénoxyalkyle en C₁-C₅ éventuellement substitué par fluor, chlore, brome ou alkyle en C₁-C₄, ou pyridyloxyalkyle en C₁-C₅, pyrimidyloxyalkyle en C₁-C₅ ou thiazolyloxyalkyle en C₁-C₅, chacun éventuellement substitué par fluor, chlore, brome, amino ou alkyle en C₁-C₄,
R² représente alkyle en C₁-C₁₆, alcényle en C₂-C₁₆, alcoxy en C₁-C₆-alkyle en C₂-C₆ ou polyalcoxy en C₁- C₆-alkyle en C₂-C₆, chacun éventuellement substitué par fluor ou chlore, cycloalkyle en C₃-C₇ éventuellement substitué par fluor, chlore, alkyle en C₁-C₄ ou alcoxy en C₁-C₄, ou
phényle ou benzyle, chacun éventuellement substitué par fluor, chlore, brome, cyano, nitro, alkyle en C₁-C₄, alcoxy en C₁-C₃, halogénoalkyle en C₁-C₃ ou halogénoalcoxy en C₁-C₃,
R³ représente alkyle en C₁-C₆ éventuellement substitué par fluor ou chlore ; ou phényle ou benzyle, chacun éventuellement substitué par fluor, chlore, brome, alkyle en C₁-C₄, alcoxy en C₁-C₄, halogénoalcoxy en C₁-C₂, halogénoalkyle en C₁-C₂, cyano ou nitro,
R⁴ et R⁵ représentent indépendamment l'un de l'autre alkyle en C₁-C₆, alcoxy en C₁-C₆, alkylamino en C₁- C₆, dialkylamino en C₁-C₆, alkylthio en C₁-C₆ ou alcénylthio en C₃-C₄, chacun éventuellement substitué par fluor ou chlore ; ou phényle, phénoxy ou phénylthio, chacun éventuellement substitué par fluor, chlore, brome, nitro, cyano, alcoxy en C₁-C₃, halogénoalcoxy en C₁-C₃, alkylthio en C₁-C₃, halogénoalkylthio en C₁-C₃, alkyle en C₁- C₃ ou halogénoalkyle en C₁-C₃,
R⁶ et R⁷ représentent indépendamment l'un de l'autre hydrogène ; alkyle en C₁-C₆, cycloalkyle en C₃-C₆, alcoxy en C₁-C₆, alcényle en C₃-C₆ ou alcoxy en C₁- C₆-alkyle en C₂-C₆, chacun éventuellement substitué par fluor ou chlore ; phényle ou benzyle, chacun éventuellement substitué par fluor, chlore, brome, halogénoalkyle en C₁-C₅, alkyle en C₁-C₅ ou alcoxy en C₁-C₅ ; ou représentent ensemble un radical alkylène en C₃-C₆ éventuellement substitué par alkyle en C₁-C₄, dans lequel un groupe méthylène est éventuellement remplacé par l'oxygène ou le soufre,
à l'exception du composé I-a-75 d'EP 528 156

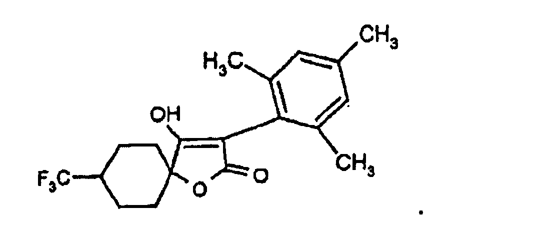

4. Composés de formule (I) selon la revendication 1, dans lesquels
V représente hydrogène, fluor, chlore, brome, méthyle, éthyle, méthoxy ou éthoxy,
W représente hydrogène, fluor, chlore, brome, méthyle, éthyle, propyle, méthoxy ou éthoxy,
X représente fluor, chlore, brome, méthyle, éthyle, propyle, iso-propyle, méthoxy, éthoxy, propoxy, iso-propoxy, trifluorométhyle, trifluorométhoxy, difluorométhoxy ou cyano,
Y représente hydrogène, fluor, chlore, brome, méthyle, éthyle, propyle, iso-propyle, tert.- butyle, méthoxy, éthoxy, propoxy, iso-propoxy, trifluorométhyle, trifluorométhoxy, difluorométhoxy, cyano ou nitro,
Z représente hydrogène, fluor, chlore, brome, méthyle, éthyle, propyle, iso-propyle, tert.- butyle, méthoxy, éthoxy, propoxy, iso-propoxy, trifluorométhyle, trifluorométhoxy, difluorométhoxy, cyano ou nitro,
Het représente un des groupes 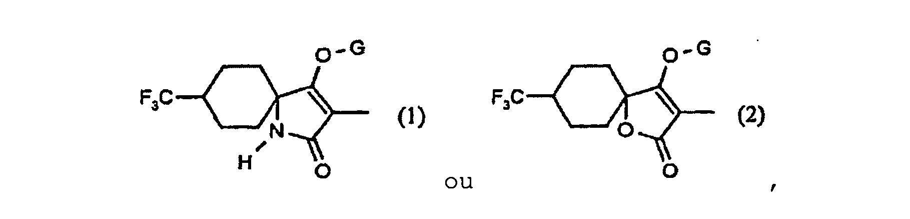
G représente hydrogène (a) ou un des groupes 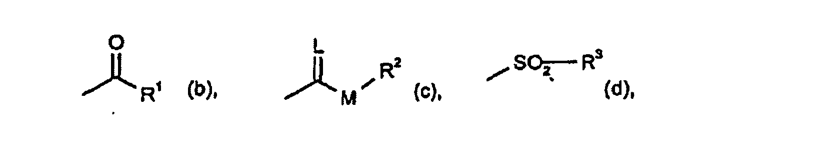 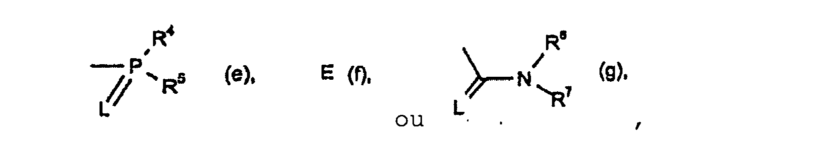 dans lesquels
E représente un ion métallique ou un ion ammonium,
L représente l'oxygène ou le soufre, et
M représente l'oxygène ou le soufre,
R¹ représente alkyle en C₁-C₁₄, alcényle en C₂-C₁₄, alcoxy en C₁-C₄-alkyle en C₁-C₆, alkylthio en C₁-C₄- alkyle en C₁-C₆, polyalcoxy en C₁-C₄-alkyle en C₁- C₄, chacun éventuellement substitué par fluor ou chlore ; ou cycloalkyle en C₃-C₆ éventuellement substitué par fluor, chlore, méthyle, éthyle, n- propyle, i-propyle, n-butyle, i-butyle, tert.- butyle, méthoxy, éthoxy, n-propoxy ou iso-propoxy, dans lequel un ou deux groupes méthylène non directement voisins sont éventuellement remplacés par l'oxygène et/ou le soufre,
phényle éventuellement substitué par fluor, chlore, brome, cyano, nitro, méthyle, éthyle, n- propyle, i-propyle, méthoxy, éthoxy, trifluorométhyle, trifluorométhoxy, méthylthio, éthylthio, méthylsulfonyle ou éthylsulfonyle,
benzyle éventuellement substitué par fluor, chlore, brome, méthyle, éthyle, n-propyle, i- propyle, méthoxy, éthoxy, trifluorométhyle ou trifluorométhoxy,
furanyle, thiényle ou pyridyle, chacun éventuellement substitué par fluor, chlore, brome, méthyle ou éthyle,
phénoxyalkyle en C₁-C₄ éventuellement substitué par fluor, chlore, méthyle ou éthyle, ou
pyridyloxyalkyle en C₁-C₄, pyrimidyloxyalkyle en C₁-C₉ ou thiazolyloxyalkyle en C₁-C₄, chacun éventuellement substitué par fluor, chlore, amino, méthyle ou éthyle,
R² représente alkyle en C₁-C₁₄, alcényle en C₂-C₁₄, alcoxy en C₁-C₄-alkyle en C₂-C₆ ou polyalcoxy en C₁- C₄-alkyle en C₂-C₆, chacun éventuellement substitué par fluor ou chlore,
cycloalkyle en C₃-C₆ éventuellement substitué par fluor, chlore, méthyle, éthyle, n-propyle, iso- propyle ou méthoxy, ou
phényle ou benzyle, chacun éventuellement substitué par fluor, chlore, cyano, nitro, méthyle, éthyle, n-propyle, i-propyle, méthoxy, éthoxy, trifluorométhyle ou trifluorométhoxy,
R³ représente méthyle, éthyle, propyle, iso-propyle, butyle, tert.-butyle, chacun éventuellement substitué par fluor ou chlore; ou phényle ou benzyle, chacun éventuellement substitué par fluor, chlore, brome, méthyle, éthyle, iso- propyle, tert.-butyle, méthoxy, éthoxy, iso- propoxy, trifluorométhyle, trifluorométhoxy, cyano ou nitro,
R⁴ et R⁵ représentent indépendamment l'un de l'autre alkyle en C₁-C₄, alcoxy en C₁-C₄, alkylamino en C₁- C₄, dialkylamino en C₁-C₄ ou alkylthio en C₁-C₄, chacun éventuellement substitué par fluor ou chlore ; ou phényle, phénoxy ou phénylthio, chacun éventuellement substitué par fluor, chlore, brome, nitro, cyano, méthyle, méthoxy, trifluorométhyle ou trifluorométhoxy,
R⁶ et R⁷ représentent indépendamment l'un de l'autre hydrogène ; alkyle en C₁-C₄, cycloalkyle en C₃-C₆, alcoxy en C₁-C₄, alcényle en C₃-C₄ ou alcoxy en C₁- C₄-alkyle en C₂-C₄, chacun éventuellement substitué par fluor ou chlore ; phényle ou benzyle, chacun éventuellement substitué par fluor, chlore, brome, méthyle, méthoxy ou trifluorométhyle ; ou représentent ensemble un radical alkylène en C₅-C₆ éventuellement substitué par méthyle ou éthyle, dans lequel un groupe méthylène est éventuellement remplacé par l'oxygène ou le soufre,
à l'exception du composé I-a-75 d'EP 528 156

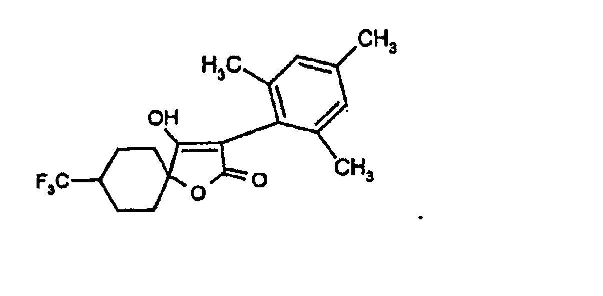

5. Procédé de fabrication de composés de formule (I) selon la revendication 1, **caractérisé en ce que**, pour obtenir
(A) des composés de formule (I-1-a) 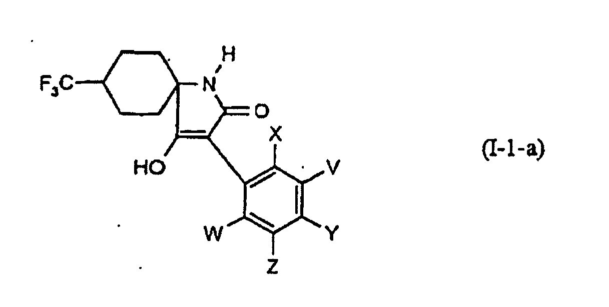 dans laquelle
V, W, X, Y et Z ont les significations données précédemment,
des composés de formule (II) 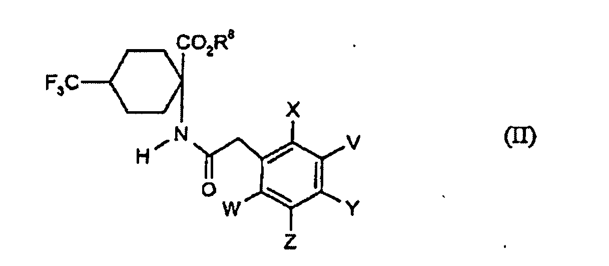 dans laquelle
V, W, X, Y et Z ont les significations données précédemment,
et
R⁸ représente alkyle,
sont condensés intramoléculairement en présence d'un diluant et en présence d'une base,
(B) des composés de formule (I-2-a) 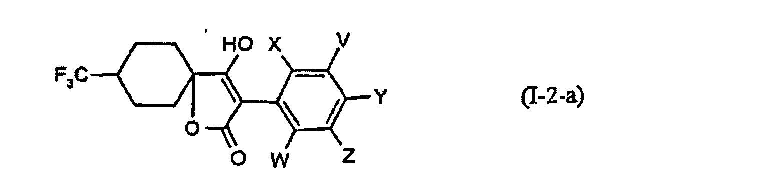 dans laquelle
V, W, X, Y et Z ont les significations données précédemment,
des composés de formule (III) 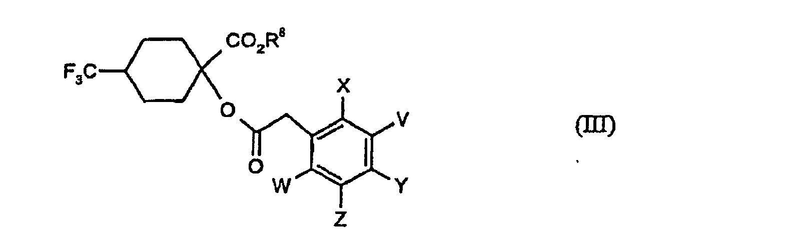 dans laquelle
V, W, X, Y, Z et R⁸ ont les significations données précédemment,
sont condensés intramoléculairement en présence d'un diluant et en présence d'une base, et les composés ainsi obtenus de formule (I-1-a) et (I-2-a) sont ensuite éventuellement mis en réaction
(C) α) avec des composés de formule (IV) 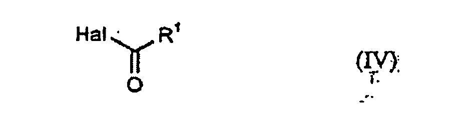 dans laquelle
R¹ a la signification donnée précédemment et
Hal représente un halogène,
ou
β) avec des composés de formule (V)
**R¹-CO-O-CO-R¹** **(V)**
dans laquelle
R¹ a la signification donnée précédemment,
éventuellement en présence d'un diluant et éventuellement en présence d'un liant acide ;
(D) avec des composés de formule (VI)
**R²-M-CO-Cl** **(VI)**
dans laquelle
R² et M ont les significations données précédemment,
éventuellement en présence d'un diluant et éventuellement en présence d'un liant acide ;
(E) avec des composés de formule (VII) 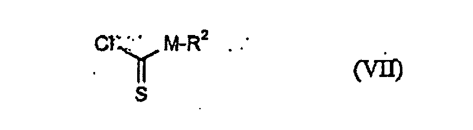 dans laquelle
M et R² ont les significations données précédemment,
éventuellement en présence d'un diluant et éventuellement en présence d'un liant acide,
(F) avec des composés de formule (VIII)
**R³-SO₂-Cl** **(VIII)**
dans laquelle
R³ a la signification donnée précédemment,
éventuellement en présence d'un diluant et éventuellement en présence d'un liant acide,
(G) avec des composés de formule (IX) 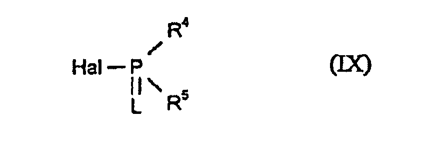 dans laquelle
L, R⁴ et R⁵ ont les significations données précédemment,
Hal représente un halogène,
éventuellement en présence d'un diluant et éventuellement en présence d'un liant acide,
(H) avec des composés de formule (X) ou (XI)
**Me(OR¹⁰)ₜ** **(X)** 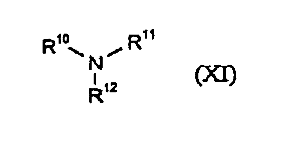
dans lesquelles
Me représente un métal mono- ou bivalent,
t représente le nombre 1 ou 2 et
R¹⁰, R¹¹, R¹² représentent indépendamment les uns des autres hydrogène ou alkyle,
éventuellement en présence d'un diluant,
(I) α) avec des composés de formule (XII)
**R⁶-N=C=L** **(XII)**
dans laquelle
R⁶ et L ont les significations données précédemment,
éventuellement en présence d'un diluant et éventuellement en présence d'un catalyseur, ou
β) avec des composés de formule (XIII) 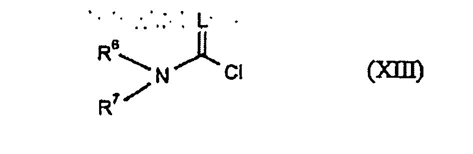 dans laquelle
L, R⁶ et R⁷ ont les significations données précédemment,
éventuellement en présence d'un diluant et éventuellement en présence d'un liant acide.

6. Composés de formule (II) 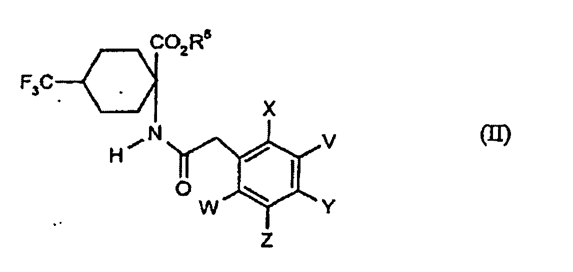 dans laquelle
V, W, X, Y et Z ont les significations données précédemment, et
R⁸ représente alkyle.

7. Composés de formule (III) 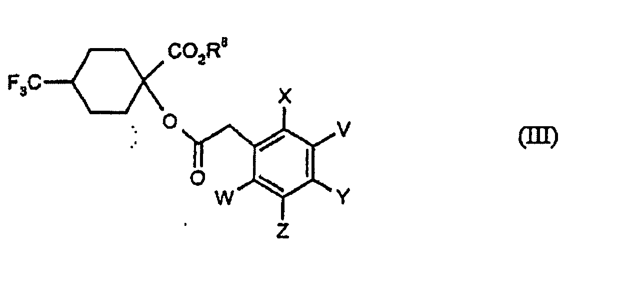 dans laquelle
V, W, X, Y, Z et R⁸ ont les significations données précédemment,
à l'exception du composé suivant

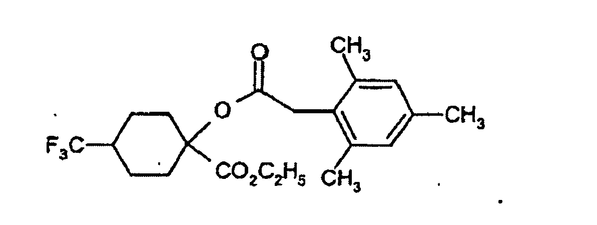

8. Composés de formule (XVI) 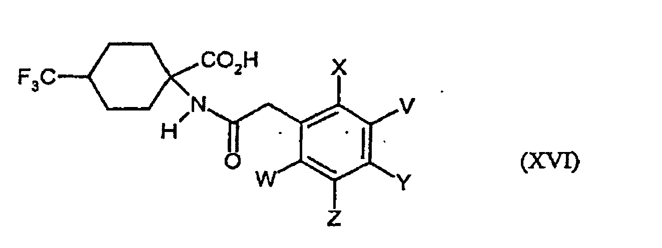 dans laquelle
V, W, X, Y et Z ont les significations données précédemment.

9. Composés de formule (XIX) 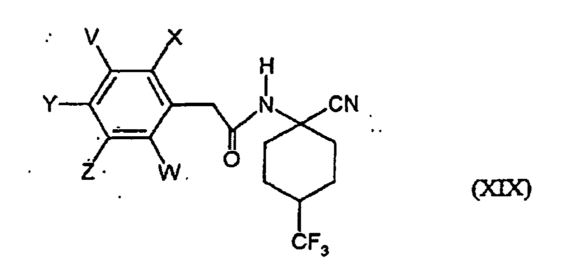 dans laquelle
V, W, X, Y et Z ont les significations données précédemment.

10. Composés de formule (XVIII)

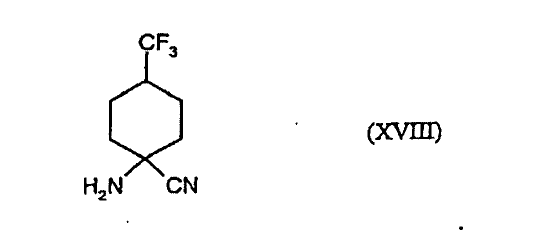

11. Composés de formule (XIV) 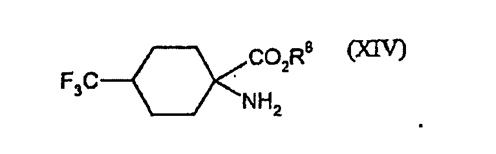 dans laquelle
R⁸ a les significations données précédemment.

12. Agent de lutte contre les organismes nuisibles et/ou agent de lutte contre les mauvaises herbes, **caractérisé par** une teneur en au moins un composé de formule (I) selon la revendication 1.

13. Utilisation de composés de formule (I) selon la revendication 1 pour lutter contre les organismes nuisibles et les mauvaises herbes, à l'exception de l'utilisation pour le traitement du corps humain et animal.

14. Procédé de lutte contre les organismes nuisibles et les mauvaises herbes, **caractérisé en ce que** des composés de formule (I) selon la revendication 1 sont laissés agir sur les organismes nuisibles, les plantes et/ou leur habitat, à l'exception de l'utilisation pour le traitement du corps humain et animal.

15. Procédé de fabrication d'agents de lutte contre les organismes nuisibles et/ou d'agents de lutte contre les mauvaises herbes, **caractérisé en ce que** des composés de formule (I) selon la revendication 1 sont mélangés avec des diluants et/ou des agents tensioactifs.

16. Utilisation de composés de formule (I) selon la revendication 1 pour la fabrication d'agents de lutte contre les organismes nuisibles et d'herbicides.
